# EUROPEAN PATENT APPLICATION

(11) **EP 1 506 959 A2**
(43) Date of publication of application: **16.02.2005**
(21) Application number: 04104605.3
(22) Date of filing: 22.09.2004
(51) Int. Cl.: C07D 207/448, C07D 207/46, C07D 493/08, C07C 69/732, C07C 217/62, C07F 9/22, C07C 233/17, C07F 7/10, C07D 491/06, C07C 243/38, C07C 239/20, C07D 335/10, C07D 417/12, C07D 333/24, C07H 19/06

(54) **Derivatised molecules for mass spectrometry**

(71) Applicant: OXFORD GENE TECHNOLOGY IP LIMITED, Sandy Lane, Yarnton, Oxford OX5 1PF (GB)
(72) Inventor: Shchepinov, Mikhail S. Oxf. Gene Tech.IP Ltd., Yamton, Oxford OX5 1PF (GB)
(74) Representative: Goodfellow, Hugh Robin

(57) **Abstract**

Compounds of formula (IIa): 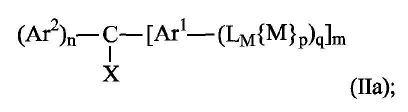 are provided where:
X is a group capable of being cleaved from the α-carbon atom to form an ion of formula (I') 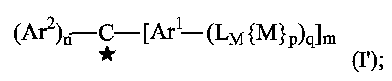
C 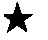 is a carbon atom bearing a single positive charge or a single negative charge;

The invention further provides compounds of formula (IIb): 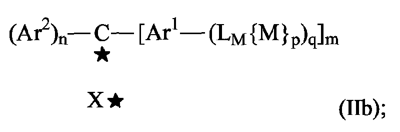 where:
X 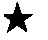 is a counter-ion to C 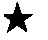 .

The compounds of formula (IIa) and (IIb) may form ions of formula (I') by either cleaving the C-X bond between X and the α-carbon atoms in the case of the compounds of formula (IIa) or dissociating X 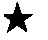 in the case of compounds of formula (IIb).

## Description

All documents cited herein are incorporated by reference in their entirety.

### TECHNICAL FIELD

This invention relates to compounds useful in mass spectrometry.

### BACKGROUND OF THE INVENTION

It is known that triphenylmethyl derivatives readily form ions. However, there remains a need for alternative and improved compounds which readily form ions and which are useful in mass spectrometry.

### DISCLOSURE OF THE INVENTION

The invention provides compounds of formula (IIa): 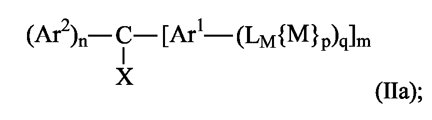 where:
X is a group capable of being cleaved from the α-carbon atom to form an ion of formula (I') 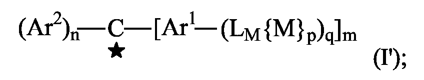
C 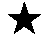 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
   optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more; and
q independently = 1 or more.

The invention further provides compounds of formula (IIb): 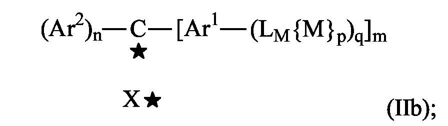 where:
X 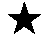 is a counter-ion to C 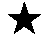 ;
and C 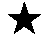 , M, Ar¹, Ar², L_{M}, n, m, p and q are as defined above.

The compounds of formula (IIa) and (IIb) may form ions of formula (I') by either cleaving the C-X bond between X and the α-carbon atoms in the case of the compounds of formula (IIa) or dissociating X 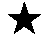 in the case of compounds of formula (IIb).

Ions of formula (I') are stabilised by the resonance effect of the aromatic groups Ar¹ a nd Ar². Electron-withdrawing groups, when C 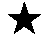 is an anion, or electron-donating groups, when C 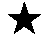 is a cation, may optionally be provided on Ar¹ and/or Ar² to assist this resonance effect.

The compounds of formula (IIa) and (IIb) are useful in the methods disclosed in UK patent application GB 03 284 14.8.

### Solid Supports

The invention also provides solid supports of formula (IVai), (IVaii) or (IVaiii): 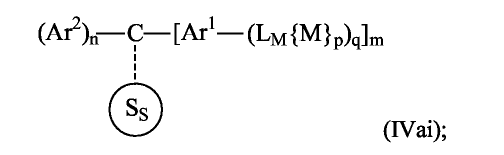 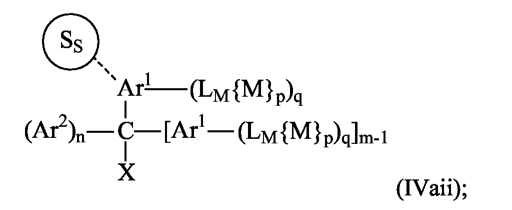 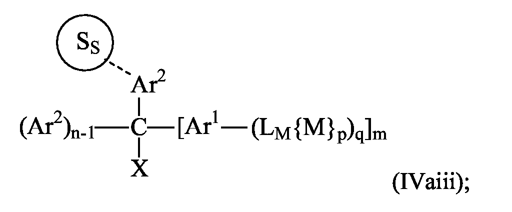 where:
X, Ar¹, Ar², L_{M}, M, n, m, p and q are as defined above;
S_{S} is a solid support;
C---S_{S} comprises a cleavable bond between C and S_{S};
S_{S}---Ar¹ comprises a cleavable bond between Ar¹ and S_{S}; and
S_{S}---Ar² comprises a cleavable bond between Ar² and S_{S}.

The cleavable bond of C- - -S_{S}, S_{S}- - -Ar¹ or S_{S}- - -Ar² may be a covalent, ionic, hydrogen, dipole-dipole or van der Waals bond.

The solid supports of formula (IVai), (IVaii) and (IVaiii) may form ions of formula (I'):
(a) for modified solid supports of formula (IVai) by cleaving the C-Sₛ bond between the α-carbon atom of the modified solid support of formula (IVai) and the solid support Sₛ to form the ion of formula (I');
(b) for modified solid supports of formula (IVaii) by, either simultaneously or sequentially, cleaving the C-X bond between X and the α-carbon atom and cleaving the S_{S}- - -Ar¹ bond between the solid support and the Ar¹ group to form the ion of formula (I'); or
(c) for modified solid supports of formula (IVaiii) by, either simultaneously or sequentially, cleaving the C-X bond between X and the α-carbon atom and cleaving the S_{S}- - -Ar² bond between the solid support and the Ar² group to form the ion of formula (I').

The invention also provides solid supports of formula (IVbii) or (IVbiii): 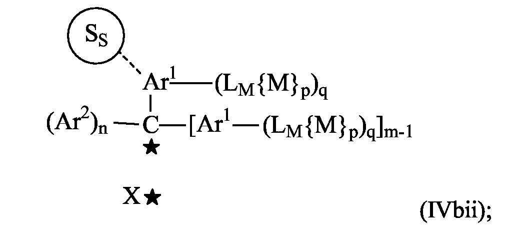 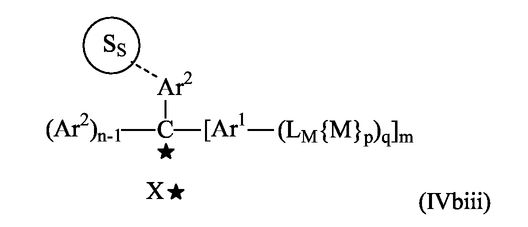 where: X 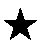 , Ar¹, Ar², L_{M}, M, n, m, p, q, Sₛ, C- - -Sₛ, Sₛ- - -Ar¹ and Sₛ--Ar² are as defined above.

The solid supports of formula (IVbii) and (IVbiii) may form ions of formula (I'):
(a) for modified solid supports of formula (IVbii) by, either simultaneously or sequentially, dissociating X 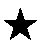 from the derivative of formula (IVbii) and cleaving the Sₛ- - Ar¹ bond between the solid support and the Ar¹ group to form an ion of formula (I'); or
(b) for modified solid supports of formula (IVbiii) by, either simultaneously or sequentially, dissociating X 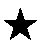 from the derivative of formula (IVbiii) and cleaving the Sₛ---Ar² bond between the solid support and the Ar² group to form an ion of formula (I').

The invention also provides solid supports of formula (IVaiv) or (IVbiv): 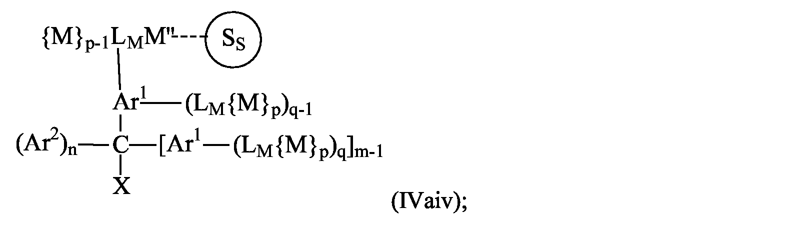 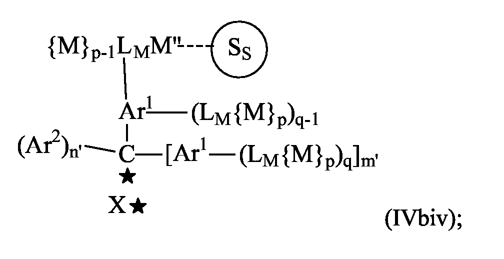 where:
X, X 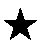 , Ar¹, Ar², L_{M}, M, p, q, n, m, and S_{S} are as defined above;
M"---S_{S} comprises a bond between M" and S_{S}; and
M" is the same as M except that S_{S} is bound to a portion of M which does not form part of the residue of M" remaining attached to the ion of formula (I') which residue is produced after reaction of group M".

In this embodiment of the invention, the solid support is bound to a part of group M" which does not go on to form part of the residue of M" remaining attached to the ion of formula (I') which residue is produced after reaction of group M".

The solid supports of formula (IVai), (IVaii), (IVaiii), (IVbii), (IVbiii), (IVaiv) and (IVbiv) are useful in the methods disclosed in UK patent application GB 03 284 14.8.

### Further Embodiments

### L_{M} bound to Ar¹ by more than one bond

The above-mentioned embodiments of the invention may also be provided in which L_{M} is bound to Ar¹ by more than one covalent bond (*e.g.* 2 or 3 bonds) which are either single, double or triple covalent bonds, or one or more multiple bonds (*e.g.* double or triple covalent bonds). All the other features of the invention are the same except the groups which relate to the bond or bonds between Ar¹ and L_{M}.

### Disclaimers

Preferably, all embodiments of the invention (including products of formulae (I') and (IIa)) involving or relating to the compound of formula (XI) are disclaimed

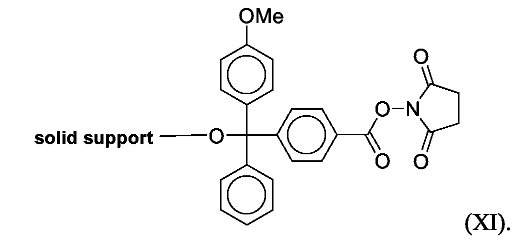

Preferably, all embodiments of the invention (including products of formulae (I') and (IIa)) involving or relating to the compound of formula (XIa) are disclaimed

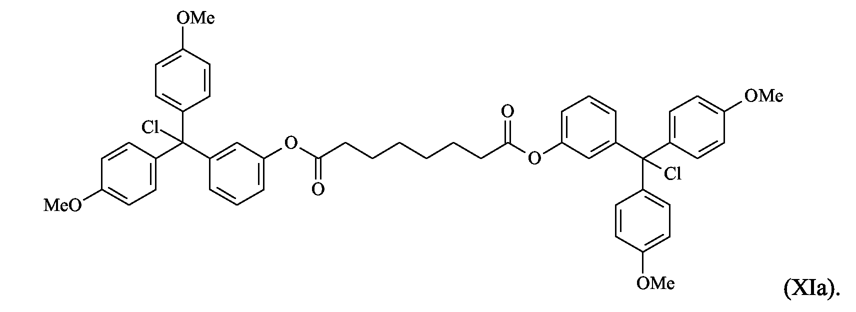

Preferably, all embodiments of the invention (including products of formulae (I') and (IIa)) involving or relating to the compound of formula (XIb) are disclaimed.

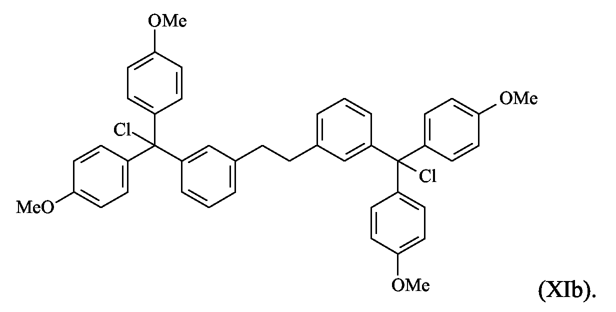

Preferably, all embodiments of the invention (including products of formulae (I') and (IIa)) involving or relating to the compound of formula (XIc) are disclaimed

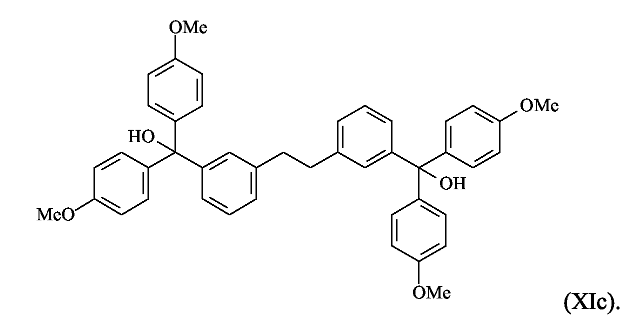

Preferably, all embodiments of the invention (including products of formulae (I') and (IIa)) involving or relating to the compound of formula (XId) are disclaimed

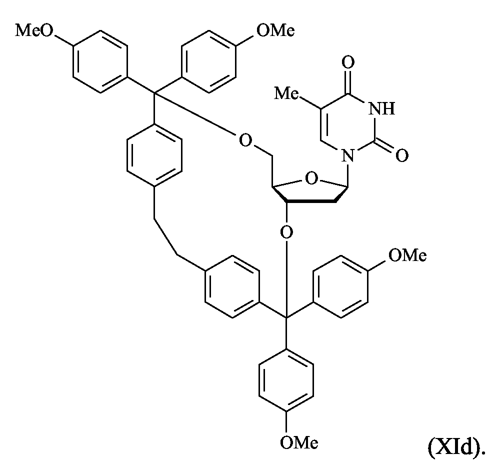

Preferably, all embodiments of the invention (including products of formulae (I') and (IIa)) involving or relating to the compound of formula (XIe) are disclaimed

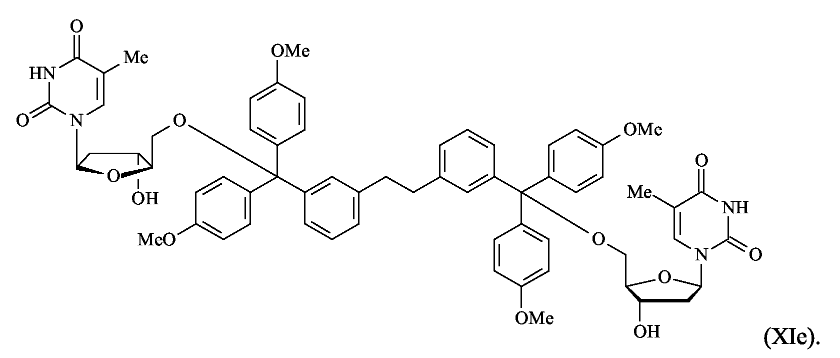

Preferably, all embodiments of the invention (including products of formulae (I') and (IIa)) involving or relating to the compound of formula (XIe) are disclaimed

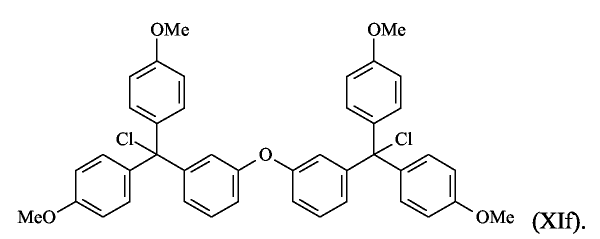

Preferably, all embodiments of the invention (including products of formulae (I') and (IIa)) involving or relating to the compound of formula (XIg-j) are disclaimed

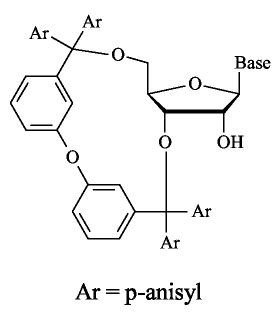

| **Formula** | **Base** |
|---|---|
| XIg | Uridine |
| XIh | N⁴-benzoyl-cytidine |
| XIi | N⁶-benzoyl-adenosine |
| XIj | N²-phenylacetyl-guanosine |

Preferably, all embodiments of the invention (including products of formulae (I') and (IIa)) involving or relating to the compound of formula (XIk-n) are disclaimed

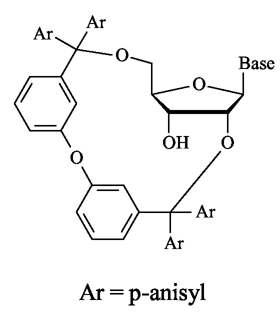

| **Formula** | **Base** |
|---|---|
| XIk | Uridine |
| XI1 | N⁴-benzoyl-cytidine |
| XIm | N⁶-benzoyl-adenosine |
| XIn | N ²-phenylacetyl-guanosine |

***Preferred Embodiments***

*Definition of C* 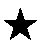

Preferably, C 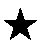 bears a single positive charge such that ions of the invention are cations and the ion of formula (I') has the following structure: 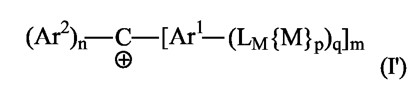 and the compounds of formulae (IIb), (IVbii), (IVbiii) and (IVbiv) have the structures disclosed in table 1.

### n, m, p and q

For the purposes of compounds of the invention having n-1 groups Ar², n may not be less than 1.

Preferably n = 2 and m = 1.

Preferably p = 1, 2 or 3. Preferably p = 1.

Preferably q = 1, 2 or 3. Preferably q = 1.

Preferably n = 2, m = 1, p = 1 and q = 1. The ion of formula (I') thus has the structure: 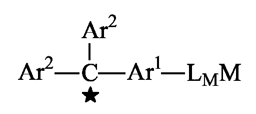 or more preferably 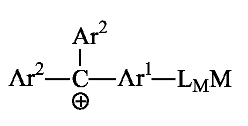 and the compounds of formulae (IIa), (IIb), (IVai), (IVaii), (IVaiii), (IVaiv), (IVbii), (IVbiii) and (IVbiv) have the structures disclosed in table 2.

### Group M

The group M is a reactive functional group. Reactive functional groups include groups capable of reacting to form a covalent linkage and groups capable of ionic bonding, hydrogen bonding, dipole-dipole bonding or van der Waals bonding. Particularly preferred groups M are those capable of reacting to form a covalent linkage.

The group M is bound to L_{M} by one or more covalent bonds (*e.g.* 2 or 3 bonds, especially 2 such as 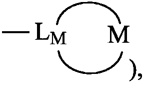 which are either single, double or triple covalent bonds (preferably single bonds). Preferably, M is bound to L_{M} by one single bond.

Alternatively, or in addition, M is bound by more than one L_{M}, such L_{M} either being attached to the same or different Ar¹ or Ar². In a preferred embodiment M is bound by more than one L_{M} from different Ar¹ or Ar², *e*.*g*.:

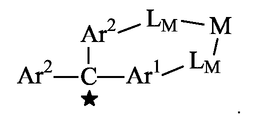

### Covalent Linkage

Particularly preferred groups M are those capable of reacting to form a covalent linkage.

Examples of group M bound to L_{M} by one bond include -NR₂ *e.g.* -NHR (*e.g.* -NHMe (*e.g.* compound 17b), especially -NH₂ ( *e.g.* compounds 12c & 13b); -SR *e.g.* -SH; -OR *e.g. -*OH (*e.g.* compound 3a); -B(R)Y; -BY₂; -C(R)₂Y; -C(R)Y₂; -CY₃; -C(=Z)Y *e.g.* -C(=O)Y; -Z-C(=Z)Y; - C(=Z)R *e.g.* -C(=Z)H, especially -C(=O)H; -C(R)(OH)OR; -C(R)(OR)₂; -S(=O)Y; -Z-S(=O)Y; -S(=O)₂Y; -Z-S(=O)₂Y; -S(=O)₃Y; -Z-S(=O)₃Y; -P(=Z)(ZR)Y *e.g.* -P(=O)(OH)Y; -P(=Z)Y₂; -Z-P(=Z)(ZR)Y; -Z-P(=Z)Y₂; -P(=Z)(R)Y *e.g.* -P(=O)(H)Y; -Z-P(=Z)(R)Y; or -N=C(=Z) *e.g.* - N=C(=O).

Other examples of group M bound to L_{M} by one bond are -P(ZR)Y *e.g.* -P(OH)Y; -PY₂; -Z-P(ZR)Y; -Z-PY₂; -P(R)Y *e*.*g*. -P(H)Y; -Z-P(R)Y. A particularly preferred group M is -Z-P(ZR)Y, especially a phosphoramidite group:

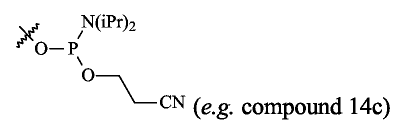

Another example of group M bound to L_{M} by one bond is -Y. In particular, when group M is halo (especially iodo), M may be bound to an aliphatic (*e.g.* compound 17c) or aromatic carbon (*e.g.* compounds 28c & 28d). When M is halo (*e*.*g*. iodo) and is bound to an aromatic carbon, L_{M} may, for example, be a single bond.

Examples of group M bound to L_{M} by two bonds include -N(R)- *e.g.* -NH-; -S-; -O-; -B(Y)-; - C(R)(Y)-; -CY₂-; -C(=O)-; -C(OH)(OR)-; -C(OR)₂-.

Examples of group M bound to L_{M} by three bonds include

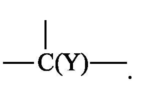

Preferred groups M include electrophilic groups, especially those susceptible to SN₂ substitution reactions, addition-elimination reactions and addition reactions, *e.g.* -B(R)Y; -BY₂; -C(R)₂Y; -C(R)Y₂; -CY₃; -C(=Z)Y *e.g.* -C(=O)Y, especially -C(O)OH (*e.g.* compound 24b) and -C(O)NH₂ (*e.g.* compound 19e); -Z-C(=Z)Y; -C(=Z)R *e.g.* -C(=Z)H, especially -C(=O)H; -C(R)(OH)OR; -C(R)(OR)₂; -S(=O)Y; -Z-S(=O)Y; -S(=O)₂Y; -Z-S(=O)₂Y; -S(=O)₃Y; -Z-S(=O)₃Y; -P(=Z)(ZR)Y *e.g.* -P(=O)(OH)Y; -P(=Z)Y₂; -Z-P(=Z)(ZR)Y; -Z-P(=Z)Y₂; -P(=Z)(R)Y *e.g.* -P(=O)(R)Y; -Z-P(=Z)(H)Y; -N=C(=Z) *e.g.* -N=C(=O); -B(Y)-; -C(R)(Y)-; -CY₂-; -C(=O)-; -C(OH)(OR)-; - C(OR)₂-; or

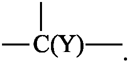

Still further preferred examples of group M are orthoesters, *e.g.* -C(OR)₃. In a preferred embodiment, the R groups are linked together to form a hydrocarbyl group, *e.g.* a C₁₋₈alkyl group. A preferred example of group M in this embodiment is:

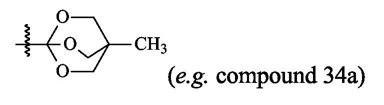

Another preferred group M is maleimido (*e*.*g*. compound 18d).

Y is independently a leaving group, including groups capable of leaving in an SN₂ substitution reaction or being eliminated in an addition-elimination reaction.

Preferred examples of Y include halogen (preferably iodo), C₁₋₈hydrocarbyloxy (*e.g.* C₁₋₈alkoxy), C₁₋₈hydrocarbyloxy substituted with one or more A, C₁₋₈heterohydrocarbyloxy, C₁₋₈heterohydrocarbyloxy substituted with one or more A, mesyl, tosyl, pentafluorophenyl, -O-succinimidyl (formula VII) or a sulfo sodium salt thereof (sulfoNHS - formula VIIa), - S-succinimidyl, or phenyloxy substituted with one or more A *e.g.* p-nitrophenyloxy (formula VIII) or pentafluorophenoxy (formula VIIIa) (*e.g.* compound 16).

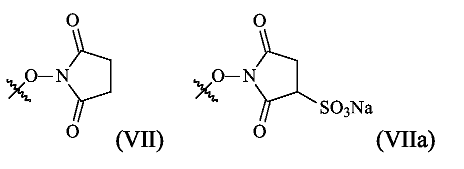

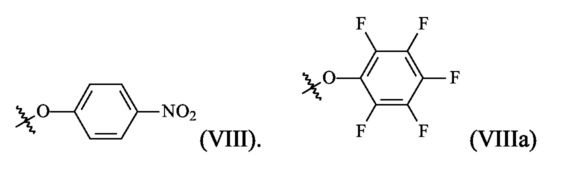

Thus, preferred groups M are:

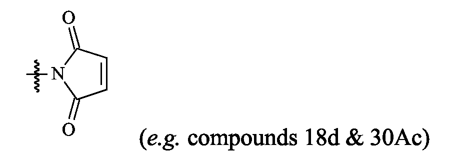

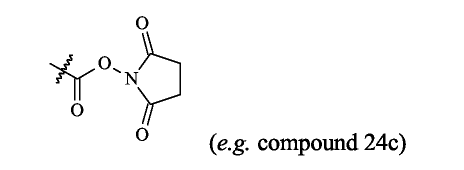

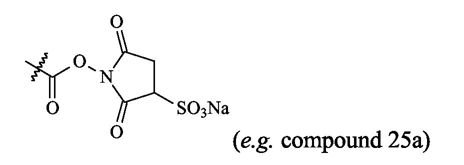

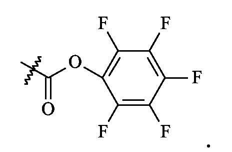

Other preferred examples of Y include -ZR. Particularly preferred examples of Y are -ZH (*e.g.* -OH or -NH₂) and -Z-C₁₋₈alkyl groups such as -NH-C₁₋₈alkyl groups (*e.g.* -NHMe) and -O-C₁₋₈alkyl groups (*e.g.* -O-t-butyl). Thus, preferred groups M are -C(O)-NH-C₁₋₈alkyl (*e*.*g*. -C(O)NHMe) and -C(O)-O-C₁₋₈alkyl (*e*.*g*. -C(O)-O-t-butyl (*e*.*g*. compounds 24a & 33a)).

Other preferred examples of Y include -Z-ZR. Particularly preferred examples include -NR-NR₂, especially -NH-NH₂ (*e*.*g*. compounds 35Ab, 35Bc and 35Bd), and -ONR₂, especially -O-NH₂ *(e.g.* compounds 35Cc and 35Cd).

Z is independently O, S or N(R). Preferred (=Z) is (=O).

R is independently H, C₁₋₈hydrocarbyl (*e*.*g*. C ₁₋₈alkyl) or C₁₋₈hydrocarbyl substituted with one or more A.

R is preferably H.

Particularly preferred groups M include -C(=O)Y, especially -C(=O)-O-succinimidyl and - C(=O)-O-(p-nitrophenyl).

In a further embodiment, M may be -Si(R)₂-Y, with Y being halo (*e.g.* chloro) being especially preferred. Preferred groups R in this embodiment are C₁₋₈alkyl, especially methyl. A particularly preferred group M in this embodiment is -Si(Me)₂Cl (*e.g.* compound 19d).

In a further embodiment, M may be -C(Ar²)₂X. Preferred groups Ar² and X are set out below. In this embodiment it is preferred that L_{M} is a bond. A particularly preferred group M in this embodiment is:

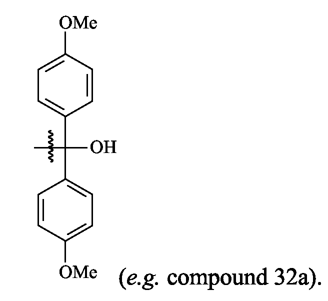

Other groups M include groups capable of reacting in a cycloaddition reaction, especially a Diels-Alder reaction.

In the case of Diels-Alder reactions, the group M is either a diene or a dienophile. Preferred diene groups are 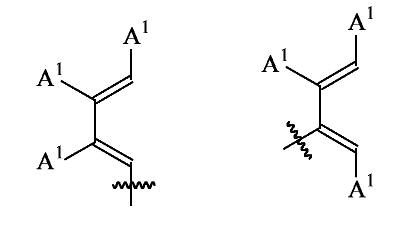 and multivalent derivatives formally formed by removal of one or more hydrogen atoms, where A¹ is -R¹ or -Z¹R¹, where R¹ and Z¹ are defined below.

Preferred dienophile groups are -CR¹=CR¹₂, -CR¹=C(R¹)A², -CA²=CR¹₂, -CA²=C(R¹)A² or - CA²=CA²₂, and multivalent derivatives formally formed by removal of one or more hydrogen atoms, where R¹ is defined below and A² is independently halogen, trihalomethyl, -NO₂, -CN, -N⁺(R¹)₂O⁻, -CO₂H, -CO₂R¹, -SO₃H, -SOR¹, -SO₂R¹, -SO₃R¹, -OC(=O)OR¹, -C(=O)H, -C(=O)R¹, -OC(=O)R¹, , -OC(=O)NR¹₂, -N(R¹)C(=O)R¹, -C(=S)NR¹₂, -NR¹C(=S)R¹, -SO₂NR¹₂, -NR¹SO₂R¹, - N(R¹)C(=S)NR¹₂, or -N(R¹)SO₂NR¹₂, where R¹ is defined below. A particularly preferred dienophile group is maleimidyl.

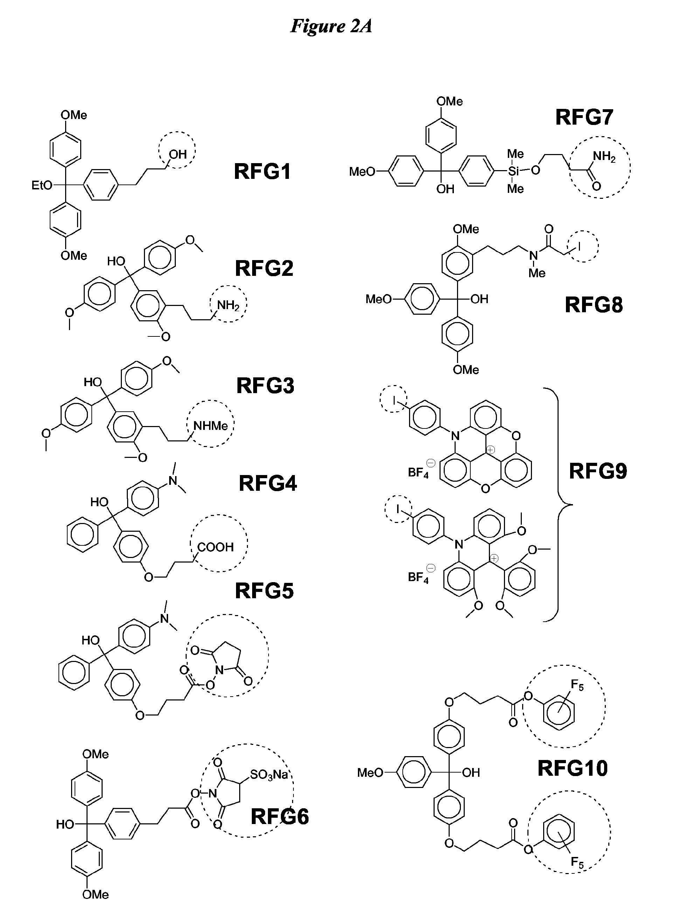

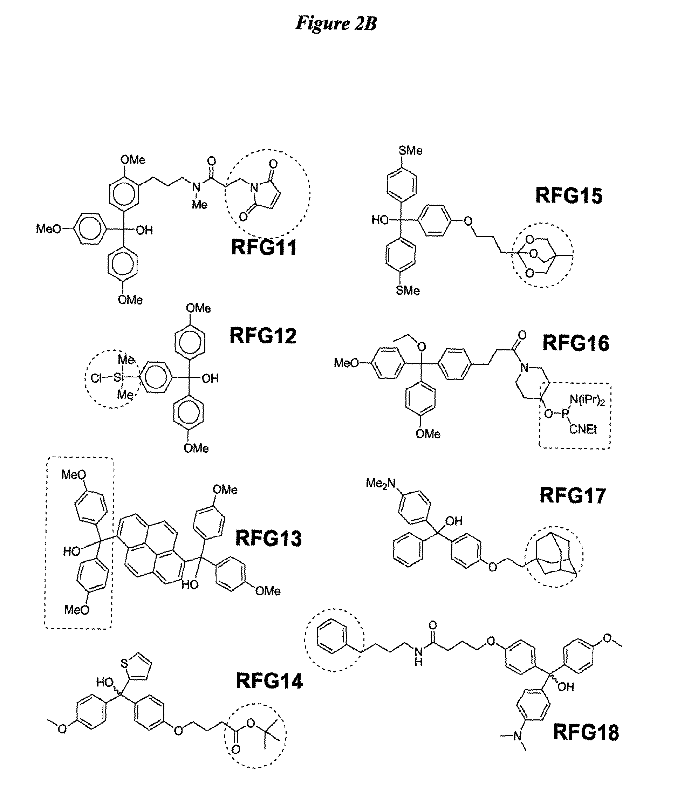

Preferred examples of group M are shown in figures 2A and 2B.

### Ionic Bonding

Where group M is a reactive functional group capable of ionic bonding, group M typically comprises one or more chelating ligands.

Suitable chelating ligands which can bind anions include polyamines and cryptands.

Suitable chelating ligands which can bind cations include polyacidic compounds (*e.g.* EDTA) and crown ethers.

### Hydrogen Bonding

Where group M is a reactive functional group capable of hydrogen bonding, M will typically bear one or more hydroxy, amino or thio hydrogen atoms or a group bearing an atom having one or more lone pair of electrons (*e.g.* an oxygen, sulphur or nitrogen atom).

Preferred groups capable of hydrogen bonding include biotin, avidin and streptavidin.

### Dipole-Dipole Bonding

Where group M is a reactive functional group capable of dipole-dipole bonding, the dipole-dipole bond may be formed between permanent dipoles or between a permanent dipole and an induced dipole.

Preferred groups M capable of dipole-dipole bonding comprise acid groups, or -(NMe₃)⁺, carboxy, carboxylate, phosphate or sulphate groups.

### Van der Waals Bonding

Where group M is a reactive functional group capable van der Waals bonding, M will typically comprise a hydrocarbyl or heterohydrocarbyl group (usually a large hydrocarbyl group having at least ten carbon atoms up to about 50 carbon atoms), optionally substituted with one or more A. Polyfluorinated hydrocarbyl and heterohydrocarbyl groups are particularly preferred. Typically, the hydrocarbyl or heterohydrocarbyl groups are aryl or heteroaryl groups or groups of the formula -C(R⁶)₂Ar³, -C(R⁶)(Ar³)² or -C(Ar³)₃, where Ar³ is independently defined the same as Ar² and R⁶ is H, C₁₋₈ hydrocarbyl, C₁₋₈ hydrocarbyl substituted by one or more A, C₁₋₈ heterohydrocarbyl or C₁₋₈ heterohydrocarbyl substituted by one or more A.

A preferred group capable of van der Waals bonding is tetrabenzofullerene (formula X).

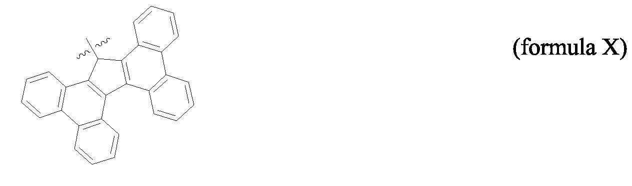

Other preferred groups capable of van der Waals bonding are adamantyl (*e.g.* 2 -adamantyl (*e.g.* compound 36a)) and phenyl (*e.g.* example 37b).

Preferably, these groups are linked to a hydrocarbylene group (*e.g.* C₁₋₈ alkylene group) which forms L_{M} or a part thereof.

### Group M"

M" is the same as M except that Sₛ is bound to a portion of M which does not from part of the residue of M" remaining attached to the ion of formula (I') which residue is produced after reaction of group M". Thus, M" is a residue of M formable by the conjugation of M and Sₛ. However, M" need not necessarily be formed by the conjugation of M and Sₛ.

M"- - -Sₛ comprises a covalent, ionic, dipole-dipole, hydrogen, or van der Waals bond. The covalent, ionic, hydrogen, dipole-dipole or van der Waals bond may be direct between M" and Sₛ or may be provided by one or more binding groups present on M" and/or Sₛ.

Examples of groups which can form these types of bond, and methods for cleaving these types of bond, are set out below in connection with C---S_{S} bonds, *etc.*

Preferred groups M" are groups M having a leaving group, wherein the group Sₛ is bound to the leaving group, *e.g.* groups M mentioned above having a leaving group Y, wherein the group Sₛ is bound to the leaving group Y.

A particularly preferred group M" is:

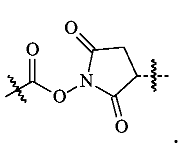

### L_{M}

Where the group L_{M} is a linker atom or group, it has a sufficient number of linking covalent bonds to link L_{M} to the group Ar¹ by a single covalent bond (or more, as appropriate) and to link L_{M} to the p instances of M groups (which may be attached to L_{M} by one or more bonds).

The group L_{M} may be directly bound to the aromatic part of Ar¹, bound to one or more of the substituents A of Ar¹, or both. Preferably, L_{M} is bound directly to the aromatic part of Ar¹.

In an alternative embodiment, L_{M} may be bound to L₅.

When L_{M} is a linker atom, preferred linker atoms are O or S, particularly O.

When L_{M} is a linker group, preferred linker groups, in the orientation Ar¹-(L_{M}{M}ₚ)_{q}, are -E^{M}-, - (D^{M})ₜ-, -(E^{M}-D^{M})ₜ-, -(D^{M}-E^{M})ₜ-, -E^{M}-(D^{M}-E^{M})ₜ- or -D^{M}-(E^{M}-D^{M})ₜ-, where a sufficient number of linking covalent bonds, in addition to the covalent bonds at the chain termini shown, are provided on groups E^{M} and D^{M} for linking the p instances of M groups.

D^{M} is independently C₁₋₈hydrocarbylene or C₁₋₈hydrocarbylene substituted with one or more A. Preferred D^{M} are C₁₋₈alkylene, C₁₋₈alkenylene and C₁₋₈alkynylene, especially C₁₋₈alkylene and C₁₋₈alkynylene, each optionally substituted with one or more A (preferably unsubstituted). A preferred substituent A is ²H. Preferred L_{M} in the orientation Ar¹-(L_{M}{M}ₚ)_{q} are: -CH₂CH₂-(*e.g.* compounds 1a & 2a); -C-≡C-CH₂CH₂CH₂- (*e.g.* compounds 6b, 6c, 6d, 7a, 7b & 7c); -(CH₂)₅-(*e.g.* compounds 8a, 8b & 8c);-CD₂CD₂CH₂CH₂CH₂-; -C-≡C-CH₂- (*e.g.* compounds 12b & 12c) and-CH₂CH₂CH₂- (*e.g.* compounds 4a, 5a, 13a & 13b).

E^{M}, in the orientation Ar¹-(L_{M}{M}ₚ)_{q}, is independently -Z^{M}-, -C(=Z^{M})-, -Z^{M}C(=Z^{M})-, -C(=Z^{M})Z^{M}-, -Z^{M}C(=Z^{M})Z^{M}-, -S(=O)-, -Z^{M}S(=O)-, -S(=O)Z^{M}-, -Z^{M}S(=O)Z^{M}-, -S(=O)₂-, -Z^{M}S(=O)₂-, -S(=O)₂Z^{M}-, - Z^{M}S(=O)₂Z^{M}-, where Z^{M} is independently O, S or N(R^{M}) and where R^{M} is independently H, C₁₋₈hydrocarbyl (*e.g.* C₁₋₈alkyl) or C₁₋₈hydrocarbyl substituted with one or more A. Preferably E^{M} is, in the orientation Ar¹-(L_{M}{M}ₚ)_{q}, -O-, -S-, -C(=O)-, -C(=O)O-, -C(=S)-, -C(=S)O-, -OC(=S)-, -C(=O)S-, -SC(=O)-, -S(O)-, -S(O)₂-, -NR^{M}-, -C(=O)N(R^{M})-, -C(=S)N(R^{M})-, -N(R^{M})C(=_{O})-, -N(R^{M})C(=S)-, -S(=O)N(R^{M})-, -N(R^{M})S(=O)-, -S(=O)₂N(R^{M})-, -N(R^{M})S(=O)₂-, -OC(=O)O-, -SC(=O)O-, -OC(=O)S-, -N(R^{M})C(=O)O-, -OC(=O)N(R^{M})-, -N(R^{M})C(=O)N(R^{M})-, -N(R^{M})C(=S)N(R^{M})-, -N(R^{M})S(=O)N(R^{M})- or N(R^{M})S(=O)₂N(R^{M})-.

Alternative groups E^{M} to those defined above, in the orientation Ar¹-(L_{M}{M}ₚ)_{q}, are -Z^{M}-Si(R^{M})₂-Z^{M}-, -Si(R^{M})₂-Z^{M}- and -Z^{M}-Si(R^{M})₂-. The group -Si(R^{M})₂-Z^{M}- is particularly preferred. Z^{M} is preferably O. R^{M} is preferably C₁₋₈alkyl, preferably methyl. These groups E^{M} are particularly preferred in the groups -(E^{M}-D^{M})ₜ-, especially when t=1 and D^{M} is C₁₋₈alkylene. The following group is especially preferred:

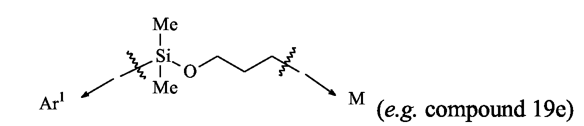

In addition to the above definition of D^{M}, D^{M} may also be C₁₋₈heterohydrocarbylene or C₁₋₈heterohydrocarbylene substituted with one or more A. In this embodiment, C₁₋₈cycloheteroalkylene groups are particularly preferred, *e.g.*: 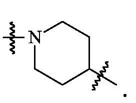 Thus, preferred L_{M} groups -D^{M}-E^{M}-D^{M}- are, in the orientation Ar¹-(L_{M}{M}ₚ)_{q}, - C₁₋₈alkylene-C(O)-C₁₋₈cycloheteroalkylene (preferably where the hetero atom is N and is bound to the carboxy), especially: 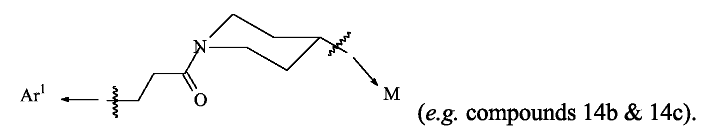 t = 1 or more, *e.g.* from 1 to 50, 1to 40, 1 to 30, 1 to 20 or 1 to 10. Preferably t = 1, 2, 3, 4, 5, 6, 7, 8, 9, or 10.

Preferably, L_{M} links one group M to Ar¹, M is linked to L_{M} by a single covalent bond and therefore no additional bonds are required (*e.g.* L_{M}{M}₁ may be -E^{M}-{M}, -(D^{M})ₜ-{M}, -(E^{M}-D^{M})ₜ-{M}, -(D^{M}-E^{M})ₜ-{M}, -E^{M}-(D^{M}-E^{M})ₜ-{M} or -D^{M}-(E^{M}-D^{M})ₜ-{M}).

Where L_{M} incl udes a group which also falls within the definition of group M, the group M is preferably more reactive than the group included in L_{M}.

L_{M} is preferably -(D^{M})ₜ-, -(E^{M}-D^{M})ₜ-, or -D^{M}-(E^{M}-D^{M})ₜ-.

When group L_{M} is -(D^{M})ₜ-, t is preferably 1. D^{M} is preferably C₁₋₈alkylene, preferably C₁₋₅alkylene, preferably methylene or ethylene.

When group L_{M} is -(E^{M}-D^{M})ₜ-, or -D^{M}-(E^{M}-D^{M})ₜ-, E^{M} is preferably (in the orientation Ar¹-(L_{M}{M}ₚ)_{q}), -C(=O)N(R^{M})- ( *e.g.* -C(=O)NH-) or O (preferably O), and D^{M} is preferably C₁₋₈alkylene, preferably ethylene, propylene, butylene or pentylene. t is preferably 1. Especially preferred L_{M} are, in the orientation Ar¹-(L_{M}{M}ₚ)_{q}, -O-CH₂CH₂CH₂- (*e.g.* compounds 15a, 15b, 15c & 16a) and -O-CH₂CH₂CH₂CH₂CH₂- (*e.g.* compounds 10a, 10b, 10c, 11a, 11b & 11c).

Another preferred group -D^{M}-(E^{M}-D^{M})ₜ- is where D^{M} is C₁₋₈alkylene and t is 1. Preferred E^{M} in this group, in the orientation Ar¹-(L_{M}{M}ₚ)_{q}, are -Z^{M}C(=Z^{M})- (especially -N(R^{M})C(=O)-, *e.g.* -N(Me)C(=O)-) and -C(=Z^{M})Z^{M}- (especially -C(=O)O-). Particularly preferred L_{M} groups are:

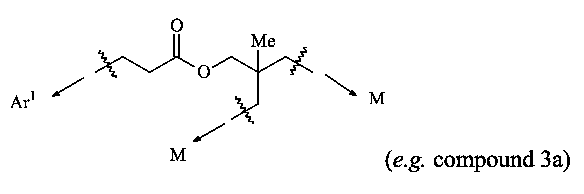

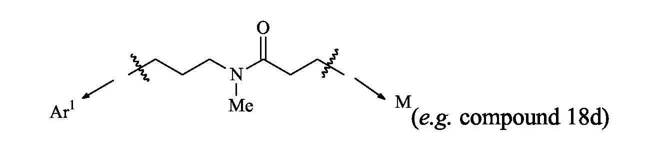

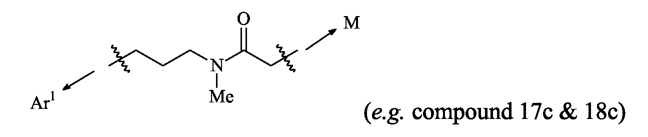

The group -(E^{M}-D^{M})ₜ- is preferred, a particularly preferred example of which is (in the orientation Ar¹-(L_{M}{M}ₚ)_{q}) -C(=O)NH-CH₂CH₂CH₂-O-CH₂CH₂-O-CH₂CH₂-O-CH₂CH₂CH₂-.

The group -(D^{M}-E^{M})ₜ- is also preferred when D^{M} is C₁₋₈alkylene and t is 1. Preferred E^{M} in this group, in the orientation Ar¹-(L_{M}{M}ₚ)_{q} are -Z^{M}C(=Z^{M})- and -C(=Z^{M})Z^{M}-, especially -Z^{M}C(=Z^{M})-(particularly -N(R^{M})C(=O)-, *e.g.* -N(Me)C(=O)-). A particularly preferred example is -CH₂CH₂CH₂N(Me)C(O)-.

In an alternative embodiment it is preferred that L_{M} is a single covalent bond.

When Ar² is phenyl, L_{M} is preferably provided in a position ortho or para to C 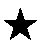 . When Ar² is other than phenyl, L_{M} is preferably attached to an atom which bears the charge in at least one of the resonance structures of the ions of formula (I').

Where C 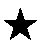 is a cation, L_{M} is preferably an electron-donating group. Where C 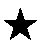 is an anion, L_{M} is preferably an electron-withdrawing group.

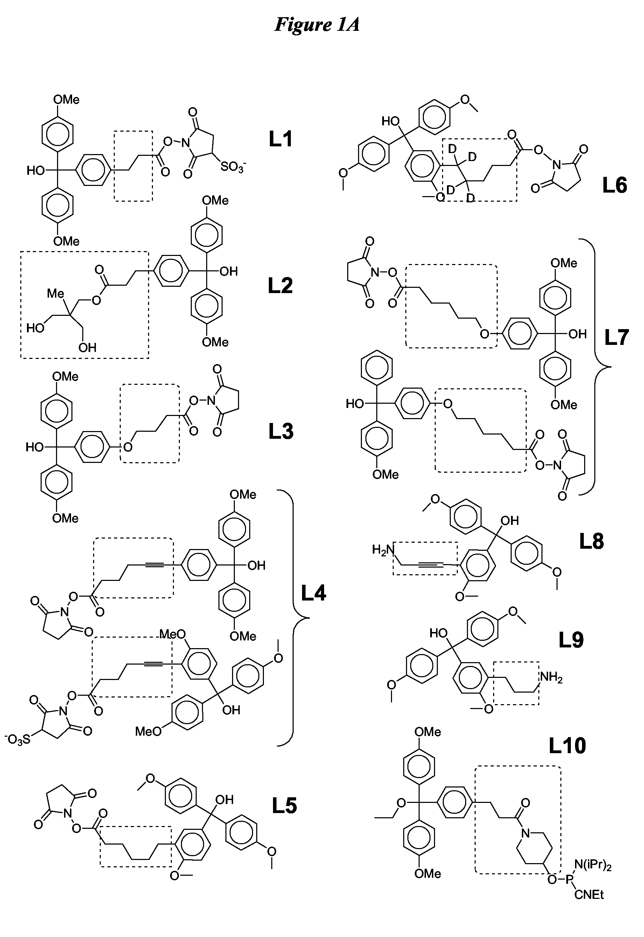

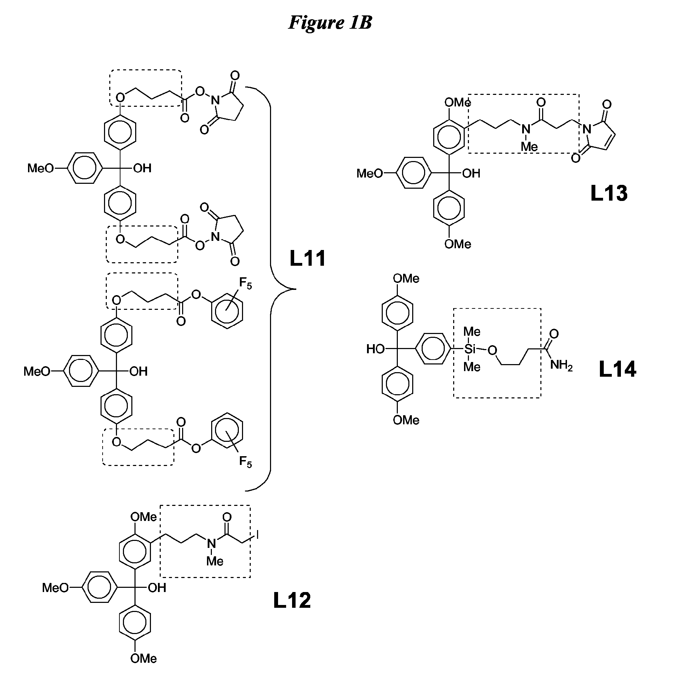

Preferred examples of L_{M} are shown in figures 1A and 1B.

### C--- Sₛ, Sₛ- - - Ar¹ and Sₛ - Ar² Bonds

C- - -Sₛ, Sₛ- - -Ar¹ and Sₛ- - -Ar² comprise a cleavable covalent, ionic, hydrogen, dipole-dipole or van der Waals bond (also known as a dispersion bond or a London forces bond). The covalent, ionic, hydrogen, dipole-dipole or van der Waals bond may be direct between C and Sₛ, Ar¹ and Sₛ, or Ar² and Sₛ, or may be provided by one or more binding groups present on C and/or Sₛ, Ar¹ and/or Sₛ, or Ar² and/or Sₛ, respectively.

### Covalent Bonding

Where the bond is covalent, the bond may be direct (*e.g.* C-Sₛ, Ar¹-Sₛ or Ar²-Sₛ, respectively) or may be provided by a linker atom or group L⁴ (*e.g.* C-L⁴-Sₛ, Ar¹-L⁴-Sₛ or Ar²-L⁴-Sₛ, respectively).

When L⁴ is a linker group, preferred linker groups are -E⁴-, -(D⁴)_{t"}-, -(E⁴-D⁴)_{t"}-, -(D⁴-E⁴)ₜ"-, - E⁴-(D⁴-E⁴)_{t"} or -D⁴(E⁴-D⁴)_{t"}.

D⁴ is independently C₁₋₈hydrocarbylene or C₁₋₈hydrocarbylene substituted with one or more A.

E⁴ is, in the orientation C-L⁴-Sₛ, independently -Z⁴-, -C(=Z⁴)-, -Z⁴C(=Z⁴)-, -C(=Z⁴)Z⁴-, -Z⁴C(=Z⁴)Z⁴-, -S(=O)-, -Z⁴S(=O)-, -S(=O)Z⁴-, -Z⁴S(=O)Z⁴-, -S(=O)₂-, -Z⁴S(=O)₂-, -S(=O)Z⁴-, -Z⁴S(=O)Z⁴-, where Z⁴ is independently O, S or N(R⁴), and where R⁴ is independently H, C₁₋₈hydrocarbyl (*e.g.* C₁₋₈alkyl) or C₁₋₈hydrocarbyl substituted with one or more A. Preferably E⁴ is, in the orientation C-L⁴-Sₛ, -O-, -S-, -C(=O)-, -C(=O)O-, -C(=S)-, -C(=S)O-, -OC(=S)-, -C(=O)S-, -SC(=O)-, -S(O)-, -S(O)₂-, -N(R⁴)- -C(=O)N(R⁴)-, -C(=S)N(R⁴)-, -N(R⁴)C(=O)-, -N(R⁴)C(=S)-, -S(=O)N(R⁴)-, -N(R⁴)S(=O)-, -S(=O)₂N(R⁴)-, -N(R⁴)S(=O)₂-, -OC(=O)O-, -SC(=O)O-, -OC(=O)S-, -N(R⁴)C(=O)O-, -OC(=O)N(R⁴)-, -N(R⁴)C(=O)N(R⁴)-, -N(R⁴)C(=S)N(R⁴)-, -N(R⁴)S(=O)N(R⁴)- or -N(R⁴)S(=O)₂N(R⁴)-.

t" = 1 or more, *e.g.* from 1 to 50, 1to 40, 1 to 30, 1 to 20 or 1 to 10. Preferably t" = 1, 2, 3, 4, 5, 6, 7, 8, 9, or 10.

Where L⁴ includes a group which also falls within the definition of group M, the group M is preferably more reactive than the group included in L⁵.

L⁴ is preferably a linker atom, preferably O or S, particularly O.

When the solid support Sₛ is gold, L⁴ is preferably covalently attached to the Sₛ by a sulphide or disulphide group.

### Ionic Bonding

Where the bond is ionic, the bond is typically direct (*e.g.* C 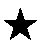 Sₛ 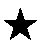 , where Sₛ 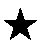 is a solid support counterion to C 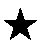 ).

Alternatively, it may be provided by binding groups, *e.g.* chelating ligands, present on C or Sₛ, Ar¹ or Sₛ, or Ar² or Sₛ, respectively. In the case of C- - -Sₛ bonds, the chelating ligand is typically only present on Sₛ and chelates with C 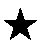 .

Suitable chelating ligands which can bind anions include polyamines and cryptands.

Suitable chelating ligands which can bind cations include polyacidic compounds (*e.g.* EDTA) and crown ethers.

### Hydrogen Bonding

Where the bond is a hydrogen bond, the bond is usually provided by binding groups present on C or Sₛ, Ar¹ or Sₛ, or Ar² or Sₛ, respectively.

Typically, in order to form the hydrogen bond, one of C or Sₛ, Ar¹ or Sₛ, or Ar² or Sₛ, as appropriate, will have a binding group bearing one or more hydroxy, amino or thio hydrogen atoms, and the other of C or Sₛ, Ar¹ or Sₛ, or Ar² or Sₛ, respectively, will have a binding group bearing an atom having one or more lone pair of electrons (*e.g.* an oxygen, sulphur or nitrogen atom). Preferably, one of C or Sₛ, Ar¹ or Sₛ, or Ar² or Sₛ, as appropriate, will have a binding group comprising biotin, and the other of C or Sₛ, Ar¹ or Sₛ, or Ar² or Sₛ, respectively, will have a binding group comprising avidin or streptavidin.

Alternatively, the hydrogen bond may be direct.

### Dipole-Dipole Bonding

Where the bond is a dipole-dipole bond, it may be formed between permanent dipoles or between a permanent dipole and an induced dipole.

Typically, in order to form the dipole-dipole bond, one of Sₛ and the compound of the invention has a permanent dipole and the other of Sₛ and the compound of the invention has an induced dipole or a permanent dipole, the attraction between the dipoles forming a dipole-dipole bond.

Preferably, Sₛ comprises binding groups (*e.g.* acid groups, -(NMe₃)⁺, carboxy, carboxylate, phosphate or sulphate groups) which produce a dipole at the surface of the solid support to bind the compound of the invention.

### Van der Waals Bonding

Where the bond is a van der Waals bond, the bonding is usually provided by binding groups present on C or Sₛ, Ar¹ or Sₛ, or Ar² or Sₛ, respectively.

Typically, in order to form the van der Waals bond, at least one, but preferably both, of C or Sₛ, Ar¹ or Sₛ, or Ar² or Sₛ, as appropriate, will have a hydrocarbyl or heterohydrocarbyl group (usually a large hydrocarbyl group having at least ten carbon atoms up to about 50 carbon atoms), optionally substituted with one or more A. Polyfluorinated hydrocarbyl and heterohydrocarbyl groups are particularly preferred. Typically, the hydrocarbyl or heterohydrocarbyl groups are aryl or heteroaryl groups or groups of the formula -C(R⁶)₂Ar³, -C(R⁶)(Ar³)₂ or -C(Ar³)₃, where Ar³ is independently defined the same as Ar² and R⁶ is H, C₁₋₈ hydrocarbyl, C₁₋₈ hydrocarbyl substituted by one or more A, C₁₋₈ heterohydrocarbyl or C₁₋₈ heterohydrocarbyl substituted by one or more A.

A preferred binding group is tetrabenzofullerene (formula X).

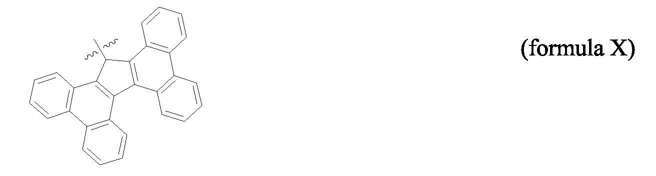

Alternatively, the van der Waals bond may be direct.

### Bond Cleavage

Preferably, the ions of formula (I') have a pKᵣ₊ value of at least *zz*, where *zz* is 0 or more (*e.g.* 0, 1, 2, 3, 4, 5, 6, 7, 8, 9, 10, 11, 12, 13, 14). More preferably, *zz* is 1 or more, still more preferably 2 or more, still more preferably 3 or more.

Preferably, the compounds of formula (IIa) or (IIb) or the solid supports of formula (IVai), (IVaii), (IVaiii), (IVbii), (IVbiii), (IVaiv) or (IVbiv) provide ions of formula (I') having a pKᵣ₊ value of at least *zz*, where *zz* is defined above.

### C-XBonds

The C-X bonds are cleavable by irradiation, electron bombardment, electrospray, fast atom bombardment (FAB), inductively coupled plasma (ICP) or chemical ionisation. Preferably, the C-X bonds are cleavable by irradiation or chemical ionisation.

The term 'irradiation' includes, for example, laser illumination, in particular as used in MALDI mass spectrometry. Laser light of about 340 nm is particularly preferred because it is typically used in MALDI mass spectrometers.

The term 'electron bombardment' includes, for example, bombardment with electrons having energy of about 70 ev.

Chemical ionisation can be effected, for example, by treatment with acid or acidic matrices (*e.g.* acidic matrices used in MALDI analysis).

Preferably group X is halogen, hydroxy, C₁₋₈hydrocarbyloxy, C₁₋₈hydrocarbyloxy substituted with one or more A, C₁₋₈heterohydrocarbyloxy, C₁₋₈heterohydrocarbyloxy substituted with one or more A, mesyl, tosyl, pentafluorophenyl, -O-succinimidyl -S-succinimidyl, or phenyloxy substituted with one or more A *e.g.* p-nitrophenyloxy. The groups pentafluorophenyl, -O-succinimidyl, - S-succinimidyl, and p-nitrophenyloxy are preferred.

Particularly preferred groups X are halogen, hydroxy, C₁₋₈hydrocarbyloxy. Especially preferred groups are hydroxy (*e.g*. compounds 61a & 62a), ethoxy (*e.g.* compound 62b) and chloro (*e.g.* compound 64b) groups.

Other preferred groups X are alkyl ethers, *e.g.* : 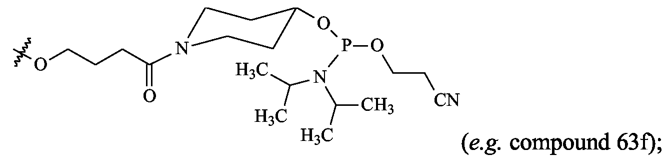 or

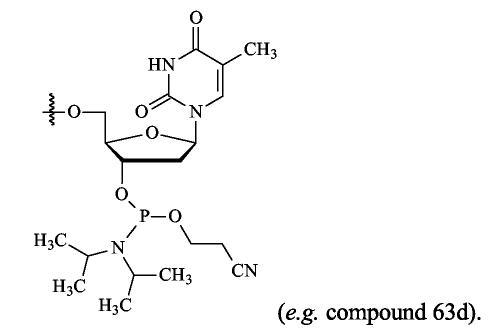

Group X may also be a -Q-oligonucleotide, where Q is O, S or N(R), where R is H, C₁₋₈hydrocarbyl or C₁₋₈hydrocarbyl substituted with one or more A. Q is preferably O.

Group X may also be a nucleoside, preferably where the nucleoside is bound via its 5' end, *e.g.* :

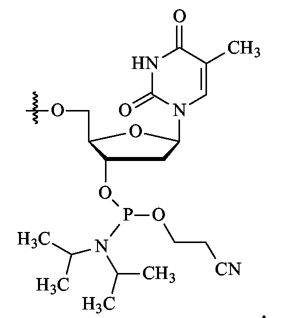

In some embodiments of the invention, X is not H. If X is H, preferably at least one of Ar¹ and Ar² is polycyclic, heterocyclic or unsubstituted.

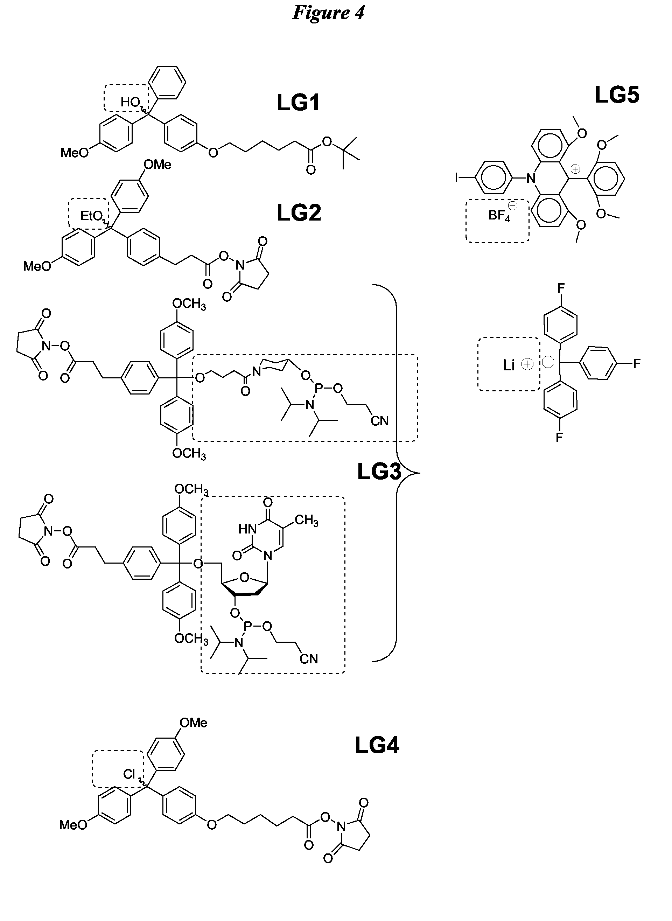

Preferred examples of group X are shown in figure 4.

*Ionic C* 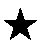 *X* 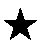 *Bonds*

X 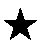 is any counterion for forming salts with compounds of the invention.

X 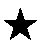 includes ions having single charges and multiple charges. Typically ions having multiple charges will be associated with an appropriate number of compounds of formula (IIb), (IVbii), (IVbiii) or (IVbiv), in order to balance the charge. Ions having multiple charges include doubly charged ions (*e.g.* SO₄²⁻) and triply charged ions. X 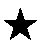 preferably has a single charge.

The counterion X 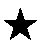 may be dissociated from the derivative of formula (IIb), (IVbii), (IVbiii), (IVbiv) or (Vbii) by irradiation, electron bombardment, electrospray, fast atom bombardment (FAB), inductively coupled plasma (ICP) or chemical ionisation. Preferably, the counterion X 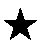 may be dissociated by irradiation.

When X 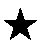 is a cation, X 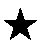 is preferably H⁺ or Li⁺, especially Li⁺.

When X 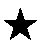 is an anion, X 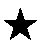 is preferably, BF₄⁻ or ClO₄⁻, especially BF₄⁻ (*e.g.* compounds 28b, 28c & 28d).

It is preferred that X 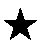 is an anion.

Preferred examples of group X 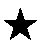 are shown in figure 4.

### C- - -Sₛ, Sₛ- - Ar¹ or Sₛ- - -Ar²

The C- - -Sₛ, Sₛ- - -Ar¹ or Sₛ- - -Ar² bonds are cleavable by irradiation, electron bombardment, electrospray, fast atom bombardment (FAB), inductively coupled plasma (ICP) or chemical ionisation. Preferably, the C- - -Sₛ, Sₛ- - Ar¹ or Sₛ- - -Ar² bonds are cleavable by irradiation or chemical ionisation.

Where appropriate, the C- - -Sₛ, Sₛ- - -Ar¹ or Sₛ- - -Ar² bonds may be cleaved simultaneously or sequentially with the cleaving of the C-X bond or the dissociation of X 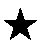 , as appropriate, by selection of suitable cleaving/dissociating conditions.

In one embodiment of the invention, the C- - -Sₛ bond in the solid support of formula (Vai) may be cleaved in sub-steps of step (iia) so that in a first sub-step a residue X (where X is the leaving group defined above) is provided and in a second subsequent sub-step the C-X bond is cleaved thereby forming the ion of formula (I). If desired, the second sub-step may be carried out substantially (*e.g.* seconds, minutes, hours or even days) after the first sub-step.

### Ar¹ and Ar²

### _{Ar}2

Ar² is independently an aromatic group or an aromatic group substituted with one or more A and is preferably independently cyclopropyl, cyclopropyl substituted with one or more A, aryl, aryl substituted with one or more A, heteroaryl, or heteroaryl substituted with one or more A.

Where aryl or substituted aryl, Ar² is preferably C₆₋₃₀ aryl or substituted C₆₋₃₀ aryl. Where heteroaryl or substituted heteroaryl, Ar² is preferably C₆₋₃₀ heteroaryl or substituted C₆₋₃₀ heteroaryl.

Examples of aryl and heteroaryl are monocyclic aromatic groups (*e*.*g*. phenyl or pyridyl), fused polycyclic aromatic groups (*e.g.* napthyl, such as 1-napthyl or 2-napthyl) and unfused polycyclic aromatic groups (*e.g.* monocyclic or fused polycyclic aromatic groups linked by a single bond, a double bond, or by a -(CH=CH)ᵣ- linking group, where r is one or more (*e.g.* 1, 2, 3, 4 or 5).

Other examples of aryl groups are monovalent derivatives of aceanthrylene, acenaphthylene, acephenanthrylene, anthracene, azulene, chrysene, coronene, fluoranthene, fluorene, *as*-indacene, *s*-indacene, indene, naphthalene, ovalene, perylene, phenalene, phenanthrene, picene, pleiadene, pyrene, pyranthrene and rubicene, which groups may be optionally substituted by one or more A.

Other examples of heteroaryl groups are monovalent derivatives of acridine, carbazole, β-carboline, chromene, cinnoline, furan, imidazole, indazole, indole, indolizine, isobenzofuran, isochromene, isoindole, isoquinoline, isothiazole, isoxazole, naphthyridine, perimidine, phenanthridine, phenanthroline, phenazine, phthalazine, purine, pyran, pyrazine, pyrazole, pyridazine, pyridine, pyrimidine, pyrrole, pyrrolizine, quinazoline, quinoline, quinolizine, quinoxaline, thiophene and xanthene, which groups may be optionally substituted by one or more A. Preferred heteroaryl groups are five- and six-membered monovalent derivatives, such as the monovalent derivatives of furan, imidazole, isothiazole, isoxazole, pyran, pyrazine, pyrazole, pyridazine, pyridine, pyrimidine, pyrrole, pyrrolizine and thiophene. The five-membered monovalent derivatives are particularly preferred, *i.e.* the monovalent derivatives of furan, imidazole, isothiazole, isoxazole, pyrazole, pyrrole and thiophene. The heteroaryl groups may be attached to the remainder of the compound by any carbon or hetero (*e.g.* nitrogen) atom.

Ar² is preferably C₆₋₃₀aryl substituted by one or more A, preferably phenyl or napthyl (*e.g.* 1-napthyl or 2-napthyl, especially 2-napthyl) substituted by one or more A, more preferably phenyl substituted by one or more A. When Ar² is phenyl, A is preferably provided in a position ortho or para to C 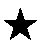 . When Ar² is other than phenyl, A is preferably attached to an atom which bears the charge in at least one of the resonance structures of the ions of formula (I).

Fused polycyclic aromatic groups, optionally substituted with one or more A, are particularly preferred.

A particularly preferred Ar² is unsubstituted pyrenyl or pyrenyl substituted with one or more A. Unsubstituted pyrenyl is preferred. The pyrenyl group may be 1-pyrenyl (*e.g.* compounds 38a, 38b, 39a, 41a & 41b), 2-pyrenyl (*e.g.* compounds 42a & 42b) or 4-pyrenyl (*e.g.* compounds 43a & 43b).

Preferred heteroaryl Ar² groups, whether substituted or unsubstituted, are pyridyl, pyrrolyl, thienyl and furyl, especially thienyl.

A preferred Ar² group is thiophenyl or thiophenyl substituted with one or more A. Unsubstituted thiophenyl is preferred. Examples of thiophenyl are thiophen-2-yl and thiophen-3-yl, with thiophen-2-yl being especially preferred (*e.g.* compounds 50a, 51a & 51b).

When substituted, Ar² is preferably substituted by 1, 2 or 3 A. Ar² is preferably:

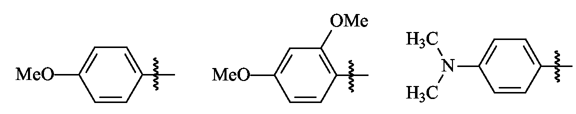

When unsubstituted, Ar² is preferably:

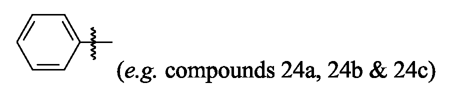

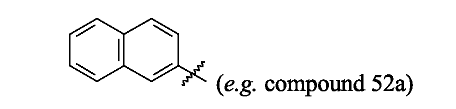

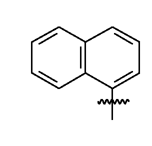

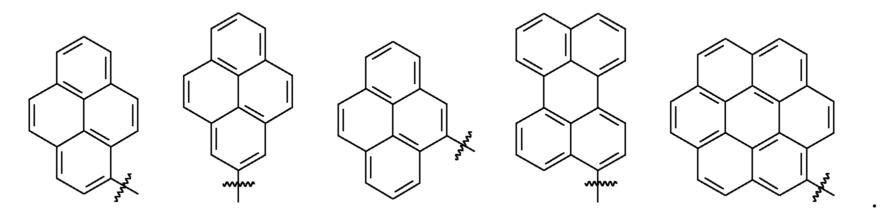

In another preferred embodiment, Ar² is cyclopropyl or cyclopropyl substituted with one or more A. Unsubstituted cyclopropyl is preferred (*e.g.* compound 44a). One or more, preferably one, of Ar² may be cyclopropyl.

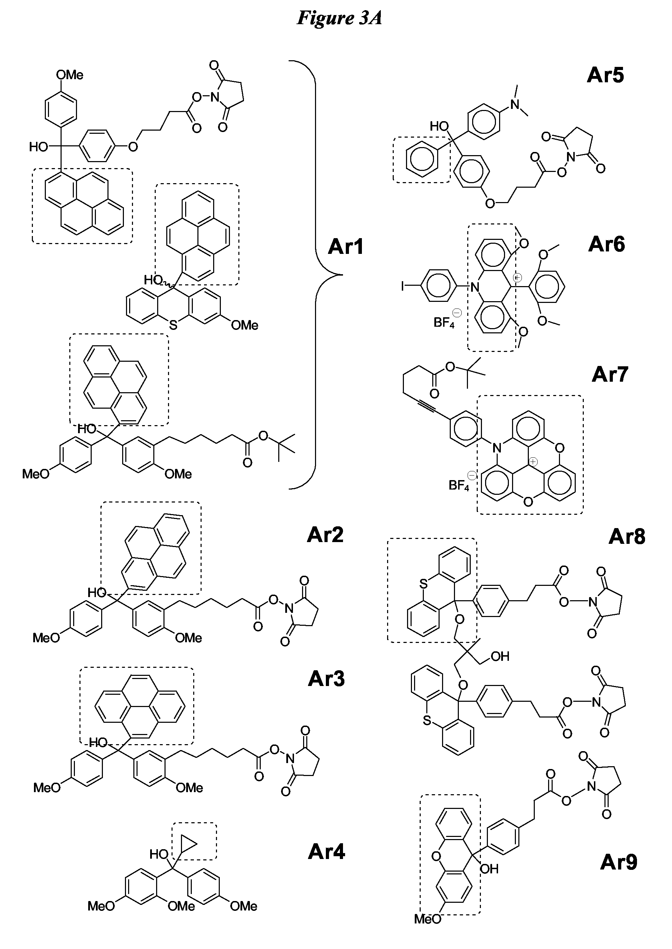

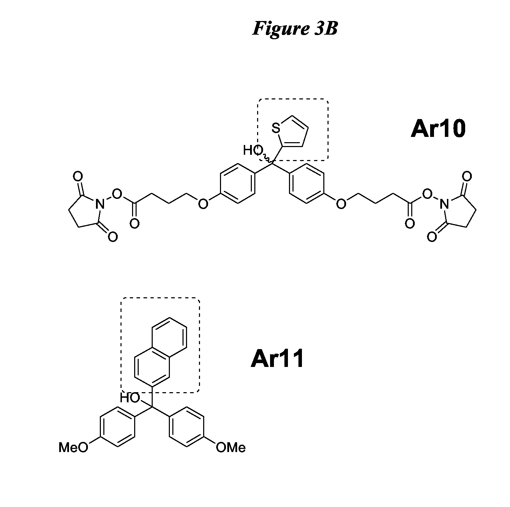

Preferred examples of group Ar² are shown in figures 3A and 3B.

### _{Ar}1

Ar¹ is independently an aromatic group or an aromatic group substituted with one or more A. The definition of Ar¹is the same as Ar² (as defined above), except that the valency of the group Ar¹ is adapted to accommodate the q instances of the linker L_{M}. Preferred Ar² groups are also preferred Ar¹ groups, (as defined above), except that the valency of the group Ar¹ is adapted to accommodate the q instances of the linker L_{M}.

When q = 1, Ar¹ is a divalent radical and is preferably independently cyclopropylene, cyclopropylene substituted with one or more A, arylene, arylene substituted with one or more A, heteroarylene, or heteroarylene substituted with one or more A.

Where arylene or substituted arylene, Ar¹ is preferably C₆₋₃₀ arylene or substituted C₆₋₃₀ arylene. Where heteroarylene or substituted heteroarylene, Ar¹ is preferably C₆₋₃₀ heteroarylene or substituted C₆₋₃₀ heteroarylene.

Examples of arylene and heteroarylene are monocyclic aromatic groups (*e.g.* phenylene or pyridylene), fused polycyclic aromatic groups (*e.g.* napthylene) and unfused polycyclic aromatic groups (*e.g.* monocyclic or fused polycyclic aromatic groups linked by a single bond, a double bond, or by a -(CH=CH)ᵣ- linking group, where r is one or more (*e.g.* 1, 2, 3, 4 or 5).

Other examples of arylene groups are polyvalent derivatives (where the valency is adapted to accommodate the q instances of the linker L_{M}) of aceanthrylene, acenaphthylene, acephenanthrylene, anthracene, azulene, chrysene, coronene, fluoranthene, fluorene, *as*-indacene, *s*-indacene, indene, naphthalene, ovalene, perylene, phenalene, phenanthrene, picene, pleiadene, pyrene, pyranthrene and rubicene, which groups may be optionally substituted by one or more A.

Other examples of heteroarylene groups are polyvalent derivatives (where the valency is adapted to accommodate the q instances of the linker L_{M}) of acridine, carbazole, β-carboline, chromene, cinnoline, furan, imidazole, indazole, indole, indolizine, isobenzofuran, isochromene, isoindole, isoquinoline, isothiazole, isoxazole, naphthyridine, perimidine, phenanthridine, phenanthroline, phenazine, phthalazine, purine, pyran, pyrazine, pyrazole, pyridazine, pyridine, pyrimidine, pyrrole, pyrrolizine, quinazoline, quinoline, quinolizine, quinoxaline, thiophene and xanthene, which groups may be optionally substituted by one or more A. Preferred heteroaryl groups are five- and six-membered polyvalent derivatives, such as the polyvalent derivatives of furan, imidazole, isothiazole, isoxazole, pyran, pyrazine, pyrazole, pyridazine, pyridine, pyrimidine, pyrrole, pyrrolizine and thiophene. The five-membered polyvalent derivatives are particularly preferred, *i.e.* the polyvalent derivatives of furan, imidazole, isothiazole, isoxazole, pyrazole, pyrrole and thiophene. The heteroaryl groups may be attached to the remainder of the compound by any carbon or hetero (*e.g.* nitrogen) atom.

Ar¹ is preferably C₆₋₃₀arylene substituted by one or more A, preferably phenylene or napthylene substituted by one or more A, more preferably phenylene substituted by one or more A. When Ar¹ is phenylene, A is preferably provided in a position ortho or para to C 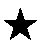 . When Ar¹ is other than phenylene, A is preferably attached to an atom which bears the charge in at least one of the resonance structures of the ions of formula (I).

When substituted, Ar¹ is preferably substituted by 1, 2 or 3 A.

When unsubstituted, preferred Ar¹ are:

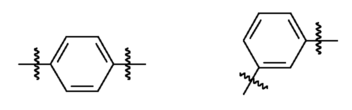

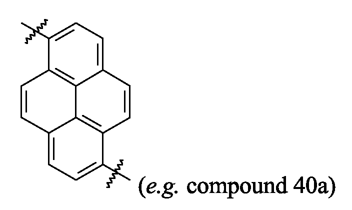

Preferred examples of group Ar¹ are shown in figures 3A and 3B.

### Combinations of Ar

Optionally two or three of the groups Ar¹ and Ar² are linked together by one or more L⁵, where L⁵ is independently a single bond or a linker atom or group; and/or two or three of the groups Ar¹ and Ar² together form an aromatic group or an aromatic group substituted with one or more A.

When L⁵ is a linker group, preferred linker groups are -E⁵-, -(D⁵)_{t'}-, -(E⁵-D⁵)_{t'}-, -(D⁵-E⁵)_{t'}-, -E⁵-(D⁵-E⁵)_{t'}- or -D⁵-(E⁵-D⁵)_{t'}-.

D⁵ is independently C₁₋₈hydrocarbylene or C₁₋₈hydrocarbylene substituted with one or more A. E⁵ is independently -Z⁵-, -C(=Z⁵)-, -Z⁵C(=Z⁵)-, -C(=Z⁵)Z⁵-, -Z⁵C(=Z⁵)Z⁵-, -S(=O)-, -Z⁵S(=O)-, -S(=O)Z⁵-, -Z⁵S(=O)Z⁵-, -S(=O)₂-, -Z⁵S(=O)₂-, -S(=O)₂Z⁵-, -Z⁵S(=O)₂Z⁵-, where Z⁵ is independently O, S or N(R⁵) and where R⁵ is independently H, C₁₋₈hydrocarbyl or C₁₋₈hydrocarbyl substituted with one or more A. Preferably E⁵ is -O-, -S-, -C(=O)-, -C(=O)O-, -C(=S)-, -C(=S)O-, -OC(=S)-, -C=O)S-, -SC(=O)-, -S(O)-, -S(O)₂-, -N(R⁵)-, -C(=O)N(R⁵)-, -C(=S)N(R⁵)-, -N(R⁵)C(=O)-, -N(R⁵)C(=S)-, -S(=O)N(R⁵)-, -N(R⁵)S(=O)-, -S(=O)₂N(R⁵)-, -N(R⁵)S(=O)₂-, -OC(=O)O-, -SC(=O)O-, -OC(=O)S-, -N(R⁵)C(=O)O-, -OC(=O)N(R⁵)-, -N(R⁵)C(=O)N(R⁵)-, -N(R⁵)C(=S)N(R⁵)-, - N(R⁵)S(=O)N(R⁵)- or -N(R⁵)S(=O)₂N(R⁵)-.

t' = 1 or more, *e.g.* from 1 to 50, 1to 40, 1 to 30, 1 to 20 or 1 to 10. Preferably t' = 1, 2, 3, 4, 5, 6, 7, 8, 9, or 10. Most preferably t'=1.

Where L⁵ includes an atom or group which also falls within the definition of group M, the group M is preferably more reactive than the group included in L⁵.

L⁵ is preferably a linker atom, preferably O or S, particularly O.

When L⁵ is a linker group, a preferred L⁵ is -N(R⁵)-.

In another embodiment in which L⁵ is a linker group, L⁵ is -S(=O)- (*e.g.* compound 56b)

When two of the groups Ar¹ and Ar² are linked together by one or more (*e.g.* 2, 3 or 4) L⁵, they are preferably linked together by one L⁵, preferably O.

Preferred combinations of Ar are two Ar² (*e.g.* two Ar² phenyl groups) linked together by one L⁵ (*e.g.* O or S).

Particularly preferred combinations of Ar are two Ar² phenyl groups, optionally substituted by one or more A (preferably unsubstituted), linked together by one L⁵ (*e.g.* O or S), where is L⁵ is ortho to C 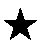 with respect to both phenyl groups. Especially preferred combinations of two Ar² groups are: 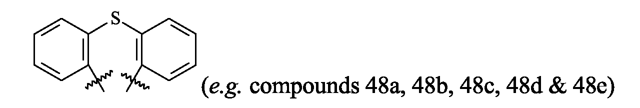 and

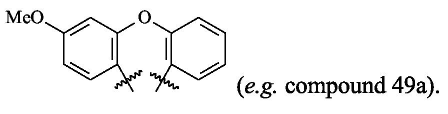

In another embodiment, at least one L_{M} is linked to an atom or group L⁵. In this embodiment, the preferred L⁵ mentioned above are, where appropriate, modified to remove substituents R⁵ in order to accommodate L_{M}, *e*.*g*. the R⁵ substituent of the group -N(R⁵)- is replaced by L_{M}. In this embodiment, the L⁵ group to which L_{M} is bound is preferably:

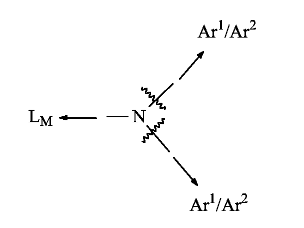

Preferred combinations of Ar¹ and/or Ar² in this embodiment are:

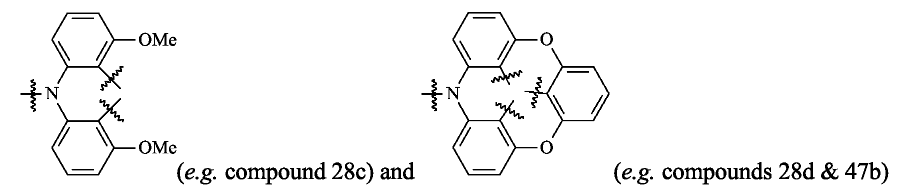

When two or three of the groups Ar¹ and Ar² together form an aromatic group or an aromatic group substituted with one or more A, the aromatic group may be a carbocyclic aromatic group or a carbocyclic aromatic group in which one or more carbon atoms are each replaced by a hetero atom. Typically, in an aromatic group in which one or more carbon atoms are each replaced by a hetero atom, up to three carbons are so replaced, preferably up to two carbon atoms, more preferably one carbon atom.

Preferred hetero atoms are O, Se, S or N, more preferably O, S or N.

When two or three of the groups Ar¹ and Ar² together form an aromatic group or an aromatic group substituted with one or more A, preferred aromatic groups are C₈₋₅₀ aromatic groups.

The aromatic groups may be monocyclic aromatic groups (*e.g.* radicals of suitable valency derived from benzene), fused polycyclic aromatic groups (*e.g.* radicals of suitable valency derived from napthalene) and unfused polycyclic aromatic groups (*e.g.* monocyclic or fused polycyclic aromatic groups linked by a single bond, a double bond, or by a -(CH=CH)ᵣ- linking group, where r is one or more (*e.g.* 1, 2, 3, 4 or 5).

When two or three of the groups Ar¹ and Ar² together form a carbopolycyclic fused ring aromatic group, preferred groups are radicals of suitable valency obtained from napthalene, anthracene or phenanthracene, chrysene, aceanthrylene, acenaphthylene, acephenanthrylene, azulene, fluoranthene, fluorene, *as*-indacene, *s*-indacene, indene, phenalene, and pleiadene.

When two or three of the groups Ar¹ and Ar² together form a carbopolycyclic fused ring aromatic group in which one or more carbon atoms are each replaced by a hetero atom, preferred groups are radicals of suitable polyvalency obtained from acridine, carbazole, β-carboline, chromene, cinnoline, indole, indolizine, isobenzofuran, isochromene, isoindole, isoquinoline, naphthyridine, perimidine, phenanthridine, phenanthroline, phenazine, phthalazine, pteridine, purine, pyrrolizine, quinazoline, quinoline, quinolizine and quinoxaline.

### Substitution of Ar¹ and Ar² - Anions and Cations

When C 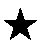 is a cation, A is preferably an electron-donating group, including -R¹ or -Z¹R¹, where R¹ and Z¹ are defined below. Preferably, R¹ is C₁₋₈hydrocarbyl, more preferably C₁₋₈alkyl, especially methyl. Z¹ is preferably O, S or NR¹. R¹ may be substituted with one or more S_{ub}², but is preferably unsubstituted. When C 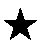 is a cation, A is preferably -OMe (*e.g.* compound 55a), -SMe (*e.g.* compounds 53a, 53b, 53c, 53d & 53e), -N(Me)₂ (*e.g.* compounds 54a, 54b & 54c) or Me (*e.g.* compound 58a). When C 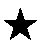 is a cation, A, when an electron-donating group, is preferably provided (especially in relation to Ar¹ or Ar² being phenyl) in a position ortho or para to C 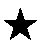 , preferably para. Furthermore, when C 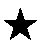 is a cation, A, when an electron-withdrawing group (*e.g.* F (*e.g.* compound 57a)), is preferably provided (especially in relation to Ar¹ or Ar² being phenyl) in a position meta to C 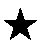 . Thus, preferred groups Ar¹ and Ar² are as follows:

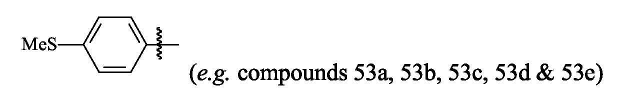

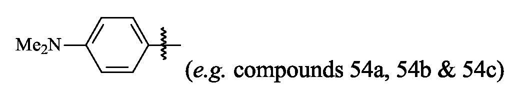

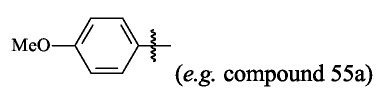

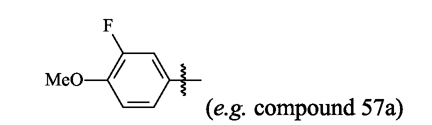

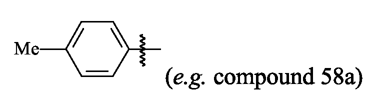

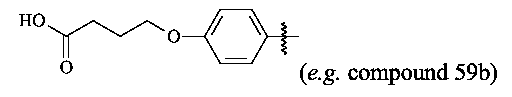

When C 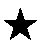 is an anion, A is preferably an electron-withdrawing group, including halogen, trihalomethyl, -NO₂, -CN, -N⁺(R¹)₂O⁻, -CO₂H, -CO₂R¹, -SO₃H, -SOR¹, -SO₂R¹, -SO₃R¹, -OC(=O)OR¹, -C(=O)H, -C(=O)R¹, -OC(=O)R¹, -C(=O)NH₂, -C(=O)NR¹₂, -N(R¹)C(=O)OR¹, -N(R¹)C(=O)NR¹₂, -OC(=O)NR¹₂, -N(R¹)C(=O)R¹, -C(=S)NR¹₂, -NR¹C(=S)R¹, -SO₂NR¹₂, -NR¹SO₂R¹, -N(R¹)C(=S)NR¹₂, or -N(R¹)SO₂NR¹₂, where R¹ is defined below. When C 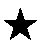 is an anion, A, when an electron-withdrawing group, is preferably provided (especially in relation to Ar¹ or Ar² being phenyl) in a position ortho or para to C 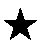 , preferably para. Furthermore, when C 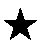 is an anion, A, when an electron-donating group, is preferably provided (especially in relation to Ar¹ or Ar² being phenyl) in a position meta to C 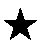 .

The group A may also comprise one or more isotopes of the atoms making up group A (*e.g.* example 60), thus, as discussed in more detail below, allowing the masses of the compounds of the invention to be varied. Preferred isotopes are ¹³C, ¹⁸O and ²H. When providing a series of compounds which differ only in their masses, ¹³C and ¹⁸O are particularly preferred as ²H atoms may cause a substantial change in the chemical properties of the compound due to the kinetic isotope effect.

### Solid Supports

'Solid supports' for use with the invention include polymer beads, metals, resins, columns, surfaces (including porous surfaces) and plates (*e.g.* mass-spectrometry plates).

The solid support is preferably one suitable for use in a mass spectrometer, such that the invention can be conveniently accommodated into existing MS apparatus. Ionisation plates from mass spectrometers are thus preferred solid supports, *e.g.* gold, glass-coated or plastic-coated plates. Solid gold supports are particularly preferred.

### Varying the mass of compounds of the invention

Within the general formulae (I), (IIa), (IIb), (IIIa), (IIIb), (IVai), (IVaii), (IVaiii), (IVaiv), (IVbii), (IVbiii), (IVbiv), (Vai), (Vaii), (Vaiii), (Vaiv), (Vbii), (Vbiii) and (Vbiv), there is much scope for variation. There is thus much scope of variation in the mass of these compounds.

The masses of the compounds of the invention can be varied via L_{M}, Ar¹ and/or Ar². Preferably, the masses of the compounds of the invention are varied by varying A on the groups Ar¹ and/or Ar².

In this aspect of invention, compounds of the invention advantageously comprise one or more of F or I as substituents A of the groups Ar¹, Ar² or Ar³. F and I each only have one naturally occurring isotope, ¹⁹F and ¹²⁷I respectively, and thus by varying the number of F and I atoms present in the structure of the compounds, can provide a series of molecular mass labels having substantially identical shaped peaks on a mass spectrum.

Compounds of the invention may also include one or more ²H atoms, preferably as a substituent A or a part thereof of the groups L_{M}, Ar¹, Ar² or Ar³ (in particular L_{M}), in order to vary the masses of the compounds of the invention. The compounds of the invention may include isotopes of ¹³C and ¹⁸O, prefererably as a substituent A or a part thereof of the groups L_{M}, Ar¹, Ar² or Ar³ (in particular Ar¹, Ar² or Ar³), in order to vary the masses of the compounds of the invention. Compounds comprising ²H, ¹³C and ¹⁸O may also be used to provide a series of molecular mass labels having substantially identical shaped peaks on a mass spectrum, by varing the number of ²H, ¹³C and ¹⁸O atoms present in the structure of the compounds. When providing a series of compounds which differ only in their masses, ¹³C and ¹⁸O are particularly preferred as ²H atoms may cause a substantial change in the chemical properties of the compound due to the kinetic isotope effect.

In order to increase the molecular mass of the compounds of the invention and to increase the number of available sites for substitution by A, especially F and I, one or more of Ar¹ and Ar² may be substituted by one or more dendrimer radicals of appropriate valency, either as substituent A or group L_{M}.

Preferred dendrimer radicals are the radicals obtained from the dendrimers of US 6,455,071 and PAMAM dendrimers.

### Chemical Groups

The ions of the invention are stabilised by the resonance effect of the aromatic groups Ar¹ and Ar². The term 'C 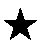 is a carbon atom bearing a single positive charge or a single negative charge' therefore not only includes structures having the charge localised on the carbon atom but also resonance structures in which the charge is delocalised from the carbon atom.

The term 'linker atom or group' includes any divalent atom or divalent group.

The term 'aromatic group' includes quasi and/or pseudo-aromatic groups, *e.g.* cyclopro pyl and cyclopropylene groups.

The term 'halogen' includes fluorine, chlorine, bromine and iodine.

The term 'hydrocarbyl' includes linear, branched or cyclic monovalent groups consisting of carbon and hydrogen. Hydrocarbyl groups thus include alkyl, alkenyl and alkynyl groups, cycloalkyl (including polycycloalkyl), cycloalkenyl and aryl groups and combinations thereof, *e.g.* alkylcycloalkyl, alkylpolycycloalkyl, alkylaryl, alkenylaryl, cycloalkylaryl, cycloalkenylaryl, cycloalkylalkyl, polycycloalkylalkyl, arylalkyl, arylalkenyl, arylcycloalkyl and arylcycloalkenyl groups. Preferred hydrocarbyl are C₁₋₁₄ hydrocarbyl, more preferably C₁₋₈ hydrocarbyl.

Unless indicated explicitly otherwise, where combinations of groups are referred to herein as one moiety, *e.g.* arylalkyl, the last mentioned group contains the atom by which the moiety is attached to the rest of the molecule.

The term 'hydrocarbylene' includes linear, branched or cyclic divalent groups consisting of carbon and hydrogen formally made by the removal of two hydrogen atoms from the same or different (preferably different) skeletal atoms of the group. Hydrocarbylene groups thus include alkylene, alkenylene and alkynylene groups, cycloalkylene (including polycycloalkylene), cycloalkenylene and arylene groups and combinations thereof, *e.g.* alkylenecycloalkylene, alkylenepolycycloalkylene, alkylenearylene, alkenylenearylene, cycloalkylenealkylene, polycycloalkylenealkylene, arylenealkylene and arylenealkenylene groups. Preferred hydrocarbylene are C₁₋₁₄ hydrocarbylene, more preferably C₁₋₈ hydrocarbylene.

The term 'hydrocarbyloxy' means hydrocarbyl-O-.

The terms 'alkyl', 'alkylene', 'alkenyl', 'alkenylene', 'alkynyl', or 'alkynylene' are used herein to refer to both straight, cyclic and branched chain forms. Cyclic groups include C₃₋₈ groups, preferably C₅₋₈ groups.

The term 'alkyl' includes monovalent saturated hydrocarbyl groups. Preferred alkyl are C₁₋₈, more preferably C₁₋₄ alkyl such as methyl, ethyl, n-propyl, i-propyl or t-butyl groups.

Preferred cycloalkyl are C₅₋₈ cycloalkyl.

The term 'alkoxy' means alkyl-O-.

The term 'alkenyl' includes monovalent hydrocarbyl groups having at least one carbon-carbon double bond and preferably no carbon-carbon triple bonds. Preferred alkenyl are C₂₋₄ alkenyl.

The term 'alkynyl' includes monovalent hydrocarbyl groups having at least one carbon-carbon triple bond and preferably no carbon-carbon double bonds. Preferred alkynyl are C₂₋₄ alkynyl.

The term 'aryl' includes monovalent aromatic groups, such as phenyl or naphthyl. In general, the aryl groups may be monocyclic or polycyclic fused ring aromatic groups. Preferred aryl are C₆-C₁₄aryl.

Other examples of aryl groups are monovalent derivatives of aceanthrylene, acenaphthylene, acephenanthrylene, anthracene, azulene, chrysene, coronene, fluoranthene, fluorene, *as*-indacene, *s*-indacene, indene, naphthalene, ovalene, perylene, phenalene, phenanthrene, picene, pleiadene, pyrene, pyranthrene and rubicene.

The term 'alkylene' includes divalent saturated hydrocarbylene groups. Preferred alkylene are C₁₋₄ alkylene such as methylene, ethylene, n-propylene, i-propylene or t-butylene groups.

Preferred cycloalkylene are C₅₋₈ cycloalkylene.

The term 'alkenylene' includes divalent hydrocarbylene groups having at least one carbon-carbon double bond and preferably no carbon-carbon triple bonds. Preferred alkenylene are C₂₋₄ alkenylene.

The term 'alkynylene' includes divalent hydrocarbylene groups having at least one carbon-carbon triple bond and preferably no carbon-carbon double bonds. Preferred alkynylene are C₂₋₄ alkynylene.

The term 'arylene' includes divalent aromatic groups, such phenylene or naphthylene. In general, the arylene groups may be monocyclic or polycyclic fused ring aromatic groups. Preferred arylene are C₆-C₁₄arylene.

Other examples of arylene groups are divalent derivatives of aceanthrylene, acenaphthylene, acephenanthrylene, anthracene, azulene, chrysene, coronene, fluoranthene, fluorene, *as*-indacene, *s*-indacene, indene, naphthalene, ovalene, perylene, phenalene, phenanthrene, picene, pleiadene, pyrene, pyranthrene and rubicene.

The term 'heterohydrocarbyl' includes hydrocarbyl groups in which up to three carbon atoms, preferably up to two carbon atoms, more preferably one carbon atom, are each replaced independently by O, S, Se or N, preferably O, S or N. Heterohydrocarbyl groups thus include heteroalkyl, heteroalkenyl and heteroalkynyl groups, cycloheteroalkyl (including polycycloheteroalkyl), cycloheteroalkenyl and heteroaryl groups and combinations thereof, *e.g.* heteroalkylcycloalkyl, alkylcycloheteroalkyl, heteroalkylpolycycloalkyl, alkylpolycycloheteroalkyl, heteroalkylaryl, alkylheteroaryl, heteroalkenylaryl, alkenylheteroaryl, cycloheteroalkylaryl, cycloalkylheteroaryl, heterocycloalkenylaryl, cycloalkenylheteroaryl, cycloalkylheteroalkyl, cycloheteroalkylalkyl, polycycloalkylheteroalkyl, polycycloheteroalkylalkyl, arylheteroalkyl, heteroarylalkyl, arylheteroalkenyl, heteroarylalkenyl, arylcycloheteroalkyl, heteroarylcycloalkyl, arylheterocycloalkenyl and heteroarylcycloalkenyl groups. The heterohydrocarbyl groups may be attached to the remainder of the compound by any carbon or hetero (*e.g.* nitrogen) atom.

The term 'heterohydrocarbylene' includes hydrocarbylene groups in which up to three carbon atoms, preferably up to two carbon atoms, more preferably one carbon atom, are each replaced independently by O, S, Se or N, preferably O, S or N. Heterohydrocarbylene groups thus include heteroalkylene, heteroalkenylene and heteroalkynylene groups, cycloheteroalkylene (including polycycloheteroalkylene), cycloheteroalkenylene and heteroarylene groups and combinations thereof, *e.g.* heteroalkylenecycloalkylene, alkylenecycloheteroalkylene, heteroalkylenepolycycloalkylene, alkylenepolycycloheteroalkylene, heteroalkylenearylene, alkyleneheteroarylene, heteroalkenylenearylene, alkenyleneheteroarylene, cycloalkyleneheteroalkylene, cycloheteroalkylenealkylene, polycycloalkyleneheteroalkylene, polycycloheteroalkylenealkylene, aryleneheteroalkylene, heteroarylenealkylene, aryleneheteroalkenylene, heteroarylenealkenylene groups. The heterohydrocarbylene groups may be attached to the remainder of the compound by any carbon or hetero (*e.g.* nitrogen) atom.

Where reference is made to a carbon atom of a hydrocarbyl or other group being replaced by an O, S, Se or N atom, what is intended is that: 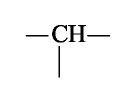 is replaced by 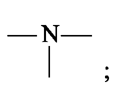 -CH= is replaced by -N=; or
-CH₂- is replaced by -O-, -S- or -Se-.

The term 'heteroalkyl' includes alkyl groups in which up to three carbon atoms, preferably up to two carbon atoms, more preferably one carbon atom, are each replaced independently by O, S, Se or N, preferably O, S or N.

The term 'heteroalkenyl' includes alkenyl groups in which up to three carbon atoms, preferably up to two carbon atoms, more preferably one carbon atom, are each replaced independently by O, S, Se or N, preferably O, S or N.

The term 'heteroalkynyl' includes alkynyl groups in which up to three carbon atoms, preferably up to two carbon atoms, more preferably one carbon atom, are each replaced independently by O, S, Se or N, preferably O, S or N.

The term 'heteroaryl' includes aryl groups in which up to three carbon atoms, preferably up to two carbon atoms, more preferably one carbon atom, are each replaced independently by O, S, Se or N, preferably O, S or N. Preferred heteroaryl are C₅₋₁₄heteroaryl. Examples of heteroaryl are pyridyl, pyrrolyl, thienyl or furyl.

Other examples of heteroaryl groups are monovalent derivatives of acridine, carbazole, β-carboline, chromene, cinnoline, furan, imidazole, indazole, indole, indolizine, isobenzofuran, isochromene, isoindole, isoquinoline, isothiazole, isoxazole, naphthyridine, perimidine, phenanthridine, phenanthroline, phenazine, phthalazine, purine, pyran, pyrazine, pyrazole, pyridazine, pyridine, pyrimidine, pyrrole, pyrrolizine, quinazoline, quinoline, quinolizine, quinoxaline, thiophene and xanthene. Preferred heteroaryl groups are five- and six-membered monovalent derivatives, such as the monovalent derivatives of furan, imidazole, isothiazole, isoxazole, pyran, pyrazine, pyrazole, pyridazine, pyridine, pyrimidine, pyrrole, pyrrolizine and thiophene. The five-membered monovalent derivatives are particularly preferred, *i.e.* the monovalent derivatives of furan, imidazole, isothiazole, isoxazole, pyrazole, pyrrole and thiophene.

The term 'heteroalkylene' includes alkylene groups in which up to three carbon atoms, preferably up to two carbon atoms, more preferably one carbon atom, are each replaced independently by O, S, Se or N, preferably O, S or N.

The term 'heteroalkenylene' includes alkenylene groups in which up to three carbon atoms, preferably up to two carbon atoms, more preferably one carbon atom, are each replaced independently by O, S, Se or N, preferably O, S or N.

The term 'heteroalkynylene' include alkynylene groups in which up to three carbon atoms, preferably up to two carbon atoms, more preferably one carbon atom, are each replaced independently by O, S, Se or N, preferably O, S or N.

The term 'heteroarylene' includes arylene groups in which up to three carbon atoms, preferably up to two carbon atoms, more preferably one carbon atom, are each replaced independently by O, S, Se or N, preferably O, S or N. Preferred heteroarylene are C₅₋₁₄heteroarylene. Examples of heteroarylene are pyridylene, pyrrolylene, thienylene or furylene.

Other examples of heteroarylene groups are divalent derivatives (where the valency is adapted to accommodate the q instances of the linker L_{M}) of acridine, carbazole, β-carboline, chromene, cinnoline, furan, imidazole, indazole, indole, indolizine, isobenzofuran, isochromene, isoindole, isoquinoline, isothiazole, isoxazole, naphthyridine, perimidine, phenanthridine, phenanthroline, phenazine, phthalazine, purine, pyran, pyrazine, pyrazole, pyridazine, pyridine, pyrimidine, pyrrole, pyrrolizine, quinazoline, quinoline, quinolizine, quinoxaline, thiophene and xanthene. Preferred heteroarylene groups are five- and six-membered divalent derivatives, such as the divalent derivatives of furan, imidazole, isothiazole, isoxazole, pyran, pyrazine, pyrazole, pyridazine, pyridine, pyrimidine, pyrrole, pyrrolizine and thiophene. The five-membered divalent derivatives are particularly preferred, *i.e.* the divalent derivatives of furan, imidazole, isothiazole, isoxazole, pyrazole, pyrrole and thiophene.

### Substitution

A is independently a substituent, preferably a substituent S_{ub}¹. Alternatively, A may be ²H.

S_{ub}¹ is independently halogen, trihalomethyl, -NO₂, -CN, -N⁺(R¹)₂O⁻, -CO₂H, -CO₂R¹, -SO₃H, -SOR¹, -SO₂R¹, -SO₃R¹, -OC(=O)OR¹, -C(=O)H, -C(=O)R¹, -OC(=O)R¹, -NR¹₂, -C(=O)NH₂, -C(=O)NR¹₂, - N(R¹)C(=O)OR¹, -N(R¹)C(=O)NR¹₂, -OC(=O)NR¹₂, -N(R¹)C(=O)R¹, -C(=S)NR¹₂, -NR¹C(=S)R¹, -SO₂NR¹₂, -NR¹SO₂R¹, -N(R¹)C(=S)NR¹₂, -N(R¹)SO₂NR¹₂, -R¹ or -Z¹R¹. Z¹ is O, S, Se or NR¹.

R¹ is independently H, C₁₋₈hydrocarbyl, C₁₋₈hydrocarbyl substituted with one or more S_{ub}², C₁₋₈heterohydrocarbyl or C₁₋₈heterohydrocarbyl substituted with one or more S_{ub}².

S_{ub}² is independently halogen, trihalomethyl, -NO₂, -CN, -N⁺(C₁₋₆alkyl)₂O⁻, -CO₂H, -CO₂C₁₋₆alkyl, -SO₃H, -SOC₁₋₆alkyl, -SO₂C₁₋₆alkyl, -SO₃C₁₋₆alkyl, -OC(=O)OC₁₋₆alkyl, -C(=O)H, -C(=O)C₁₋₆alkyl, -OC(=O)C₁₋₆alkyl, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -C(=S)N(C₁₋₆alkyl)₂, -N(C₁₋₆alkyl)C(=S)C₁₋₆alkyl, -SO₂N(C₁₋₆alkyl)₂, -N(C₁₋₆alkyl)SO₂C₁₋₆alkyl, -N(C₁₋₆alkyl)C(=S)N(C₁₋₆alkyl)₂, -N(C₁₋₆alkyl)SO₂N(C₁₋₆alkyl)₂, C₁₋₆alkyl or -Z¹C₁₋₆alkyl.

Where reference is made to a substituted group, the substituents are preferably from 1 to 5 in number, most preferably 1.

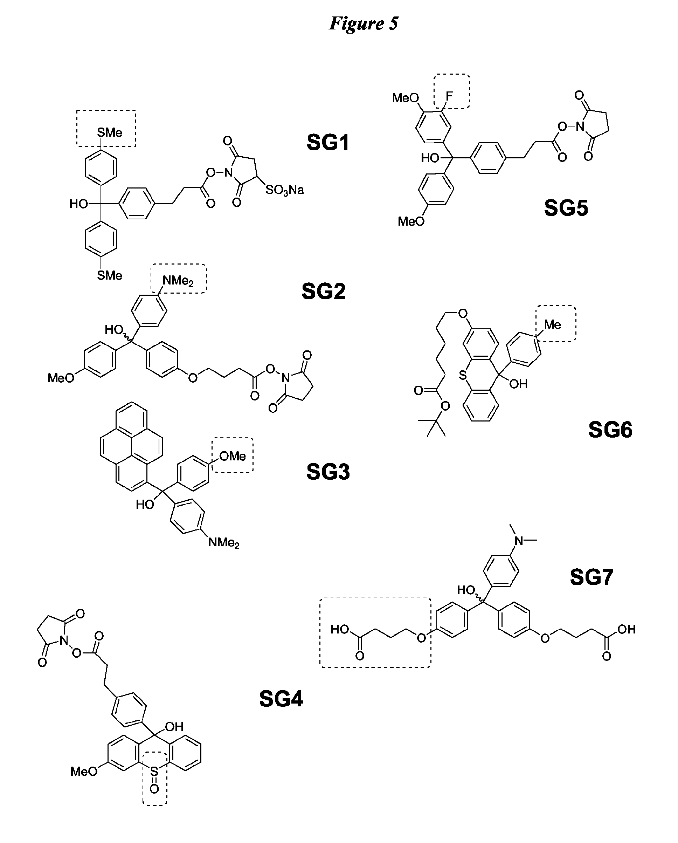

Preferred examples of substituent group A are shown in figure 5.

### Miscellaneous

A may optionally be a monovalent dendrimer radical or a monovalent dendrimer radical substituted with one or more substituents S_{ub}¹.

### General

The term "comprising" means "including" as well as "consisting" *e.g.* a composition "comprising" X may consist exclusively of X or may include something additional *e.g.* X + Y.

The term "about" in relation to a numerical value x means, for example, *x*±10%.

The word "substantially" does not exclude "completely" *e.g.* a composition which is "substantially free" from Y may be completely free from Y. Where necessary, the word "substantially" may be omitted from the definition of the invention.

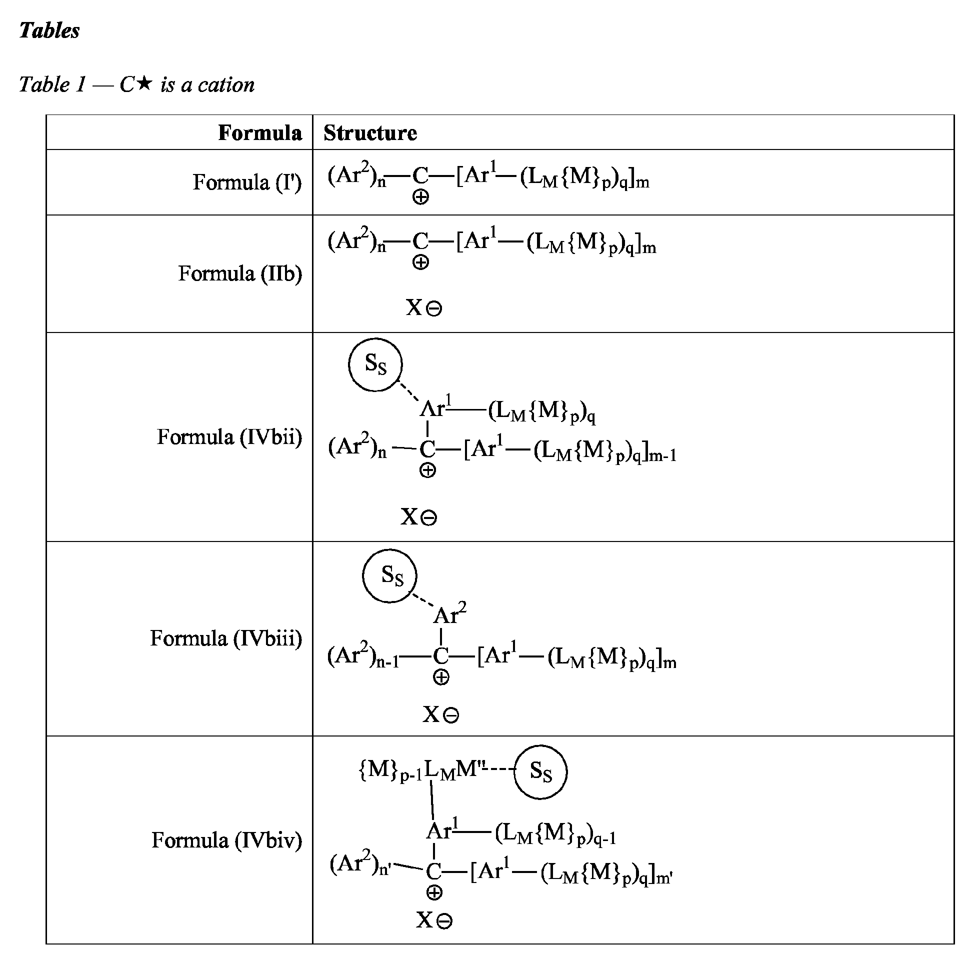

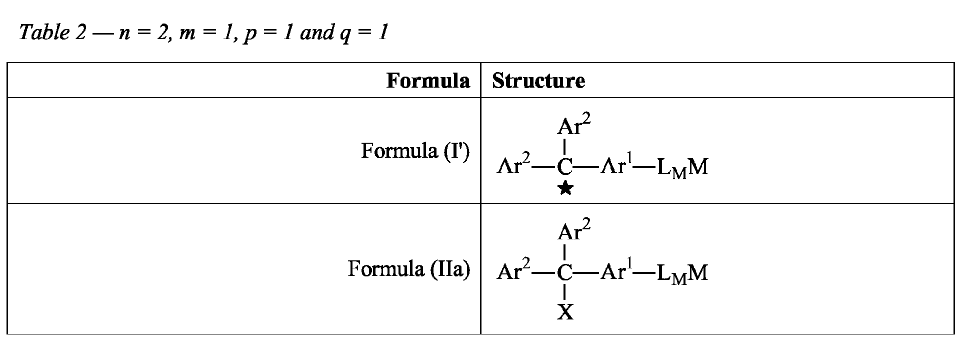

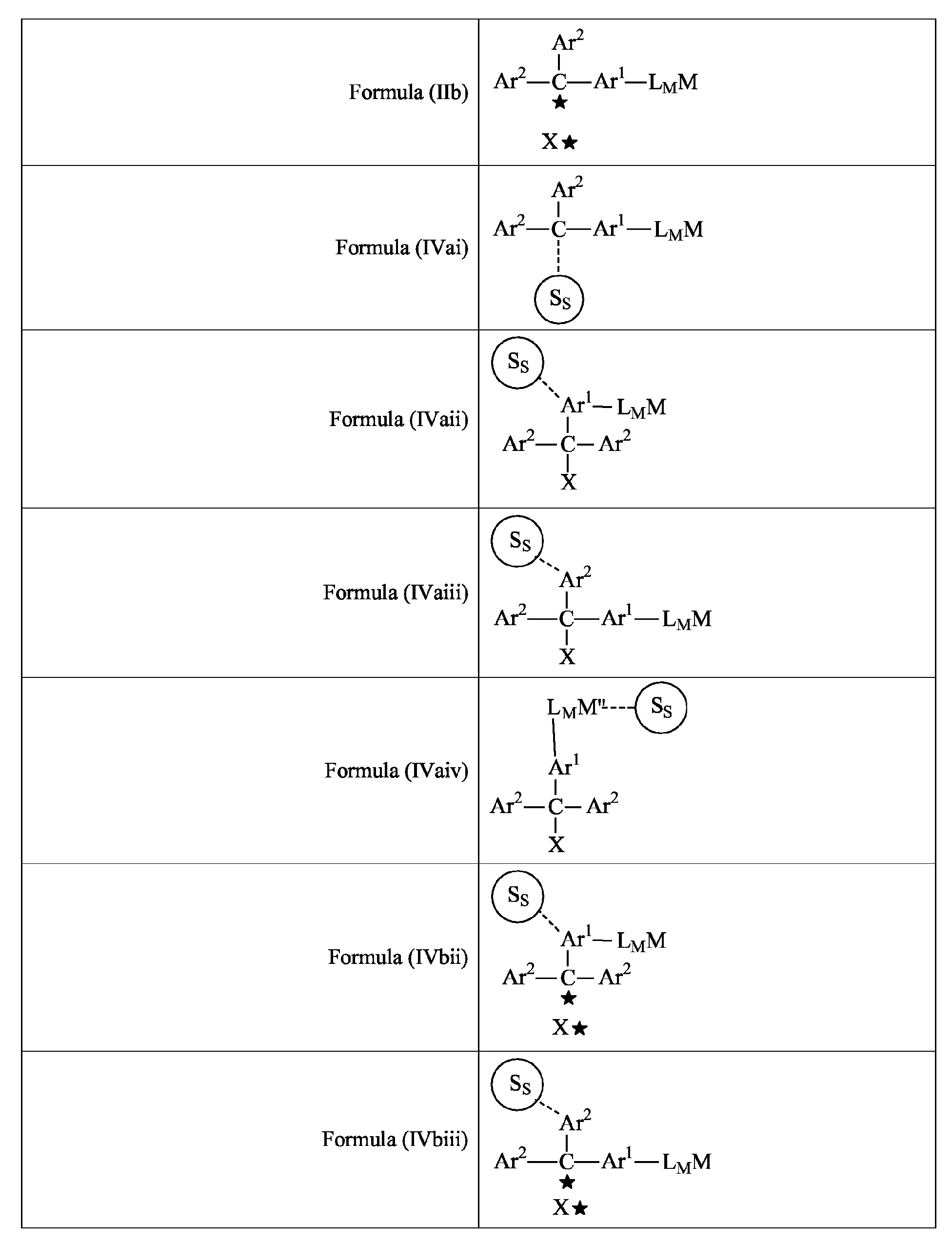

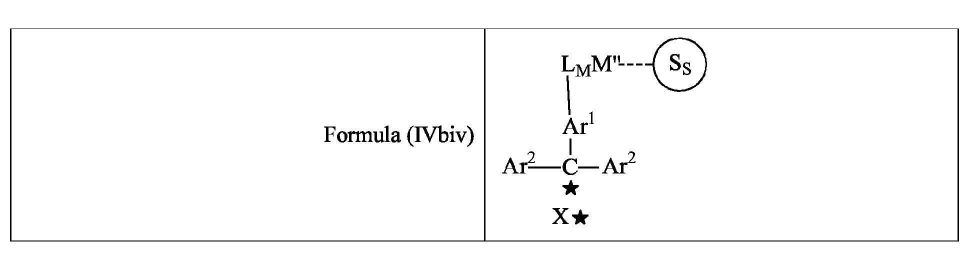

### BRIEF DESCRIPTION OF THE DRAWINGS

Figures 1A and 1B show preferred examples of group L_{M}.
Figures 2A and 2B show preferred examples of group M.
Figures 3A and 3B shows preferred examples of groups Ar¹ and Ar².
Figure 4 shows preferred examples of groups X and X 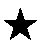 .
Figure 5 shows preferred examples of substituent group A.

### MODES FOR CARRYING OUT THE INVENTION

The compounds of the invention may be prepared by a number of processes as described below.

### L_{M}

*L*_{*M*} *= -CH*_{*2*}*CH*_{*2*}

### Example 1

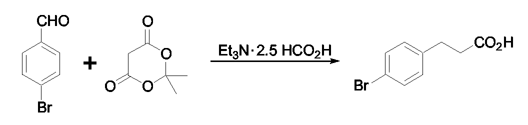

**3-(4-Bromophenyl)propionic acid**. 4-Bromobenzaldehyde (92.5 g, 0.50 mol) and Meldrum's acid (72.0 g, 0.50 mol) were dissolved in triethylamine-formic acid reagent (1.5 L) and the mixture was refluxed for 20 h, then diluted with water (4 L) and acidified with 6N HCl (1 L) until pH reached 2. The precipitated solid was filtered off, washed with diluted HCl, and dissolved in 5% NaHCO₃. The aqueous salt solution was washed with ether (4x400 mL), filtered and acidified with diluted HCl. The resulting 3-(4-bromophenyl)propionic acid was filtered off and dried *in vacuo* over KOH and P₄O₁₀, yield 69.4 g (60 %). The compound was pure according to NMR. *R*_{f} 0.33 (CHCl₃-EtOAc, 9:1). An analytical sample was crystallyzed from toluene and showed mp 134°C. NMR (DMSO-*d*₆): 9.52 (br. s, 1H, O*H*); 8.00 (d, 2H, *J* = 8.1 Hz, Ar*H*); 7.73 (d, 2H, *J* = 8.1 Hz, Ar*H*), 3.41 (t, 2H, *J =* 7.5 Hz, ArC*H*₂), 3.14 (t, 2H, *J*= 7.5 Hz, C*H*₂CO).

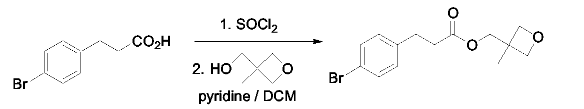

**(3-Methyl-3-oxetanyl)methyl 3-(4-bromophenyl)propionate.** 3-(4-Bromophenyl)propionic acid chloride was prepared in usual manner from 3-(4-bromophenyl)propionic acid (5.50 g, 24 mmol) and oxalyl chloride (4.2 ml, 50 mmol) in benzene (30 mL) with following evaporation. 3-Methyl-3-oxetanemethanol (2.30 ml, 23 mmol) and pyridine (3.8 mL, 48 mmol) were dissolved in DCM (50 mL) and crude 3-(4-bromophenyl)propionyl chloride in DCM (10 mL) was added dropwise within 30 min with stirring and cooling on a water bath. The mixture was stirred for 16 h, then diluted with CHCl₃ (200 mL), washed with water (2x200 mL), 5% NaHCO₃ (2x200 mL), 5% citric acid (3x100 mL) and 10% NaHCO₃ (100 mL), dried over Na₂SO₄, evaporated and chromatographed on silica gel in CHCl₃. Yield 5.90 g (82%). Colorless oil which solidifies upon storage, mp around 30°C. *R*_{f} 0.20 (toluene-EtOAc, 9:1). NMR (CDCl₃): 7.39 (d, 2H, *J*= 8.2 Hz, Ar*H*); 7.07 (d, 2H, *J*= 8.2 Hz, Ar*H*); 4.44 (d, 2H, *J*= 6.0 Hz), 4.34 (d, 2H, *J*= 6.0 Hz) (C*H*₂OC*H*₂); 4.13 (s, 2H, C*H*₂OCO); 2.91 (t, 2H, *J* = 7.6 Hz, ArC*H*₂), 2.66 (t, 2H, *J*= 7.6 Hz, C*H*₂CO); 1.27 (s, 3H, C*H*₃).

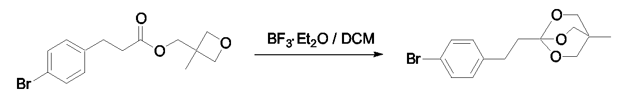

**1-[2-(4-Bromophenyl)ethyl]-4-methyl-2,6,7-trioxabicyclo[2.2.2]octane.** 3-Methyl-3-oxetanyl)-methyl 3-(4-bromophenyl)propionate (31.32 g, 100 mmol) was dissolved in dry DCM (100 mL), cooled on NaCl-ice bath to -15°C, and boron trifluoride diethyl etherate (3.1 mL, 25 mmol) was added in one portion. The mixture was stirred for 1h, then allowed to warm to room temperature, quenched with triethylamine (14 mL, 100 mmol) and diluted with diethyl ether (150 mL). The boron trifluoride-diethyl ether complex was filtered off, the solution was evaporated, and the residue was purified by flash chromatography on silica gel in toluene. Finally, the compound was crystallyzed from toluene-petroleum ether to give colorless needles (24.86 g, 79%), mp 184°C (toluene-petroleum ether). *R*_{f} 0.35 (toluene-EtOAc, 9:1). NMR (DMSO-*d*₆): 7.43 (d, 2H, *J*= 8.3 Hz, Ar*H*); 7.14 (d, 2H, *J*= 8.3 Hz, Ar*H*); 3.84 (s, 6H, C*H*₂O); 2.62 (m, 2H, ArC*H*₂); 1.82 (m, 2H, ArCH₂C*H*₂); 0.76 (s, 3H, C*H*₃).

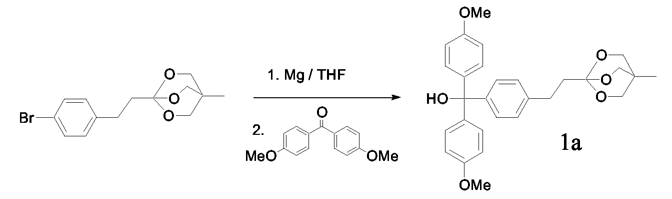

**1-(2-{4-[Hydroxy-bis(4-methoxyphenyl)methyl]phenyl{ethyl)-4-methyl-2,6,7-trioxabicyclo-[2.2.2]octane.** Magnesium turnings (0.30 g, 12.5 mmol) was placed in a three-neck flask (250 mL) and activated by heating with a iodine crystal. The flask was then equipped with dropping funnel, reflux condenser, and argon inlet. THF (20 mL) was added to magnesium and 5-7 mL of the solution of 1-[2-(4-bromophenyl)ethyl]-4-methyl-2,6,7-trioxabicyclo[2.2.2]octane (3.13 g, 10 mmol) in THF (40 mL) was added. The mixture was heated under Ar near to boiling until a reaction started, then the remaining aryl bromide was added dropwise and the mixture was refluxed for 30 min. Using Ar pressure, the solution was filtered through glass wool in a 250 mL flask containing a solution of 4,4'-dimethoxybenzophenone (2.42 g, 10 mmol) in THF (40 mL). The mixture was refluxed for 2 h, then cooled, quenched with 5% NaHCO₃ (200 mL), and extracted with EtOAc (2x100 mL). The organic layer was separated, dried over Na₂SO₄, and evaporated. The residue was chromatographed on silica gel column (2→10% EtOAc in toluene containing 0.5% Et₃N). The resulting oil was triturated in petroleum ether to give the desired to give the desired tritanol (1.50 g, 31%) colorless crystals, mp 145°C (dec.). *R*_{f} 0.44 (toluene-EtOAc, 1:1). NMR (DMSO-*d*₆): 7.07 (m, 8H, Ar*H*); 6.83 (d, 4H, *J*= 8.8 Hz, Ar*H*); 6.11 (s, 1H, O*H*); 3.84 (s, 6H, C*H*₂O); 3.72 (s, 6H, OC*H*₃); 2.62 (m, 2H, ArC*H*₂); 1.81 (m, 2H, ArCH₂C*H*₂); 0.76 (s, 3H, CC*H*₃).

### Example 2

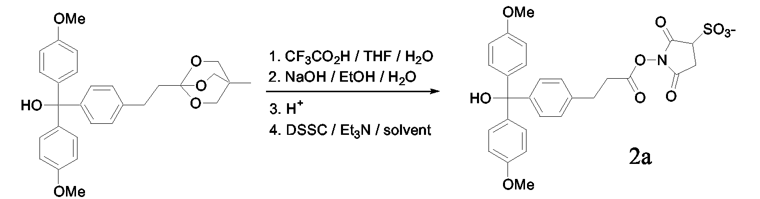

**3-{4-[Hydroxy-bis(4-methoxyphenyl)methyl]phenyl}propionic acid, *N*-oxysulfosuccinimide ester.** 1-(2-{4-[Hydroxy-bis(4-methoxyphenyl)methyl]phenyl}ethyl)-4-methyl-2,6,7-trioxabicyclo-[2.2.2]octane (2.38 g, 5.0 mmol) was dissolved in the THF-water mixture (9:1, 10mL), and trifluoroacetic acid (0.77 mL, 10 mol) was added. After stirring for 10 min the mixture was evaporated and diluted with 10 % NaOH in ethanol-water (8:2, 30mL). The reaction mixture was refluxed for 30 min, cooled, and evaporated, the residue was diluted with water (50 mL), washed with Et₂O (2x50 mL), filtered, acidified with 5% oxalic acid, and extracted with ethyl acetate (3x50 mL). The solution was dried with Na₂SO₄, evaporated, and the residue was dissolved in dry MeCN (30 mL). Triethylamine (1.4 mL, 10 mmol) and *N,N'*-disulfosuccinimidyl carbonate (1.28 g, 5.0 mmol) were added and the mixture was stirred until the reaction is complete (monitoring by TLC in toluene-*tert*-butanol, 9:1), then evaporated, dissolved in EtOAc (200 mL), washed with 5% NaHCO₃ (100 mL) and water (100 mL), dried with Na₂SO₄, evaporated, and the residue was purified by column chromatography (10 to 40% EtOAc in toluene). Yield 2.05 g (86%), white amorphous solid. *R*_{f} 0.31 (toluene-EtOAc, 1:1). NMR (DMSO-*d*₆): 7.20 (d, 2H, *J*= 8.3 Hz, Ar*H*); 7.12-7.05 (m, 6H, Ar*H*); 6.83 (d, 4H, *J* = 9.0 Hz, Ar*H*); 6.15 (s, 1H, O*H*); 3.73 (s, 6H, OC*H*₃); 3.02-2.90 (m, 4H, ArC*H*₂C*H*₂); 2.81 (s, 4H, COCH₂C*H*₂CO).

*L*_{*M*} *= -CH*_{*2*}*CH*_{*2*}*-C(O)O-CH*_{*2*}*C(Me)-(CH*_{*2*}*-)*_{*2*}

### Example 3

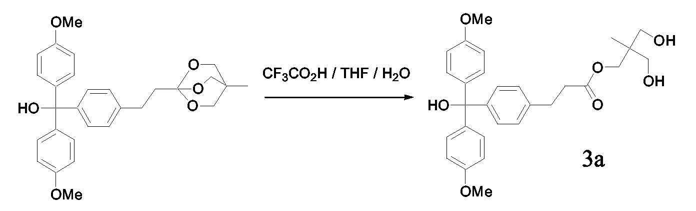

Alternatively, the intermediate diolester could be isolated by chromatography as a colorless oil: **3-{4-[hydroxy-bis(4-methoxyphenyl)methyl]phenyl{propionic acid, 2-hydroxymethyl-2-methyl-3-hydroxypropyl ester.** NMR (DMSO-*d*₆): 7.15-7.04 (m, 8H, Ar*H*); 6.83 (m, 4H, Ar*H*); 6.13 (s, 1H, CO*H*); 4.44 (t, 2H, *J =* 5.4 Hz, CH₂O*H*); 3.87 (s, 2H, C*H*₂OCO); 3.72 (s, 6H, OC*H*₃); 3.24 (m, 4H, C*H*₂OH); 2.83 (t, 2H, *J*= 7.6 Hz, ArC*H*₂); 2.61 (t, 2H, *J*= 7.6 Hz, COC*H*₂); 0.74 (s, 3H, CC*H*₃).

*L*_{*M*} *= -OCH*_{*2*}*CH*_{*2*}*CH*_{*2*}*-*

### Example 4

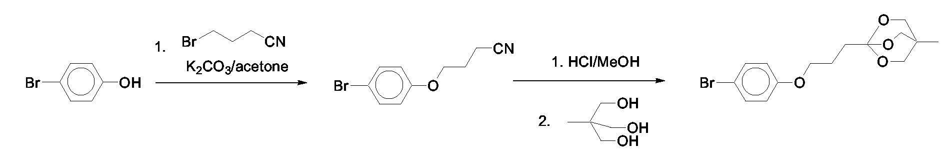

**4-(4-Bromophenoxy)butyronitrile.** To a solution of 4-bromophenol (17.3 g, 0.10 mol) in dry acetone (300 mL) dry K₂CO₃ (70 g, 0.5 mol) and 4-bromobutyronitrile (16.3 g, 0.11 mol) were added, and the mixture was refluxed for approximately 10h, until starting 4-bromophenol consumes (monitoring by TLC in CHCl₃). The mixture was cooled, filtered, and solid inorganic salts were washed with acetone. The combined filtrate was evaporated, and the residue was dissolved in CHCl₃ (100 mL), filtered again, and evaporated. The residue was triturated in petroleum ether, filtered, and dried *in vacuo* to give desired aryl alkyl ether (22.3 g, 93%) as colorless crystals, mp 62°C, *R*_{f} 0.57 (toluene-EtOAc, 4:1). NMR (DMSO-*d*₆): 7.45 (d, 2H, *J* = 9.0 Hz, Ar*H*); 6.92 (d, 2H, *J* = 9.0 Hz, Ar*H*); 4.02 (t, 2H, *J*= 6.1 Hz, OC*H*₂); 2.64 (t, 2H, *J*= 7.1 Hz, C*H*₂CN); 2.01 (m, 2H, CH₂C*H*₂CH₂); NMR (CDCl₃): 7.37 (d, 2H, *J*= 8.9 Hz, Ar*H*); 6.77 (d, 2H, *J*= 8.9 Hz, Ar*H*); 4.03 (t, 2H, *J*= 5.7 Hz, OC*H*₂); 2.57 (t, 2H, *J*= 7.1 Hz, C*H*₂CN); 2.01 (m, 2H, CH₂C*H*₂CH₂).

**1-[3-(4-Bromophenoxy)propyl]-4-methyl-2,6,7-trioxabicyclo[2.2.21 octane.** An ice cooled solution of 4-(4-bromophenoxy)butyronitrile (15.4 g, 64 mmol) in dry Et₂O (50 mL) and dry MeOH (2.6 mL, 64 mmol) was saturated with dry HCl within 3 h and then kept overnight at ambient temperature. The precipitate formed was filtered off, washed with Et₂O, and suspended in the mixture of Et₂O (70 mL) and MeOH (20 mL), then refluxed for 48 h and cooled. The solid was filtered off, the filtrate was evaporated, diluted with petroleum ether (100 mL) and filtered again. The filtrate was evaporated, dissolved in dry MeOH (30 mL), then 1,1,1-tris(hydrohymethyl)ethane (9.01 g, 75 mmol) and BF₃ etherate (0.15 mL, 1.1 mmol) were added. The mixture was stirred for 3 h, evaporated, and the residue was chromatographed on silica gel column in 0→5% EtOAc in toluene containing 1% Et₃N to yield the desired orthoester (6.13 g, 28%) as a colorless solid, mp 93°C, *R*_{f} 0.55 (toluene-EtOAc, 4:1). NMR (DMSO-*d*₆): 7.41 (d, 2H, *J* = 8.9 Hz, Ar*H*); 6.87 (d, 2H, *J* = 8.9 Hz, Ar*H*); 3.94 (t, 2H, *J* = 6.3 Hz, ArOC*H*₂); 3.82 (s, 6H, OC*H*₂); 1.80-1.67 (m, 4H, OCH₂C*H*₂C*H*₂); 0.74 (s, 3H, C*H*₃).

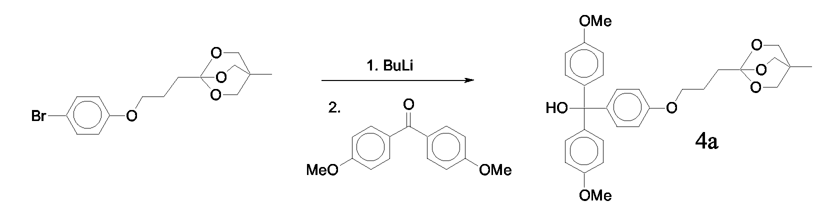

**1-(3-{4-[Hydroxy-bis(4-methoxyphenyl)methyl]phenoxy}propyl)-4-methyl-2,6,7-trioxabicyclo[2.2.2]octane.** To a stirred and coolled to -70°C mixture of THF (40 mL) and 0.9M BuLi in hexane (11.1 mL, 10 mmol) the solution of 1-[3-(4-Bromophenoxy)propyl]-4-methyl-2,6,7-trioxabicyclo[2.2.2]octane (1.716 g, 5.0 mmol) in THF (50 mL) was added dropwise within 30 min. The mixture was then heatet to -40°C and kept for 1h, cooled to -70°C and the solution of 4,4'-dimethoxybenzophenone (1.211 g, 5.0 mmol) in THF (50 mL) was added dropwise within 30 min. The mixture was allowed to warm to room temperature and stirred overnight at ambient temperature. The solution was evaporated to dryness, the residue was dissolved in EtOAc (200 mL), washed with 5% NaHCO₃ (2×200 mL), water (2×200 mL), dried with Na₂SO₄, and evaporated. The residue was dissolved in toluene containing 1% Et₃N (10 mL) and applied to a silicagel column. The column was eluted with stepwise gradient 5→10→15% EtOAc in toluene containing 1% Et₃N. The desired product was triturated in petroleum ether filtered off and dried *in vacuo.* Yield 720 mg (28%), colorless crystals, mp 132°C (dec.). *R*_{f} 0.40 (toluene-EtOAc, 1:1).

### Example 5

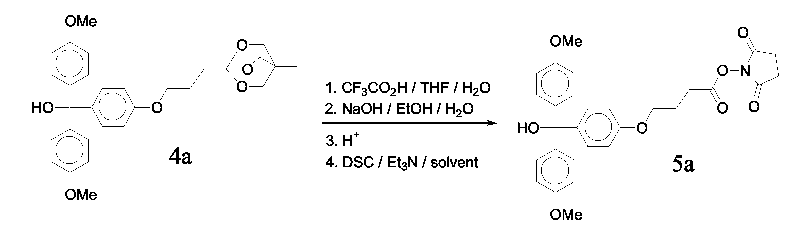

**4-{4-[Hydroxy-bis(4-methoxyphenyl)methyl]phenoxy}butanoic acid, *N*-oxysuccinimide ester.** 1-(3-{4-[Hydroxy-bis(4-methoxyphenyl)methyl]phenoxy}propyl)-4-methyl-2,6,7-trioxabicyclo-[2.2.2]octane (1.01 g, 2.0 mmol) was dissolved in the THF-water mixture (4:1, 50 mL), and trifluoroacetic acid (0.23 mL, 3 mmol) was added. After stirring for 1 h the mixture was half evaporated and diluted with EtOAc (200 mL), washed with water (100 mL), 5% NaHCO₃ (2×100 mL), evaporated. The resudue was dissolved in EtOH (100 mL), and NaOH (1.0 g, 25 mmol) was added and dossolved in the mixture. The solution was stirred overnight at ambient temperature, filtered and evaporated. The residue was dissolved in water (200 mL), washed with Et₂O (2×100 mL), acidified with solid citric acid to pH 3.5, and extracted with EtOAc (3×100 mL). A combined organic lauer was dried over Na₂SO₄ and evaporated to volume 20 mL. Triethylamine (0.7 mL, 5.0 mmol) and *N,N'*-disuccinimidyl carbonate (0.64 g, 2.5 mmol) were added and the mixture was stirred until the reaction is complete (monitoring by TLC in toluene-acetone, 2:1), then evaporated, dissolved in EtOAc (200 mL), washed with 5% NaHCO₃ (100 mL) and water (100 mL), dried with Na₂SO₄, evaporated, and the residue was purified by column chromatography (10 to 30% EtOAc in toluene). Yield 0.800 g (77%), white amorphous solid. *R*_{f} 0.30 (toluene-EtOAc, 1:1).

*L*_{*M*} *= -C≡CCH*_{*2*}*CH*_{*2*}*CH*_{*2*}*-*

### Example 6

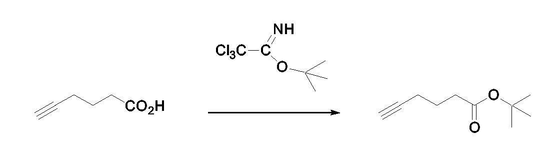

***tert*-Butyl 5-hexynoate.** To a solution of 5-hexynoic acid (5.44 g, 48.5 mmol) in DCM (50 mL) a solution of tert-butyl trichloroacetimidate (17.4 mL, 97 mmol) in petroleum ether (200 mL) and trifluoride diethyl etherate (300 µL, 2.4 mmol) were added in one portion. The mixture was stirred for 2h, filtered, half-evaporated, diluted with Et₂O (150 mL), washed with 5% NaHC03 (3×100 mL), dried over sodium sulphate, and evaporated. The residue was chromatographed in petroleum ether-ethyl acetate (24:1). Yield 5.20 g (64%). NMR (DMSO-*d*₆): 2.77 (t, 1H, *J*= 2.6 Hz, C*H*); 2.28 (t, 2H, *J* = 7.5 Hz, COC*H*₂); 2.17 (dt, 2H, *J*₁ = 2.6 Hz, *J*₂ = 7.1 Hz, ≡CC*H*₂); 1.65 (m, 2H, CH₂C*H*₂CH₂); 1.40 (s, 9H, C*H*₃).

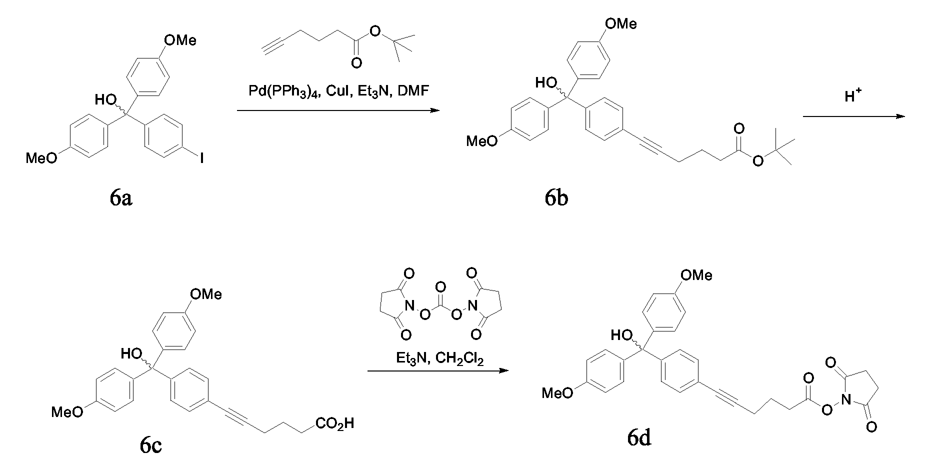

**6-{4-[Hydroxy-bis(4-methoxyphenyl)methyl]phenyl}-5-hexynoic acid, *tert*-butyl ester.** To a solution of 4-iodophenyl-bis(4-methoxyphenyl)methanol (1.785 g, 4.0 mmol), *tert*-butyl 5-hexynoate (1.010 g, 6.0 mmol) and triethylamine (1.67 mL, 12 mmol) in DMF (50 mL) under argon were successively added Pd(PPh₃)₄ (925 mg, 0.8 mmol) and CuI (305 mg, 1.6 mmol), and the reaction mixture was stirred for 16 h at room temperature. Then the mixture was diluted with DCM (300 mL), washed with 3% aqueous EDTA-(NH₄)₂ (5×200 mL) and water (5×200 mL), dried, and evaporated to dryness. The residue was chromatographed on a silica gel column (4.5×15 cm) in a 0→6% gradient of EtOAc in toluene to give the desired compound as a colorless oil (1.671 g, 86%). NMR (DMSO-*d*₆): 7.30 (d, 2H, *J* = 8.3 Hz, Ar*H*); 7.15 (d, 2H, *J* = 8.3 Hz, Ar*H*); 7.06 (d, 4H, *J* = 8.8 Hz, Ar*H*); 6.84 (d, 4H, *J*= 8.8 Hz, Ar*H*); 6.27 (s, 1H, O*H*); 3.73 (s, 6H, OC*H*₃); 2.43 (t, 2H, *J*= 7.1 Hz), 2.34 (t, 2H, *J*= 7.4 Hz), (C*H*₂CH₂C*H*₂); 1.74 (m, 2H, CH₂C*H*₂CH₂); 1.40(s, 9H, CC*H*₃).

**6-{4-[Hydroxy-bis(4-methoxyphenyl)methyl]phenyl}-5-hexynoic acid, N-oxysuccinimide ester.** To a stirred solution of *tert*-butyl 6-{4-[hydroxy-bis(4-methoxyphenyl)methyl]phenyl}-5-hexynoate (1.46 g; 3.0 mmol) in dry DCM (4 mL) trifluoroacetic acid (4 mL) was added in one portion and the mixture was stirred at ambient temperature for 3 h, then evaporated, and co-evaporated with DCM (4×50 mL). The crude acid was dissolved in DCM (50 mL), washed with water (10 mL), dried over Na₂SO₄, and half-evaporated. Triethylamine (0.85 mL, 6.0 mmol) and *N,N'*-disuccinimidyl carbonate (0.77 g, 3.0 mmol) were added and the mixture was stirred until the reaction is complete (monitoring by TLC in toluene- EtOAc, 2:1), then evaporated, dissolved in EtOAc (200 mL), washed with 5% NaHCO₃ (100 mL) and water (100 mL), dried with Na₂SO₄, evaporated, and the residue was purified by column chromatography (5 to 15% EtOAc in toluene). Yield 1.110 g (70%), white amorphous solid. NMR (DMSO-*d*₆): 7.33 (d, 2H, *J*= 8.3 Hz, Ar*H*); 7.16 (d, 2H, *J*= 8.3 Hz, Ar*H*); 7.06 (d, 4H, *J* = 8.8 Hz, Ar*H*); 6.84 (d, 4H, *J*= 8.8 Hz, Ar*H*); 6.27 (s, 1H, O*H*); 3.73 (s, 6H, OC*H*₃); 2.82 (m, 6H), 2.54 (t, 2H, *J*= 7.1 Hz), (C*H*₂CH₂C*H*₂, COC*H*₂C*H*₂CO); 1.90 (m, 2H, CH₂C*H*₂CH₂).

### Example 7

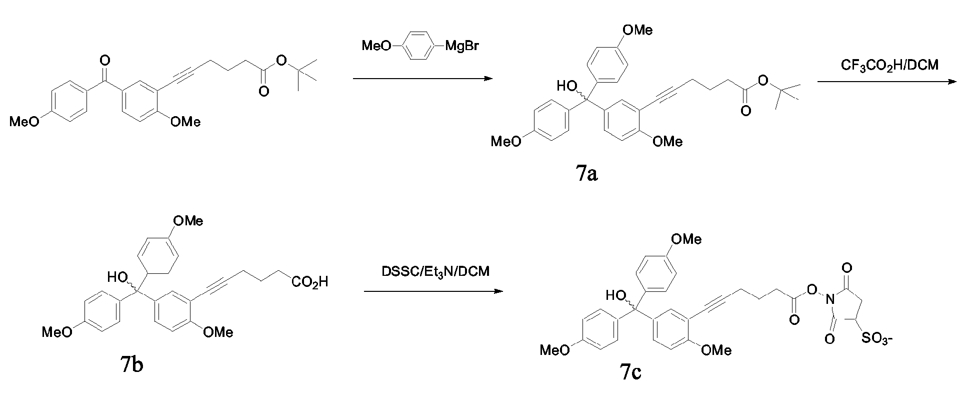

**6-{2-Methoxy-5-[hydroxy-bis(4-methoxyphenyl)methyl]phenyl}-5-hexynoic acid, *tert*-butyl ester.** To a stirred solution 3-[5-(*tert*-butyloxycarbonyl)pent-1-ynyl]-4,4'-dimethoxy benzophenone (1.18 g; 2.88 mmol) in dry THF (20 mL) 0.9 M 4-methoxyphenylmagnesium bromide (5.8 mL, 4.0 mmol) was added in one portion under argon, and the mixture was kept at ambient temperature overnight (monitoring by TLC in EtOAc-toluene 1:3). The reaction was diluted with water (50 mL) and 5% citric acid (15 mL), and extracted with EtOAc (100 mL). The organic phase was dried over Na₂SO₄, evaporated, and chromatographed on silica gel in 0→5% EtOAc in toluene with 1% of Et₃N to give the desired compound as a colourless oil. Yield 516 mg (35%). NMR (DMSO-*d*₆): 7.12 (d, 1H, ⁴*J* = 2.1 Hz, Ar*H*); 7.09-7.02 (m, 5H, Ar*H*); 6.92 (d, 1H, *J* = 8.9 Hz, Ar*H*); 6.84 (d, 4H, *J* = 8.9 Hz, Ar*H*); 6.16 (s, 1H, O*H*); 3.78 (s, 3H), 3.73 (s, 6H) (OC*H*₃); 2.40 (t, 2H, *J*= 7.0 Hz, ≡CC*H*₂); 2.33 (t, 2H, *J*= 7.5 Hz, COC*H*₂); 1.70 (m, 2H, CH₂C*H*₂CH₂); 1.39 (s, 9H, CC*H*₃).

**6-{2-Methoxy-5-[hydroxy-bis(4-methoxyphenyl)methyl]phenyl}-5-hexynoic acid, *N*-oxysulfosuccinimide ester, sodium salt.** To a stirred solution of *tert*-butyl ester of 6-{2-methoxy-5-[hydroxy-bis(4-methoxyphenyl)methyl]phenyl}-5-hexynoic acid (516 mg; 1.0 mmol) in dry DCM (3.5 mL) trifluoroacetic acid (3.5 mL) was added in one portion and the mixture was stirred at ambient temperature for 5 h, then evaporated, and co-evaporated with DCM (4×20 mL) to give free acid. Product were dissolved in DCM (10 mL), and triethylamine (0.42 mL, 3.0 mmol) and *N*,*N*-disulfosuccinimidyl carbonate (384 mg, 1.5 mmol) were added and the mixture was stirred overnight, then evaporated, dissolved in EtOAc (50 mL), washed with 5% NaHCO₃ (50 mL) and water (50 mL), dried over Na₂SO₄, evaporated, and the residue was purified by column chromatography (5→10% acetone in toluene). Yield 218 mg (39%), white amorphous solid. NMR (DMSO-*d*₆): 7.14 (d, 1H, ⁴*J*= 2.1 Hz, Ar*H*); 7.09-7.02 (m, 5H, Ar*H*); 6.93 (d, 1H, *J*= 8.8 Hz, Ar*H*); 6.84 (d, 4H, *J*= 8.7 Hz, Ar*H*); 6.17 (s, 1H, O*H*); 3.79 (s, 3H), 3.73 (s, 6H) (OC*H*₃); 2.83 (m, 2H, C*H*₂CO); 2.51 (t, 2H, *J*= 7.0 Hz, ≡CC*H*₂); 1.86 (m, 2H, CH₂C*H*₂CH₂).

*L*_{*M*} *= -CH*_{*2*}*CH*_{*2*}*CH*_{*2*}*CH*_{*2*}*CH*_{*2*}*-*

### Example 8

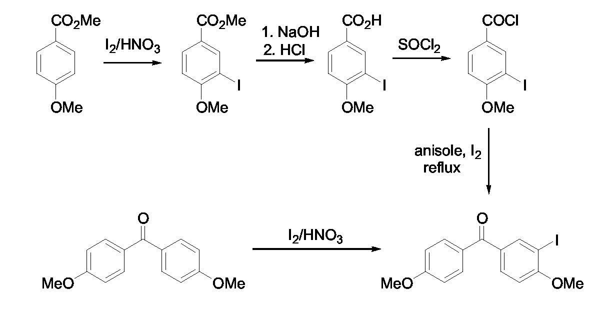

### Methyl 3-iodo-4-methoxybenzoate.

To a solution of methyl 4-methoxybenzoate (29.7 g, 0.18 mol) in CCl₄ (110 mL) iodine (22.7 g, 0.89 mol) was added and the mixture was stirred for 10 min. Nitric acid (58%, 50 mL) was added dropwise wiwhin 30 min and the mixture was refluxed for 3 h. The mixture was cooled to room temperature and precipitate formed was filtered off. The organic layer was separated, washed with 5% Na₂SO₃ (100 mL), dried over CaCl₂ and evaporated. The residue was combined with the precipitate and recrystallized twice from EtOH. Yield 30.4 g (58%).

### 3-Iodo-4-methoxybenzoyl chloride.

Methyl 3-iodo-4-methoxybenzoate (29.2 g, 0.1 mol) was suspended in EtOH (150 mL), the solution of NaOH (4.4 g, 0.11 mol) was added in one portion. The mixture was stirred and heated at 40°C overnight, then cooled, diluted with water (400 mL). 3-Iodo-4-methoxybenzoic acid was precipitated with conc. HCl, filtered off, washed with cold water, and dried over P₄O₁₀. The acid was suspended in CHCl₃ (150 mL), and SOCl₂ (9.5 mL, 0.13 mmol) was added. The mixture was stirred overnight, then evaporated and the residue was distilled under reduced pressure to give the desired acid chloride as a solid (mp ca. 50°C, bp 145-150°C/1 Torr). Yield 18.2 g (61%).

### 3-Iodo-4,4'-dimethoxybenzophenone.

**A.** The mixture of 3-iodo-4-methoxybenzoyl chloride (14.8 g, 0.05 mol), anisole (11.0 mL, 0.1 mol) and iodine (0.5 g) was refluxed for 10 h, then cooled to 60°C, diluted with iPrOH (200 mL), refluxed for 30 min, cooled and kept in freezer overnight. The precipitate was filtered off, washed with cold iPrOH (10 mL), dried, and recrystallized from EtOH. Yield 10.4 (56.5%), off-white solid. NMR (DMSO-*d*₆): 8.09 (d, 1H, ⁴*J*= 2.1 Hz, Ar*H*); 7.75-7.68 (m, 3H, Ar*H*); 7.14 (d, 1H, *J*= 8.7 Hz, Ar*H*); 7.09 (d, 2H, *J*= 8.7 Hz, Ar*H*); 3.93 (s, 3H), 3.86 (s, 3H) (OC*H*₃).
**B.** 4,4'-Dimethoxybenzophenone (18.1 g, 75 mmol) was dissolved in dioxane (70 mL) at 60°C, iodine (9.9 g, 39 mmol) was added and the mixture was stirred for 15 min. Water (20 mL) was added, and 58% HNO₃ (41 mL) was added dropwise within 2 h. The mixture was stirred at 60°C until iodine colouring disappears (ca. 6-7 h). The flask was evacuated to remove nitrogen oxides, the mixture was diluted with water (100 mL), cooled, and the precipitate was filtered off, washed with 5% NaHCO₃ and water. The solid was suspended in EtOH (200 mL), refluxed for 15 min, filtered hot, and cooled in freezer to 4°C. The solid (16 g) was filtered off and dried. It contains (GLC) 75% of desired 3-iodo-4,4'-dimethoxybenzophenone, 24% of starting 4,4'-dimethoxybenzophenone and 0.7% of 3,3'-diiodo-4,4'-dimethoxybenzophenone. This was purified by column chromatography in toluene. Yield 11.2 g (40%).

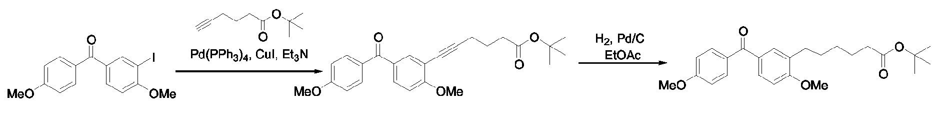

### 3-[5-(tert-Butyloxycarbonyl)pent-1-ynyl]-4,4'-dimethoxybenzophenone.

To a solution of 3-iodo-4,4'-dimethoxybenzophenone (4.50 g, 12.2 mmol) and *tert*-butyl 5-hexynoate (2.10 g, 12.5 mmol) in DMF (50 mL) Pd(PPh₃)₄ (1.40 g, 1.22 mmol), CuI (465 mg, 2.44 mmol), Et₃N (2.55 mL, 18.3 mmol) were subsequently added. The mixture was stirred overnight under argon, then diluted with water (200 mL) and extracted with EtOAc (200 mL). The organic layer was washed with water (4×200 mL), 0.1 M solution (NH₄)₂EDTA (4×200 mL) and dried over Na₂SO₄. The residue was chromatographed column in gradient of EtOAc in toluene (0 to 5%). Yield 3.43 g, viscous yellowish oil (69%). NMR (DMSO-*d*₆): 7.72-7.67 (m, 3H, Ar*H*); 7.64 (d, 1H, ⁴*J*= 2.3 Hz, Ar*H*); 7.17 (d, 1H, *J*= 8.7 Hz, Ar*H*); 7.08 (d, 2H, *J*= 9.0 Hz, Ar*H*); 3.91 (s, 3H, OC*H*₃); 3.86 (s, 3H, OC*H*₃); 2.47 (t, 2H, *J* = 7.0 Hz, ≡CC*H*₂); 2.38 (t, 2H, *J* = 7.4 Hz, COC*H*₂); 1.75 (m, 2H, CH₂C*H*₂CH₂); 1.39 (s, 9H, CC*H*₃).

**3-[5-(*tert*-Butyloxycarbonyl)pentyl]-4,4'-dimethoxybenzophenone.** *3*-[5-(*tert*-Butyloxycarbonyl)pent-1-ynyl]-4,4'-dimethoxybenzophenone (2.027 g, 4.96 mmol) was dissolved in EOAc (40 mL), and 10% Pd/C (100 mg) was added. Hydrogen gas was bubbled through the mixture with stirring. PdCl₂ (50 mg portions) were added after 2 and 4 h. The mixture was hydrogenated for additional 4 h, filtered, diluted with EtOAc (150 mL), washed with washed with 5% NaHCO₃ (100 mL) and 0.1 M (NH₄)₂EDTA (100 mL), dried over Na₂SO₄, evaporated, and the residue was purified by column chromatography (0→10% EtOAc in toluene). Yield 1.493 g (73%), colourless oil. NMR (DMSO-*d*₆): 7.69 (d, 2H, *J* = 8.7 Hz, Ar*H*); 7.57 (m, 1H, Ar*H*); 7.53 (m, 1H, Ar*H*); 7.06 (m, 3H, Ar*H*); 3.87 (s, 3H, OC*H*₃); 3.85 (s, 3H, OC*H*₃); 2.58 (t, 2H, *J*= 7.4 Hz, ArC*H*₂); 2.15 (t, 2H, *J*= 7.3 Hz, COC*H*₂); 1.50 (m, 4H, COCH₂C*H*₂CH₂C*H*₂); 1.35 (s, 9H, CC*H*₃); 1.28 (m, 2H, COCH₂CH₂C*H*₂).

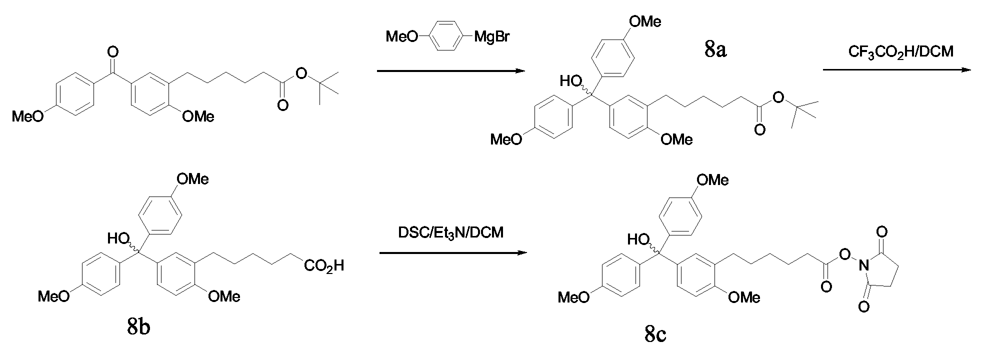

### 6-{2-Methoxy-5-[hydroxy-bis(4-methoxyphenyl)methyl]phenyl}hexanoic acid, tert-butyl ester.

To a stirred solution 3-[5-(*tert*-butyloxycarbonyl)pentyl]-4,4'-dimethoxybenzophenone (412 mg; 1.0 mmol) in dry THF (10 mL) 1 M 4-methoxyphenylmagnesium bromide (1.2 mL, 1.2 mmol) was added in one portion under argon, and the mixture was kept at ambient temperature overnight (monitoring by TLC in EtOAc-toluene 1:3). The reaction was diluted with water (50 mL) and saturated aq. NH₄Cl (20 mL), and extracted with EtOAc (2×100 mL). The organic phase was dried over Na₂SO₄, evaporated, and chromatographed on silica gel in 0→7% EtOAc in toluene with 0.5% of Et₃N to give the desired compound as a colourless oil. Yield 274 mg (53%). NMR (DMSO-*d*₆): 7.06 (m, 4H, Ar*H*); 6.97 (d, 1H, ⁴*J*= 1.9 Hz, Ar*H*); 6.88-6.79 (m, 6H, Ar*H*); 6.02 (s, 1H, O*H*); 3.74 (s, 3H, OC*H*₃); 3.72 (s, 6H, OC*H*₃); 2.45 (t, 2H, *J* = 7.5 Hz, ArC*H*₂); 2.12 (t, 2H, *J* = 7.3 Hz, COC*H*₂); 1.44 (m, 4H, COCH₂C*H*₂CH₂C*H*₂); 1.37 (s, 9H, CC*H*₃); 1.20 (m, 2H, COCH₂CH₂C*H*₂).

### 6-{2-Methoxy-5-[hydroxy-bis(4-methoxyphenyl)methyl]phenyl}hexanoic acid, N-oxysuccinimide ester.

To a stirred solution of *tert*-butyl ester of 6-{2-methoxy-5-[hydroxy-bis(4-methoxyphenyl)methyl]phenyl}hexanoic acid (274 mg; 0.53 mmol) in dry DCM (2 mL) trifluoroacetic acid (2 mL) was added in one portion and the mixture was stirred at ambient temperature for 3 h, then evaporated, and co-evaporated with DCM (4x50 mL) to give free acid. Product were dissolved in DCM (15 mL), and triethylamine (0.60 mL, 4.3 mmol) and *N,N*-disuccinimidyl carbonate (556 mg, 2.17 mmol) were added and the mixture was stirred overnight, then evaporated, dissolved in EtOAc (50 mL), washed with 5% NaHCO₃ (50 mL) and water (50 mL), dried over Na₂SO₄, evaporated, and the residue was purified by column chromatography (15→30% EtOAc in toluene). Yield 277 mg (93%), pink amorphous solid. NMR (DMSO-*d*₆): 7.71 (d, 1H, *J*= 8.9 Hz, Ar*H*); 7.07 (d, 4H, *J*= 8.9 Hz, Ar*H*); 6.99 (d, 1H, ⁴*J*= 2.1 Hz, Ar*H*); 6.89-6.78 (m, 5H, Ar*H*); 6.02 (s, 1H, O*H*); 3.89 (s, 3H), 3.86 (s, 6H) (OC*H*₃); 2.80 (s, 4H, COC*H*₂C*H*₂CO); 2.61 (t, 2H, *J* = 7.3 Hz), 2.46 (t, 2H, *J* = 7.6 Hz) (ArC*H*₂CH₂CH₂CH₂C*H*₂); 1.54 (m, 2H), 1.44 (m, 2H), 1.32 (m, 2H) (ArC*H*₂CH₂C*H*₂).

*L*_{*M*} *= -CD*_{*2*}*CD*_{*2*}*CH*_{*2*}*CH*_{*2*}*CH*_{*2*}*-*

### Example 9

Same as for L_{M} = -CH₂CH₂CH₂CH₂CH₂- (example 8), but D₂ gas (Aldrich) was used to reduce the triple bond of 3-[5-(*tert*-Butyloxycarbonyl)pent-1-ynyl]-4,4'-dimethoxybenzophenone instead of hydrogen. All other synthetic procedures were identical. MS (MALDI-TOF) of equimolar mixture of 'light' (¹H) and 'heavy' (²H) NHS esters: 543.94 (95); 547.98 (93).

*L*_{*M*} *= -OCH*_{*2*}*CH*_{*2*}*CH*_{*2*}*CH*_{*2*}*CH*_{*2*}*-*

### Example 10

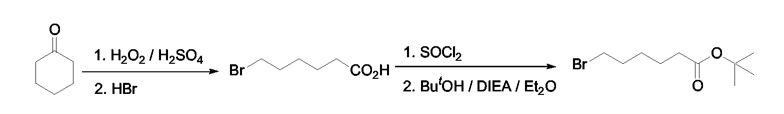

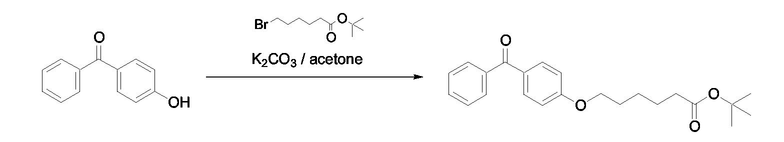

**6-Bromohexanoic acid** was prepared from cyclohexanone as described in the literature (Krayevsky et al. Zh. Obshch. Khim., 1964, 1180-1183).

**6-Bromohexanoyl chloride.** 6-Bromohexanoic acid was treated with 2 mol SOCl₂ Overnight, the excess of SOCl₂ was evaporated, and the residue was distilled under reduced pressure, bp 85-90/1 mm Hg. Yield 90-95%.

***tert*-Butyl 6-bromohexanoate.** 6-Bromohexanoyl chloride (21.4 g, 0.10 mol) was added dropwise to a solution of tert-butanol (7.4 g, 0.1 mol) and diisopropylethylamine (13 g, 0.1 mol) in diethyl ether (200 mL) within 1 h and the mixture was stirred overnight. The precipitate formed was filtered off, washed with ether (100 mL), the combined ether solution was washed with 5% NaHCO₃ (2×50 mL), 5% citric acid (50 mL) and water (50 mL), dried over CaCl₂, and passed through silica gel layer (5 cm) to give the desired ester as an oil (26.5 g, 72%).

**4-[5-(*tert*-Butyloxycarbonyl)pentyloxy]benzophenone.** To a solution of 4-hydroxybenzophenone (1.98 g, 10.0 mmol) in in dry acetone (80 mL) dry K ₂CO₃ (14 g, 0.1 mol) and *tert*-butyl 6-bromohexanoate (3.76 g, 15 mmol) were added, and the mixture was stirred for 48 h at ambient temperature, then filtered, and the solid was washed with acetone. The combined filtrate was evaporated, and the residue was dissolved in CHCl₃ (100 mL), filtered, and evaporated. The residue was chromatographed on silica gel in 0→6% EtOAc in toluene to give desired product (3.10 g, 84%). NMR (DMSO-*d*₆): 7.73 (d, 2H, *J* = 8.7 Hz, Ar*H*); 7.70-7.62 (m, 3H, Ar*H*); 7.54 (t, 2H, *J* = 7.6 Hz, Ar*H*); 7.06 (d, 2H, *J* = 8.7 Hz, Ar*H*); 4.07 (t, 2H, *J* = 6.4 Hz, OC*H*₂); 2.21 (t, 2H, *J* = 7.4 Hz, COC*H*₂); 1.74 (m, 2H), 1.56 (m, 2H), 1.42 (m, 2H) (OCH₂C*H*₂C*H*₂C*H*₂); 1.39 (s, 9H, C*H*₃).

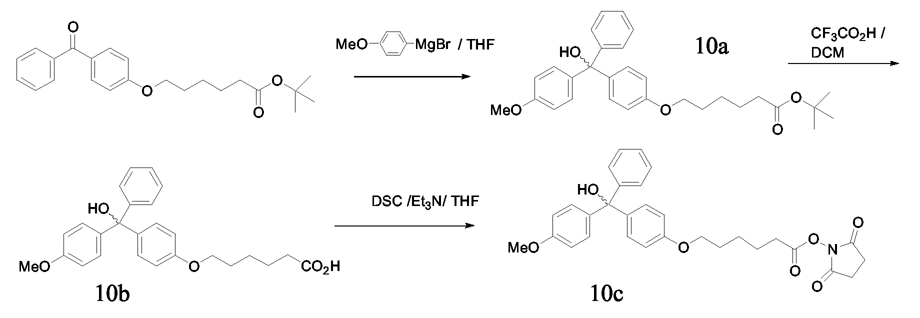

**6-{4-[Hydroxy-(4-methoxyphenyl)-phenyl-methyl]phenoxy}hexanoic acid, *tert*-butyl ester.** To a stirred solution of 4-[5-(*tert*-butyloxycarbonyl)pentyloxy]benzophenone (1.84 g; 5.0 mmol) in dry THF (30 mL) 1 M 4-methoxyphenylmagnesium bromide (7.0 mL, 7.0 mmol) was added in one portion under Ar, and the mixture was kept at ambient temperature overnight (monitoring by TLC in EtOAc-toluene 1:4). The reaction was diluted with water (300 mL) and 5% citric acid (20 mL), and extracted with EtOAc (2×100 mL). The organic phase was dried over Na₂SO₄, evaporated, and chromatographed on silica gel in 0→5% EtOAc in toluene with 0.5% of Et₃N to give the desired compound as a colourless oil. Yield 1.597 g (67%). NMR (DMSO-*d*₆): 7.26 (m, 2H, Ar*H*); 7.20 (m, 3H, Ar*H*); 7.07 (m, 4H, Ar*H*); 6.83 (m, 4H, Ar*H*); 6.17 (s, 1H, O*H*); 3.92 (t, 2H, *J*= 6.4 Hz, OC*H*₂); 3.73 (s, 3H, OC*H*₃); 2.20 (t, 2H, *J* = 7.3 Hz, COC*H*₂); 1.70 (m, 2H, OCH₂C*H*₂), 1.54 (m, 2H, COCH₂C*H*₂), 1.39 (m, 2H, OCH₂CH₂C*H*₂); 1.38 (s, 9H, C*H*₃).

**6-{4-[Hydroxy-(4-methoxyphenyl)-phenyl-methyl]phenoxy}hexanoic acid, *N*-oxysuccinimide** ester. To a stirred solution of *tert*-butyl 6-{4-[hydroxy-(4-methoxyphenyl)-phenylmethyl]phenoxy}hexanoate (953 mg; 2.0 mmol) in dry DCM (3 mL) trifluoroacetic acid (3 mL) was added in one portion and the mixture was stirred at ambient temperature for 3 h, then evaporated, and co-evaporated with DCM (4×50 mL) to give the desired acid. This was dissolved in EtOAc (40 mL), washed with water (10 mL), dried over Na₂SO₄, and evaporated to volume 15 mL. Triethylamine (0.7 mL, 5.0 mmol) and *N*,*N*'-disuccinimidyl carbonate (0.64 g, 2.5 mmol) were added and the mixture was stirred until the reaction is complete (monitoring by TLC in toluene-EtOAc, 2: 1), then evaporated, dissolved in EtOAc (200 mL), washed with 5% NaHCO₃ (100 mL) and water (100 mL), dried with Na₂SO₄, evaporated, and the residue was purified by column chromatography (5→15% EtOAc in toluene). Yield 743 mg (72%), white amorphous solid. NMR (DMSO-*d*₆): 7.26 (m, 2H, Ar*H*); 7.20 (m, 3H, Ar*H*); 7.07 (m, 4H, Ar*H*); 6.83 (m, 4H, Ar*H*); 6.17 (s, 1H, O*H*); 3.93 (t, 2H, *J*= 6.2 Hz, OC*H*₂); 3.73 (s, 3H, OC*H*₃); 2.81 (s, 4H, COC*H*₂C*H*₂CO); 2.69 (t, 2H, *J*= 7.2 Hz, COC*H*₂); 1.78-1.64 (m, 4H, OCH₂C*H*₂CH₂C*H*₂), 1.51 (m, 2H, OCH₂CH₂C*H*₂).

### Example 11

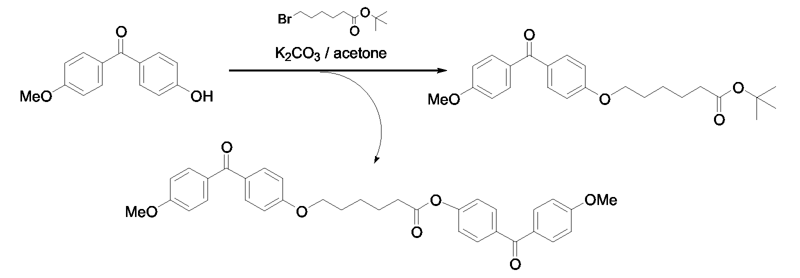

**4-[5-(*tert*-Butyloxycarbonyl)pentyloxy]-4'-methoxybenzophenone.** To a solution of 4-hydroxy-4'-methoxybenzophenone (5.84 g, 25.6 mmol) in in dry acetone (250 mL) dry K₂CO₃ (17.7 g, 0.128 mol) and *tert*-butyl 6-bromohexanoate (7.05 g, 28.1 mmol) were added, and the mixture was stirred for 96 h at ambient temperature, then filtered, and the solid was washed with acetone. The combined filtrate was evaporated, and the residue was dissolved in CHCl₃ (150 mL), filtered, and evaporated. The residue was chromatographed on silica gel in 0→20% EtOAc in toluene to give the desired product (1.99 g, 19.5%). NMR (DMSO-*d*₆): 7.70 (m, 4H, Ar*H*); 7.06 (m, 4H, Ar*H*); 4.06 (t, 2H, *J*= 6.4 Hz, OC*H*₂); 3.86 (s, 3H, OC*H*₃); 2.21 (t, 2H, J= 7.3 Hz, COC*H*₂); 1.75 (m, 2H), 1.56 (m, 2H), 1.42 (m, 2H) (OCH₂C*H*₂C*H*₂C*H*₂); 1.39 (s, 9H, CC*H*₃). **4-Methoxy-4'-[5-(4-methoxybenzoylphenoxycarbonyl)pentyloxy]benzophenone** was isolated as a side product. NMR (DMSO-*d*₆): 7.78 (m, 8H, Ar*H*); 7.19 (d, 2H, *J*= 8.2 Hz, Ar*H*); 6.95 (m, 6H, Ar*H*); 4.07 (t, 2H, *J*= 6.1 Hz, OC*H*₂); 3.88; 3.87 (2s, 6H, OC*H*₃); 2.64 (t, 2H, *J* = 7.3 Hz, COC*H*₂); 1.88 (m, 4H, OCH₂C*H*₂CH₂C*H*₂); 1.64 (m, 2H, OCH₂CH₂C*H*₂).

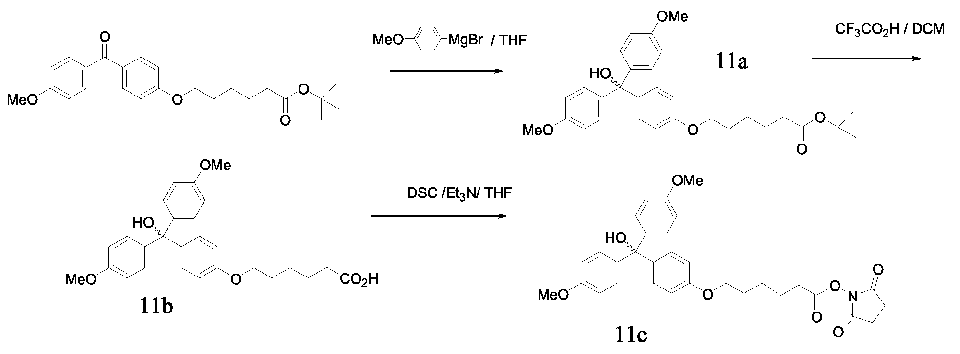

**6-{4-[Hydroxy-bis(4-methoxyphenyl)methyl]phenoxy}hexanoic acid, *tert-*butyl ester.** To a stirred solution of 4-[5-(*tert-*butyloxycarbonyl)pentyloxy]-4'-methoxybenzophenone (400 mg; 1.0 mmol) in dry THF (10 mL) 1 M 4-methoxyphenylmagnesium bromide (1.2 mL, 1.2 mmol) was added in one portion under Ar, and the mixture was kept at ambient temperature overnight (monitoring by TLC in EtOAc-toluene 1:4). The reaction was diluted with water (100 mL) and 5% citric acid (5 mL), and extracted with EtOAc (2×500 mL). The organic phase was dried over Na₂SO₄, evaporated, and chromatographed on silica gel in 0→15% EtOAc in toluene with 0.5% of Et₃N to give the desired compound as a colourless oil. Yield 334 mg (66%). NMR (DMSO-*d*₆): 7.06 (m, 6H, Ar*H*); 6.82 (m, 6H, Ar*H*); 6.07 (s, 1H, O*H*)*;* 3.92 (t, 2H, J= 6.2 Hz, OC*H*₂); 3.72 (s, 6H, OC*H*₃); 2.20 (t, 2H, J= 7.3 Hz, COC*H*₂); 1.69 (m, 2H), 1.54 (m, 2H), 1.39 (m, 2H), (OCH₂C*H*₂C*H*₂C*H*₂); 1.37 (s, 9H, CC*H*₃).

**6-{4-[Hydroxy-bis(4-methoxyphenyl)methyl]phenoxy}hexanoic acid, *N*-oxysuccinimide ester.** To a stirred solution of *tert*-butyl 6-{4-[hydroxy-bis(4-methoxyphenyl)methyl]phenoxy}hexanoate (253 mg; 0.5 mmol) in dry DCM (1.5 mL) trifluoroacetic acid (1.5 mL) was added in one portion and the mixture was stirred at ambient temperature for 4 h, then evaporated, and co-evaporated with DCM (4×20 mL) to give the corresponding acid. The latter was dissolved in EtOAc (30 mL), washed with water (10 mL), dried over Na₂SO₄, and evaporated. The residue was dissolved in DCM (15 mL), and triethylamine (0.30 mL, 2.2 mmol) and *N,N*-disuccinimidyl carbonate (256 mg, 1.0 mmol) were added and the mixture was stirred overnight, then evaporated, dissolved in EtOAc (50 mL), washed with 5% NaHCO₃ (50 mL) and water (50 mL), dried over Na₂SO₄, evaporated, and the residue was purified by column chromatography (10→25% EtOAc in toluene). Yield 240 mg (88%), white amorphous solid. NMR (DMSO-*d*₆): 7.06 (m, 6H, Ar*H*); 6.83 (m, 6H, Ar*H*); 6.07 (s, 1H, O*H*); 3.93 (t, 2H, *J*= 6.2 Hz, OC*H*₂); 3.72 (s, 3H, OC*H*₃); 2.80 (s, 4H, COC*H*₂C*H*₂CO); 2.69 (t, 2H, *J*= 7.3 Hz, C*H*₂CO₂); 1.70 (m, 4H, OCH₂C*H*₂CH₂C*H*₂); 1.50 (m, 2H, OCH₂CH₂C*H*₂).

*L*_{*M*} *= -C≡CCH*_{*2*}*-*

### Example 12

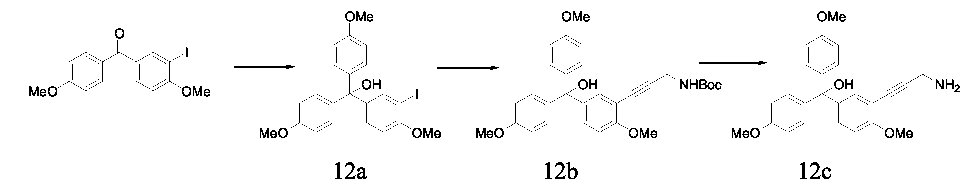

### 3-Iodo-4-methoxyphenyl-bis(4-methoxyphenyl)methanol.

To a solution of 3-iodo-4,4'-dimethoxybenzophenone (1.00 g, 2.72 mmol) in THF (20 mL) 0.86 M solution of 4-methoxyphenylmagnesium bromide in THF (6.2 mL, 5.44 mmol) was added in one portion. The mixture was kept at ambient temperature overnight, then diluted with saturated NaHCO₃ solution (200 mL) and extracted with EtOAc (200 mL). The organic layer was washed with water (3×200 mL) and dried over Na₂SO₄. The residue was chromatographed on silica gel in gradient of EtOAc in toluene (0 to 5%) with addition of 1% of Et₃N. Yield 1.00 g (77%), viscous yellowish oil. NMR (DMSO-*d*₆): 7.64 (d, 1H, ⁴*J*= 2.1 Hz, Ar*H*); 7.07 (d, 4H, *J*= 8.7 Hz, Ar*H*); 7.04 (d, 1H, *J*= 8.7 Hz, ⁴*J* = 2.1 Hz, Ar*H*); 6.90 (d, 1H, *J* = 8.7 Hz, Ar*H*); 6.85 (d, 4H, *J* = 8.7 Hz, Ar*H*); 6.24 (s, 1H, O*H*)*;* 3.80 (s, 3H), 3.73 (s, 6H) (OC*H*₃).

### N-(tert-Butyloxycarbonyl)-3-{2-methoxy-5-[hydroxy-bis(4-methoxyphenyl)methyl]-phenyl}-2-propynylamine.

To a solution of 3-iodo-4-methoxyphenyl-bis(4-methoxyphenyl)methanol (0.92 g, 1.93 mmol) and *N*-Boc-propargylamine (0.60 g, 3.86 mmol) in DMF (20 mL) Pd(PPh₃)₄ (223 mg, 0.193 mmol), CuI (74 mg, 0.386 mmol), Et₃N (404 µL, 2.90 mmol) were subsequently added. The mixture was stirred overnight under argon, then diluted with water (100 mL) and extracted with EtOAc (150 mL). The organic layer was washed with 0.1 M solution (NH₄)₂EDTA (4×100 mL), water (4×100 mL), and dried over Na₂SO₄. The residue was chromatographed on silica gel in gradient of EtOAc in toluene (0 to 30%) with addition of 1% of Et₃N. Yield 820 mg, viscous yellow oil (84%). NMR (DMSO-*d*₆): 7.25 (m, 1H, Ar*H*); 7.19-7.09 (m, 2H, ArH, N*H*); 7.07 (d, 4H, *J*= 8.8 Hz, Ar*H*); 6.95 (d, 1H, *J*= 8.8 Hz, Ar*H*); 6.84 (d, 4H, *J*= 8.8 Hz, Ar*H*); 3.93 (m, 2H, ≡CC*H*₂); 3.78 (s, 3H), 3.73 (s, 6H) (OC*H*₃); 1.37 (s, 9H, CC*H*₃).

### 3-{2-Methoxy-5-[hydroxy-bis(4-methoxyphenyl)methyl]phenyl-2-propynylamine.

To a solution of *N*-(*tert*-butyloxycarbonyl)-3-{2-methoxy-5-[hydroxy-bis(4-methoxyphenyl)methyl]phenyl-2-propynylamine (800 mg, 1.59 mmol) in DCM (5 mL) CF₃CO₂H (5 mL) was added. After completing of the deblocking (TLC control) the reaction mixture was evaporated in vacuo, dissolved in EtOAc (100 mL), washed with saturated aq. NaHCO₃ (3×100 mL), water (100 mL), and dried over Na₂SO₄. The residue was chromatographed on silica gel in gradient (0 to 20%) of methanol in toluene containing 1% Et₃N. Yield 385 mg, yellow foam (60%).

*L*_{*M*} *= -CH*_{*2*}*CH*_{*2*}*CH*₂-

### Example 13

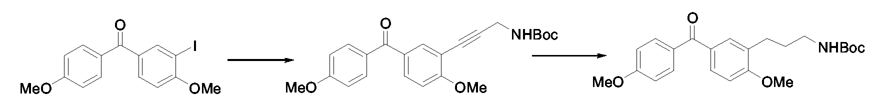

### 3-[3-(tert-Butyloxycarbonylamino)propyn-1-yl]-4,4'-dimethoxybenzophenone.

To a solution of 3-iodo-4,4'-dimethoxybenzophenone (2.50 g, 6.79 mmol) and N-Boc-propargylamine (1.26 g, 8.15 mmol) in DMF (100 mL) Pd(PPh₃)₄ (785 mg, 0.68 mmol), CuI (258 mg, 1.35 mmol), Et₃N (1.5 mL, 10.7 mmol) were subsequently added. The mixture was stirred overnight under argon, then diluted with water (200 mL) and extracted with EtOAc (200 mL). The organic layer was washed with 0.1 M solution (NH₄)₂EDTA (3×150 mL), water (3×150 mL), and dried over Na₂SO₄. The residue was chromatographed on silica gel in gradient of EtOAc in toluene (0 to 20%). Yield 1.83 g (68%), viscous yellowish oil. NMR (DMSO-*d*₆): 7.72 (dd, 1H, *J*= 8.8 Hz, ⁴*J*= 2.1 Hz, Ar*H*; 7.70 (d, 2H, *J*= 8.8 Hz, Ar*H*; 7.65 (d, 1H, ⁴*J*= 2.1 Hz, Ar*H*); 7.32 (br.s, 1H, N*H*); 7.20 (d, 1H, *J* = 8.8 Hz, Ar*H*); 7.08 (d, 2H, *J* = 8.8 Hz, Ar*H*); 4.00 (m, 2H, C*H*₂N); 3.91 (s, 3H), 3.86 (s, 3H) (OC*H*₃); 1.39 (s, 9H, CC*H*₃).

**3-[3-(*tert*-Butyloxycarbonylamino)propyl]-4,4'-dimethoxybenzophenone.** To a solution of 3-[3-(*tert*-butyloxycarbonylamino)propyn-1-yl]-4,4'-dimethoxybenzophenone (1.78 g, 4.5 mmol) in EtOAc (100 mL) 10%Pd/C (500 mg) was added and the mixture was hydrogenated for 24 h at ambient pressure. The mixture was filtered, evaporated and the residue was chromatographed on silica gel (0 to 20% gradient of EtOAc in toluene) to give the desired compound as a viscous colourless oil (980 mg, 55%). NMR (DMSO-*d*₆): 7.70 (d, 2H, *J*= 8.8 Hz, Ar*H*); 7.59 (m, 2H, Ar*H*); 7.06 (m, 3H, Ar*H*); 6.80 (br.s, 1H, N*H*); 3.87 (s, 3H), 3.85 (s, 3H) (OC*H*₃); 2.94 (m, 2H, C*H*₂NH); 2.60 (t, 2H, *J*= 7.6 Hz, ArC*H*₂); 1.66 (m, 2H, ArCH₂C*H*₂); 1.36 (s, 9H, CC*H*₃).

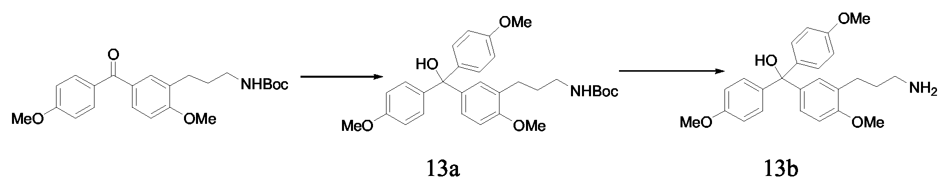

### 3-[3-(tert-Butyloxycarbonylamino)propyl]-4-methoxyphenyl-bis(4-methoxyphenyl)methanol.

To a solution of 3-[3-(*tert*-butyloxycarbonylamino)propyl]-4,4'-dimethoxybenzophenone (940 mg, 2.35 mmol) in THF (20 mL) 0.86 M solution of 4-methoxyphenylmagnesium bromide in THF (8.20 mL, 7.05 mmol) was added in one portion. The mixture was kept at ambient temperature overnight, then diluted with saturated NaHCO₃ solution (200 mL) and extracted with EtOAc (200 mL). The organic layer was washed with water (3×200 mL) and dried over Na₂SO₄. The residue was chromatographed on silica gel in gradient of EtOAc in toluene (0 to 20%) with addition of 1% of Et₃N. Yield 625 mg (52%), yellowish foam.

### 3-(3-Aminopropyn-1-yl)-4-methoxyphenyl-bis(4-methoxyphenyl)methanol.

To a stirred solution of 3-[3-(*tert*-butyloxycarbonylamino)propyl]-4-methoxyphenyl-bis(4-methoxyphenyl)methanol (610 mg; 1.20 mmol) in dry DCM (5 mL) trifluoroacetic acid (5 mL) was added in one portion and the mixture was stirred at ambient temperature for 5 h, then evaporated, and co-evaporated with DCM (4×20 mL) to give 415 mg (85%) of desired amine as an orange solid. NMR (DMSO-*d*₆): 7.08 (m, 6H, Ar*H*); 6.94 (m, 1H, Ar*H*); 6.85 (d, 4H, *J*= 9.0 Hz, Ar*H*); 6.18 (s, 1H, O*H*); 3.78 (s, 3H), 3.73 (s, 6H) (O*CH*₃); 3.46 (s, 2H, *CH*₂N); 1.86 (br. s, 2H, N*H*₂).

*L*_{*M*} *= -CH*_{*2*}*CH*_{*2*}*CON((CH*_{*2*}*)*_{*2*}*)*_{*2*}*CH-*

### Example 14

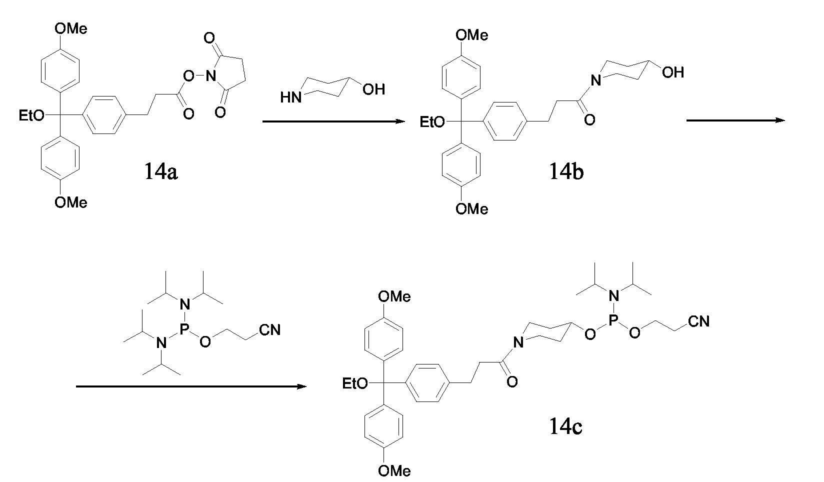

**1-(3-{4-[Ethoxy-bis(4-methoxyphenyl)-methyl]phenyl}propionyl)-4-hydroxypiperidine.** To a stirred solution of *N*-oxysuccinimide ester 3-{4-[ethoxy-bis(4-methoxyphenyl)methyl]phenyl}propionic acid (820 mg; 1.59 mmol) in dry DCM (50 mL) 4-hydroxypiperidine (322 mg, 3.18 mmol) was added and the mixture was stirred at ambient temperature 12 h (monitored by TLC in methanol-CHCl₃ 1:19). The reaction was diluted with CHCl₃ (100 mL), resulting solution was washed with water (2×100 mL) and dried over Na₂SO₄, evaporated, and chromatographed on silica gel in CHCl₃ with 2% MeOH and 1% Et₃N to give the desired compound as a colorless oil. Yield 410 mg (51%). NMR (DMSO-*d*₆): 7.23 (m, 6H, Ar*H*); 7.16 (d, 2H, *J*= 8.2 Hz, Ar*H*); 6.87 (d, 4H, *J*= 8.7 Hz, Ar*H*); 4.68 (d, 1H, *J*= 3.9 Hz, O*H*); 3.88 (m, 1H, H-2a (piperidine)); 3.73 (s, 6H, OC*H*₃); 3.68-3.56 (m, 2H, H-4, H-6a (piperidine)); 3.05 (m, 1H, H-6b (piperidine)); 3.02-2.92 (m, 3H, H-2b (piperidine), CH₃C*H*₂); 2.76 (m, 2H), 2.57 (m, 2H) (C*H*₂C*H*₂CO); 1.63 (m, 2H), 1.21 (m, 2H) (H-3, H-5 (piperidine)); 1.15 (t, 3H, *J* = 6.8 Hz, C*H*₃CH₂).

**1-(3-{4-[Ethoxy-bis(4-methoxyphenyl)-methyl]phenyl}propionyl)-4-(diisopropylamino-2-cyanethoxyphosphinoxy)piperidine.** To a stirred solution of *N*-(3-{4-[ethoxy-bis(4-methoxyphenyl)-methyl]phenyl}propionyl)-4-hydroxypiperidine (400 mg, 0.794 mmol) in dry acetonitrile (25 mL) diisopropylammonium tetrazolide (204 mg, 1.19 mmol) and bis(diisopropylamino)-2-cyanethoxyphosphine (281 mg, 1.19 mmol) were subsequently added under argon. The mixture was stirred at ambient temperature overnight (monitoring by TLC in Et₃N-CHCl₃ 1:9). After completion of the reaction the mixture was evaporated to dryness, dissolved in ethylacetate (100 mL), washed with water (100 mL), 5% NaHCO₃ (100 mL), and water (100 mL). The resulting solution was dried over Na₂SO₄, evaporated and chromatographed on silica gel in CHCl₃ with 1% of Et₃N to give the desired compound as a viscous colorless oil. Yield 290 mg (51%). ³¹P NMR (MeCN-*d*₃): 147.1452, 147.1085.

*L*_{*M*} *= -O-CH*_{*2*}*CH*_{*2*}*CH*_{*2*}*- (two L*_{*M*}*-containing Ar*^{*1*} *groups)*

### Example 15

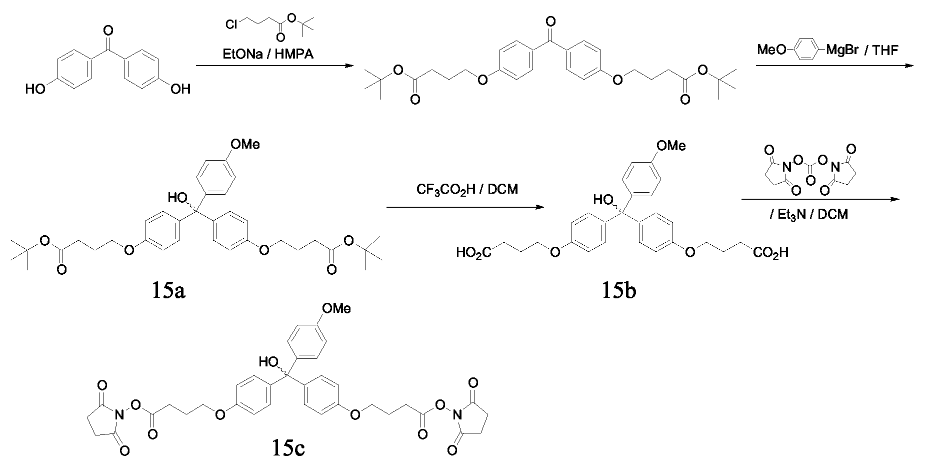

**4,4'-Bis[3-(*tert*-butyloxycarbonyl)propoxy]benzophenone.** Sodium (1.15 g, 50 mmol) was dissolved in MeOH and the resulting solution of MeONa was added to a solution of 4,4'-dihydroxybenzophenone (4.24 g, 20 mmol) in methanol (50 mL). The mixture was evaporated to dryness, the residue was dissolved in HMPA (30 mL) and *tert*-butyl 4-chlorobutyrate (8.04 g, 45 mmol) was added in one portion. The mixture was heated at 100°C for 5 h, then cooled, diluted with water (300 mL) and extracted with EtOAc (2x200 mL). The combined organic layers were washed with water (5x100 mL), dried over Na₂SO₄, and evaporated. The residue was chromatographed on silica gel in 2→5% EtOAc in toluene to give the desired compound as white crystalline solid, mp 92°C. Yield 5.49 g (55%). NMR (DMSO-*d*₆): 7.69 (d, 4H, *J*= 8.8 Hz, Ar*H*); 7.05 (d, 2H, *J*= 8.8 Hz, Ar*H*); 4.08 (t, 4H, *J*= 6.4 Hz, OC*H*₂); 2.38 (t, 4H, *J*= 7.3 Hz, COC*H*₂); 1.97 (m, 4H, CH₂C*H*₂CH₂); 1.41 (s, 18H, C*H*₃).

**4-Methoxy-4',4''-bis[3-(*tert*-butyloxycarbonyl)propoxy]tziphenylmethanol.** To a stirred solution of 4,4'-bis[3-(*tert*-butylocarbonyl)propoxy]benzophenone (2.49 g; 5.0 mmol) in dry (freshly distilled over sodium benzophenone ketyl) THF (30 mL) 1 M 4-methoxyphenyhnagnesium bromide (7.0 mL, 7.0 mmol) was added in one portion under Ar, and the mixture was kept at ambient temperature until completion of the reaction (TLC in EtOAc-toluene 1:4). The reaction was diluted with water (300 mL) and 5% citric acid (20 mL), and extracted with EtOAc (2x100 mL). The organic phase was dried over Na₂SO₄, evaporated, and chromatographed on silica gel in 3→7% EtOAc in toluene to give the desired compound as a colorless oil. Yield 1.88 g (62%).

**4-Methoxy-4',4"-bis(3-carboxypropoxy)triphenylmethanol.** To a stirred solution of 4,4'-bis[3-(*tert*-butyloxycarbonyl)propoxy]-4"-(4-methoxybenzoyl)tritanol (1.21 g; 2.0 mmol) in dry DCM (5 mL) trifluoroacetic acid (3 mL) was added in one portion and the mixture was stirred at ambient temperature for 3 h, then evaporated, and co-evaporated with DCM (4x50 mL) to give the desired acid (988 mg, 100%). NMR (DMSO-*d*₆): 7.10-7.04 (m, 6H, ArH (phenyl)); 6.86-6.81 (m, 6H, Ar*H* (phenyl)); 6.07 (s, 1H, O*H*)*;* 3.93 (m, 4H, OC*H*₂); 3.72 (s, 3H, OC*H*₃); 2.40-2.30 (m, 4H, COC*H*₂); 1.95-1.85 (m, 4H, CH₂C*H*₂C*H*₂), 1.40 (s, 18H, CC*H*₃).

**4-Methoxy-4',4''-bis{[3-(*N*-succinimidyl)oxycarbonyl]propoxy}triphenylmethanol.** To a stirred solution of 4,4'-bis(3-carboxypropoxy)-4"-(4-methoxybenzoyl)tritanol (742 mg; 1.5 mmol) in dry DCM (20 mL) triethylamine (0.56 mL, 4.0 mmol) and *N,N'*-disuccinimidyl carbonate (1.27 g, 5.0 mmol) were added and the mixture was stirred overnight, then evaporated, dissolved in EtOAc (150 mL), washed with 5% NaHCO₃ (50 mL) and water (50 mL), dried over Na₂SO₄, evaporated, and the residue was purified by column chromatography (20→30% EtOAc in toluene). Yield 826 mg (80%), white amorphous solid. *R*_{f} 0.18 (toluene-EtOAc, 1:1). NMR (DMSO-*d*₆): 7.10-7.04 (m, 6H, Ar*H* (phenyl)); 6.88-6.81 (m, 6H, Ar*H* (phenyl)); 6.09 (s, 1H, O*H*); 4.03 (t, 4H, *J =* 5.3 Hz, OC*H*₂); 3.73 (s, 3H, OC*H*₃); 2.87-2.77 (m, 12H, COC*H*₂C*H*₂CO, COC*H*₂); 2.11-2.03 (m, 4H, CH₂C*H*₂CH₂).

### Example 16

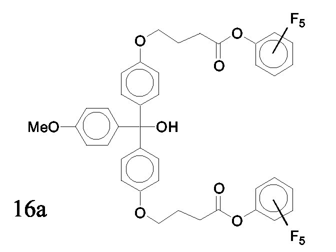

**4,4'-Bis[3-(pentafluorophenyloxyacarbonyl)propoxyl-4''-methoxy-tritanol.** This compound was made in the same way as the previous one except the pentafluorophenol was used instead of the N-hydroxysuccinimide. To a stirred solution of 4,4'-bis(3-carboxypropoxy)-4"-methoxy-tritanol (742 mg; 1.5 mmol) in dry DCM (20 mL) triethylamine (0.56 mL, 4.0 mmol) pentafluorophenyl carbonate (1.97 g, 5.0 mmol) were added and the mixture was stirred overnight, then evaporated, coevaporated with DCM and chromatographed on solica gel (5→10% EtOAc in toluene). Yield 980 mg (79%), dark red oil *R*_{f} 0.25 (toluene-EtOAc, 3:1).

*L*_{*M*} *= -CH*_{*2*}*CH*_{*2*}*CH*_{*2*}*N(Me)-C(O)-CH*_{*2*}*-*

### Example 17

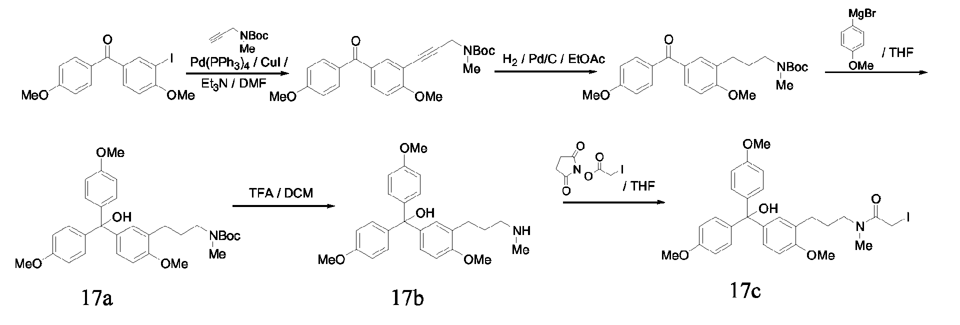

**3-[3-(N-Methyl-*tert*-butyloxycarbonylamino)propyn-1-yl]-4,4'-dimethoxybenzo-phenone.** To a solution of 3-iodo-4,4'-dimethoxybenzophenone (1.84 g, 5.00 mmol) and *N*-methyl-*N*-Boc-propargylamine (1.35 g, 8.0 mmol) in DMF (50 mL) Pd(PPh₃)₄ (577 mg, 0.50 mmol), CuI (191 mg, 1.0 mmol), Et₃N (1.5 mL, 10.7 mmol) were succesively added and the mixture was stirred overnight under Ar, then diluted with water (150 mL) and extracted with EtOAc (100 mL). The organic layer was washed with 0.1 M solution (NH₄)₂EDTA (3×50 mL), water (3×50 mL), and dried over Na₂SO₄. The residue was chromatographed on silica gel using 0→8% of EtOAc in toluene to give the desired compound as viscous yellowish oil (1.430 g, 70%).

**3-[3-(N-Methyl-*tert*-butyloxycarbonylamino)propyl]-4,4'-dimethoxybenzophenone.** To a solution of 3-[3-(N-Methyl-*tert*-butyloxycarbonylamino)propyn-1-yl]-4,4'-dimethoxybenzo-phenone (1.228 g, 3.0 mmol) in EtOAc (100 mL) 10%Pd/C (350 mg) was added and the mixture was hydrogenated for 24 h at ambient pressure. The mixture was filtered, evaporated and the residue was chromatographed on silica gel (0 to 10% gradient of EtOAc in toluene) to give the desired compound as a viscous colourless oil (920 mg, 74%). NMR (DMSO-*d*₆): 7.70 (d, 2H, *J* = 8.5 Hz, ArH, H-2', 6'); 7.59 (dd, 1H, J= 8.6 Hz, ⁴*J*= 2.1 Hz, Ar*H*, H-6); 7.56 (d, 1H, ⁴*J*= 2.1 Hz, Ar*H*, H-2); 7.10 (d, 1H, *J* = 8.6 Hz, Ar*H*, H-5); 7.07 (d, 2H, *J* = 8.5 Hz, Ar*H*, H-3', 5'); 3.89 (s, 3H), 3.86 (s, 3H) (OC*H*₃); 3.19 (t, 2H, *J*= 7.3 Hz, ArC*H*₂); 2.76 (br.s, 3H, NC*H*₃); 2.56 (t, 2H, *J*= 7.3 Hz, NC*H*₂); 1.73 (m, 2H, ArCH₂C*H*₂); 1.33 (br.s, 9H, CC*H*₃).

**3-[3-(N-Methyl-*tert*-butyloxycarbonylamino)propyl]-4-methoxyphenyl-bis(4-methoxyphenyl)methanol.** To a solution of 3-[3-(N-Methyl-*tert*-butyloxycarbonylamino)propyl]-4,4'-dimethoxybenzophenone (827 mg, 2.0 mmol) in THF (20 mL) 0.5 M solution of 4-methoxyphenyhnagnesium bromide in THF (5.0 mL, 2.5 mmol) was added in one portion. The mixture was kept at ambient temperature overnight, then quenched with water (1 mL), evaporated, the residue was partitioned between with saturated NaHCO₃ solution (150 mL) and EtOAc (150 mL). The organic layer was washed with water (2×100 mL) and dried over Na₂SO₄. The residue was chromatographed on silica gel in gradient of EtOAc in toluene (0 to 5%) with addition of 1% of Et₃N. Yield 876 mg (84%), yellowish foam.

**3-[3-Iodoacetyl(methyl]aminopropyl)-4-methoxyphenyl-bis(4-methoxyphenyl)methanol.** To a stirred solution of 3-{3-[*tert*-butyloxycarbonyl(methyl)amino]propyl}-4-methoxyphenyl-bis(4-methoxyphenyl)methanol (261 mg, 0.50 mmol) in dry DCM (2 mL) trifluoroacetic acid (2 mL) was added in one portion and the mixture was stirred at ambient temperature for 5 h, then evaporated, and co-evaporated with DCM (4×20 mL) to give desired amine as trifluoroacetic salt (orange solid). This was partitioned between toluene (150 mL) and 5% NaOH (150 mL). The organic phase was dried over Na₂SO₄ and evaporated. The residue was dissolved in dry THF (5 mL) iodoacetic acid *N*-oxysuccinimide ester (170 mg, 0.6 mmol) and DIEA (105 µL, 0.6 mmol) were added in one portion and the mixture was stirred at ambient temperature for 3 h, then evaporated, diluted with EtOAc (100 mL), washed with with 5% NaHCO₃ solution (50 mL), water (100 mL), dried over Na₂SO₄, and evaporated. The residue was chromatographed on silica gel in 5→15% EtOAc in toluene in the presence of 0.5% pyridine. Yield 221 mg (75%), yellowish foam.

*L*_{*M*} *= -CH*_{*2*}*CH*_{*2*}*CH*_{*2*}*N(Me)-C(O)-CH*_{*2*}*CH*_{*2*}*-*

### Example 18

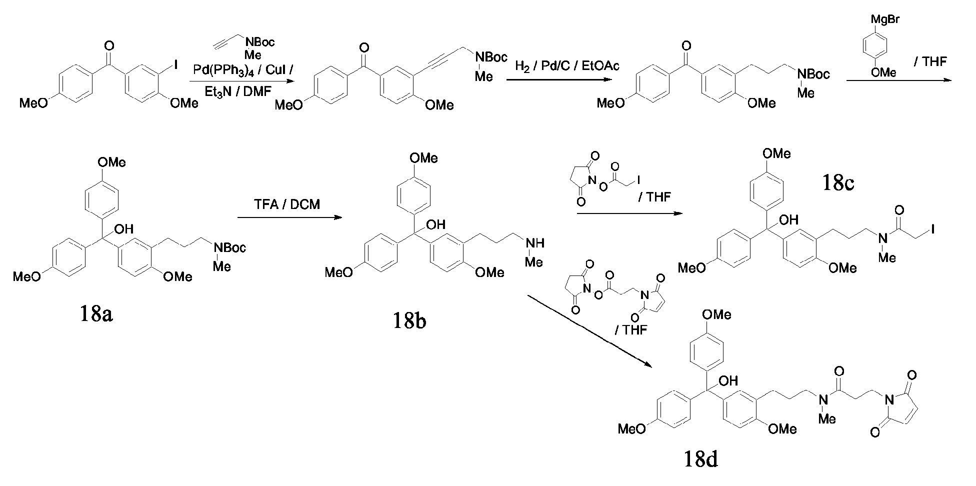

**3-{3-[*tert*-Butyloxycarbonyl(methyl)amino]propyn-1-yl}-4,4'-dimethoxybenzo-phenone.** To a solution of 3-iodo-4,4'-dimethoxybenzophenone (1.84 g, 5.00 mmol) and *N*-methyl-*N*-Boc-propargylamine (1.35 g, 8.0 mmol) in DMF (50 mL) Pd(PPh₃)₄ (577 mg, 0.50 mmol), CuI (191 mg, 1.0 mmol), Et₃N (1.5 mL, 10.7 mmol) were succesively added and the mixture was stirred overnight under Ar, then diluted with water (150 mL) and extracted with EtOAc (100 mL). The organic layer was washed with 0.1 M solution (NH₄)₂EDTA (3×50 mL), water (3×50 mL), and dried over Na₂SO₄. The residue was chromatographed on silica gel using 0→8% of EtOAc in toluene to give the desired compound as viscous yellowish oil (1.430 g, 70%).

**3-{3-[*tert*-Butyloxycarbonyl(methyl)amino]propyl}-4,4'-dimethoxybenzophenone.** To a solution of 3-{3-[*tert*-butyloxycarbonyl(methyl)amino]propyn-1-yl}-4,4'-dimethoxybenzo-phenone. (1.228 g, 3.0 mmol) in EtOAc (100 mL) 10%Pd/C (350 mg) was added and the mixture was hydrogenated for 24 h at ambient pressure. The mixture was filtered, evaporated and the residue was chromatographed on silica gel (0 to 100% gradient of EtOAc in toluene) to give the desired compound as a viscous colourless oil (920 mg, 74%).

**3-{3-[tert-Butyloxycarbonyl(methyl)amino]propyl}-4-methoxyphenyl-bis(4-methoxyphenyl)methanol.** To a solution of 3-{3-[*tert*-butyloxycarbonyl(methyl)amino]propyl}-4,4'-dimethoxybenzophenone (827 mg, 2.0 mmol) in THF (20 mL) 0.5 M solution of 4-methoxyphenyhnagnesium bromide in THF (5.0 mL, 2.5 mmol) was added in one portion. The mixture was kept at ambient temperature overnight, then quenched with water (1 mL), evaporated, the residue was partitioned between with saturated NaHCO₃ solution (150 mL) and EtOAc (150 mL). The organic layer was washed with water (2×100 mL) and dried over Na₂SO₄. The residue was chromatographed on silica gel in gradient of EtOAc in toluene (0 to 5%) with addition of 1% of Et₃N. Yield 876 mg (84%), yellowish foam.

**3-(3-Methylaminopropyl)-4-methoxyphenyl-bis(4-methoxyphenyl)methanol.** To a stirred solution of 3-{3-[*tert*-butyloxycarbonyl(methyl)amino]propyl}-4-methoxyphenyl-bis(4-methoxyphenyl)methanol (782 mg, 1.50 mmol) in dry DCM (5 mL) trifluoroacetic acid (5 mL) was added in one portion and the mixture was stirred at ambient temperature for 5 h, then evaporated, and co-evaporated with DCM (4×20 mL) to give 780 mg (96%) of desired amine as trifluoroacetic salt (orange solid).

**3-[3-Iodoacetyl(methyl]aminopropyl)-4-methoxyphenyl-bis(4-methoxyphenyl)methanol.** To a stirred solution of 3-(3-methylaminopropyl)-4-methoxyphenyl-bis(4-methoxyphenyl)methanol trifluoroacetate (267 mg, 0.50 mmol) in dry THF (5 mL) iodoacetic acid *N*-oxysuccinimide ester (170 mg, 0.6 mmol) and DIEA (105 µL, 0.6 mmol) were added in one portion and the mixture was stirred at ambient temperature for 3 h, then evaporated, diluted with EtOAc (100 mL), washed with with 5% NaHCO₃ solution (50 mL), water (100 mL), dried over Na₂SO₄, and evaporated. The residue was chromatographed on silica gel in 5→15% EtOAc in toluene in the presence of 0.5% pyridine. Yield 221 mg (75%), yellowish foam.

**3-(3-Maleimidopropyl)-4-methoxyphenyl-bis(4-methoxyphenyl)methanol.** To a stirred solution of 3-(3-methylaminopropyl)-4-methoxyphenyl-bis(4-methoxyphenyl)methanol trifluoroacetate (267 mg, 0.50 mmol) in dry THF (5 mL) 3-maleimidopropionic acid *N*-oxysuccinimide ester (136 mg, 0.6 mmol) and DIEA (105 µL, 0.6 mmol) were added in one portion and the mixture was stirred at ambient temperature for 5 h, then evaporated, diluted with EtOAc (100 mL), washed with with 5% NaHCO₃ solution (50 mL), water (100 mL), dried over Na₂SO₄, and evaporated. The residue was chromatographed on silica gel in 5→25% EtOAc in toluene in the presence of 0.5% pyridine. Yield 202 mg (71 %), yellowish foam.

*L*_{*M*} *= -Si(Me)*_{*2*}*-O-CH*_{*2*}*CH*_{*2*}*CH*_{*2*}*-*

### Example 19

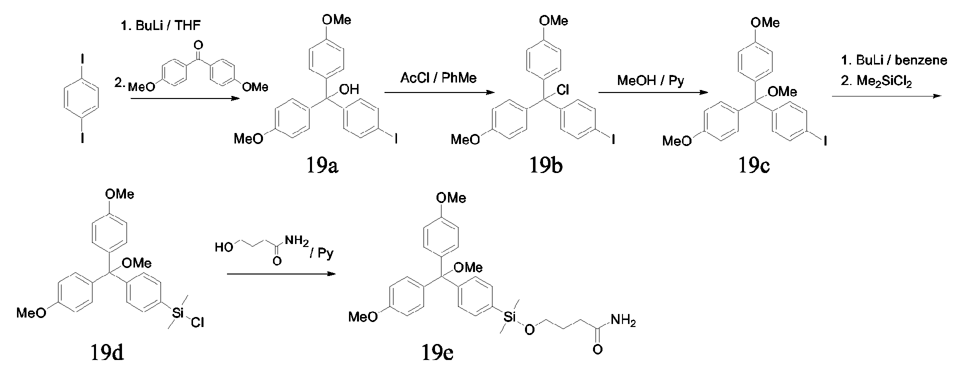

**4-Iodophenyl-bis(4-methoxyphenyl)methanol.** To a stirred and cooled to -70°C solution of 1,4-diiodobenzene (1.65 g, 5.0 mmol) in dry THF (50 mL) 1M BuLi in hexanes (10.0 mL, 10.0 mmol) was added dropwise within 30 min. The mixture was left for 1h at -70°C and the solution, then warmed to -40°C for 30 min and cooled to -70°C again. The solution of 4,4'-dimethoxybenzophenone (1.210 g, 5.0 mmol) in THF (50 mL) was added dropwise within 30 min. The mixture was allowed to warm to room temperature and stirred overnight at ambient temperature. The solution was evaporated to dryness, the residue was dissolved in EtOAc (200 mL), washed with 5% NaHCO₃ (100 mL), water (2×200 mL), dried with Na₂SO₄, and evaporated. The residue was dissolved in toluene containing 1% Et₃N (10 mL) and the desired tritanol was isolated by a column chromatography using stepwise gradient 0→2→4% EtOAc in toluene containing 0.5% Et₃N. Yield 915 mg (41%), colourless oil. NMR (DMSO-*d*₆): 7.64 (d, 2H, *J*= 8.4 Hz, Ar*H*); 7.07 (d, 4H, *J*= 8.8 Hz, Ar*H*); 7.00 (d, 2H, *J* = 8.4 Hz, Ar*H*); 6.84 (d, 4H, *J* = 8.8 Hz, Ar*H*); 6.28 (s, 1H, O*H*); 3.73 (s, 6H, C*H*₃).

**Methyl 4-iodophenyl-bis(4-methoxyphenyl)methyl ether.** A solution of 4-iodophenyl-bis(4-methoxyphenyl)methanol (670 mg, 1.5 mmol) in toluene (5 mL) and acetyl chloride (5 mL) was refluxed for 4 h, then cooled to room temperature, diluted with hexanes (150 mL) and cooled to -18°C. The solvent was decanted and the residue was washed with hexane (30 mL), dissolved in dry DCM (5 mL) and added dropwise to a mixture of MeOH (2mL) and pyridine (10 mL). The mixture was stirred for 1 h, then diluted with EtOAc (150 mL), washed with 5% NaHCO₃ (2×100 mL), water (2×100 mL), dried with Na₂SO₄, and evaporated and coevaporated with toluene (100 mL). The residue was chromatographed on silica gel in toluene containing 0.5% Et₃N to give the title compound (545 mg, 79%) as a white foam.

**Methyl 4-[(3-aminocarbonylpropoxy)dimethylsilyl]phenyl-bis(4-methoxyphenyl) methyl ether.** A solution of methyl 4-iodophenyl-bis(4-methoxyphenyl)methyl ether (460 mg, 1.0 mmol) in THF (10 mL) was cooled to -70°C and 1M BuLi in hexanes (1.1 mL, 1.1 mmol) was added dropwise within 10 min. The mixture was warmed and kept at -40°C for 1 h. The solution of dichlorodimethylsilane (130 mg, 1.0 mmol) in THF (2 mL) was added dropwise within 10 min. The mixture was allowed to warm to 0°C (approximately 1 h), and the solution of 4-hydroxybutyramide (206 mg, 2 mmol) in pyridine was added dropwise within 30 min. The mixture was allowed to warm to room temperature, evaporated, diluted with with EtOAc (100 mL), washed with 5% NaHCO₃ (2×50 mL), water (2×50 mL), dried with Na₂SO₄, and evaporated and coevaporated with toluene (100 mL). The residue was chromatographed on silica gel in 0→5→10% EtOAc in toluene containing 0.5% Et₃N to give the desired compound (235 mg, 48%) as a off white foam.

### Group M

*M = hydroxyl*

### Example 20

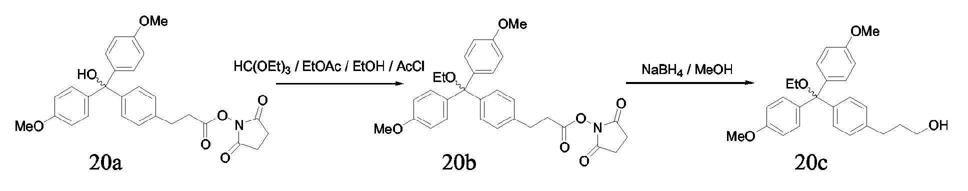

**3-{4-[Ethoxy-bis(4-methoxyphenyl)methyl]phenyl}propionic acid, *N*-oxysuccinimide ester. 3**-{4-[Hydroxy-bis(4-methoxyphenyl)methyl]phenyl}propionic acid, *N*-oxysuccinimide ester (978 mg, 2.0 mmol) was dissolved in ethyl acetate (50 mL) and absolute ethanol (5 mL). Triethyl orthoformiate (600 mg, 4 mmol) and acetyl chloride (25 µL) were added to the mixture and this was left with stirring overnight at ambient temperature. The mixture was evaporated and the residue was chromatographed on silica gel column in 30% EtOAc in toluene. Yield 724 mg (70%).

**3-{4-[Ethoxy-bis(4-methoxyphenyl)methyl]phenyl}propanol.** 3-{4-[Ethoxy-bis(4-methoxyphenyl)methyl]phenyl}propionic acid, *N*-oxysuccinimide ester (517 mg, 1.0 mmol) was dissolved in methanol (5 mL) and sodium borohydride (190 mg, 5.0 mmol) was added in one portion. The mixture was left overnight, then evaporated, diluted with water (100 mL) and ethyl acetate (100 mL). The organic layer was separated and washed with 5% NaHCO₃ (100 mL), then dried over Na₂SO₄ and evaporated. The residue was chromatographed on silica gel in 5→15% EtOAc in toluene. Yield 390 mg (96%), colourless oil.

### Example 21

Example 3 relates to compound 3a in which M is hydroxyl.

*M = amino*

### Example 22

Examples 12 and 13 relate to compounds 12c and 13b in which M is amino.

*M* = *methylamino*

### Example 23

Example 17 relates to compound 17b in which M is methylamino

*M* = *carboxyl or NHS ester groups*

### Example 24

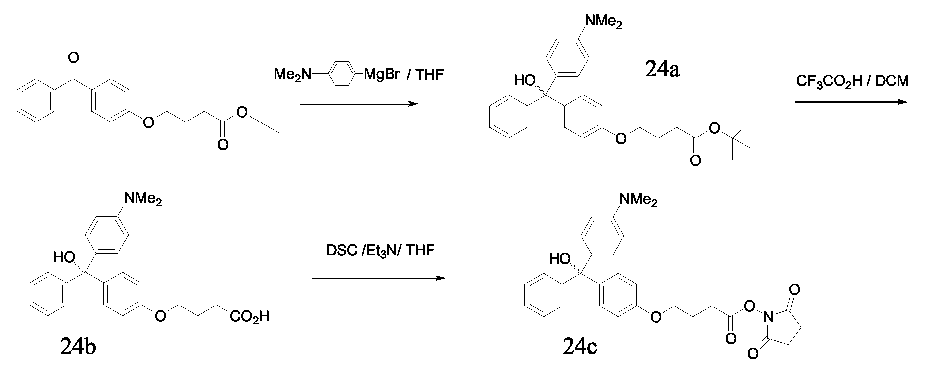

**4-{4-[Hydroxy-(4-dimethylaminophenyl)-phenyl-methyl]phenoxy}butanoic acid, *tert*-butyl ester.** To a stirred solution of 4-[3-(*tert*-butyloxycarbonyl)propoxy]benzophenone (1.36 g; 4.0 mmol) in dry THF (30 mL) 0.5 M 4-dimethylaminophenylmagnesium bromide (10.0 mL, 5.0 mmol) was added in one portion under Ar, and the mixture was kept at ambient temperature overnight (monitoring by TLC in EtOAc-toluene 1:9). The reaction was diluted with water (200 mL) and 5% NaHCO₃ (100 mL), and extracted with EtOAc (2×100 mL). The organic phase was dried over Na₂SO₄, evaporated, and chromatographed on silica gel in toluene with 1.0% of Et₃N to give the desired compound as a pinkish oil. Yield 1.809 g (98%). NMR (DMSO-*d*₆): 7.26 (m, 2H, Ar*H*); 7.19 (m, 3H, Ar*H*); 7.07 (d, 2H, *J*= 8.8 Hz, Ar*H*); 6.96 (d, 2H, *J*= 8.8 Hz, Ar*H*); 6.81 (d, 2H, *J*= 8.8 Hz, Ar*H*); 6.62 (d, 2H, *J*= 8.8 Hz, Ar*H*); 6.02 (s, 1H, O*H*); 3.94 (t, 2H, *J*= 6.3 Hz, OC*H*₂); 2.86 (s, 6H, NC*H*₃); 2.35 (t, 2H, *J*= 7.3 Hz, COC*H*₂); 1.91 (m, 2H, CH₂C*H*₂CH₂), 1.40 (s, 9H, CC*H*₃).

**4-{4-[Hydroxy-(4-dimethylaminophenyl)-phenyl-methyl]phenoxy}butanoic acid, *N*-oxysuccinimide ester.** To a stirred solution of *tert*-butyl 4-{4-[hydroxy-(4-dimethylaminophenyl)-phenyl-methyl]phenoxy}butanoate (923 mg; 2.0 mmol) in dry DCM (3 mL) trifluoroacetic acid (3 mL) was added in one portion and the mixture was stirred at ambient temperature for 1 h, then evaporated, and co-evaporated with DCM (4×50 mL) to give the desired acid. This was dissolved in DCM (40 mL), water (2 mL) and Et₃N (5 mL), washed with water (2×50 mL), dried over Na₂SO₄, and evaporated to dryness. The residue was dissolved in DCM (15 mL), triethylamine (0.7 mL, 5.0 mmol) and *N*,*N*'-disuccinimidyl carbonate (0.77 g, 3 mmol) were added and the mixture was stirred until the reaction is complete (monitoring by TLC in toluene-EtOAc, 2:1), then evaporated, dissolved in EtOAc (100 mL), washed with 5% NaHCO₃ (100 mL) and water (100 mL), dried with Na₂SO₄, evaporated, and the residue was purified by column chromatography (5→10→15% EtOAc in toluene). Yield 458 mg (43%), pink amorphous solid. NMR (DMSO-*d*₆): 7.26 (m, 2H, Ar*H*); 7.19 (m, 3H, Ar*H*); 7.07 (d, 2H, *J*= 8.7 Hz, Ar*H*); 6.96 (d, 2H, *J*= 8.7 Hz, Ar*H*); 6.84 (d, 2H, *J*= 8.7 Hz, Ar*H*); 6.62 (d, 2H, *J*= 8.7 Hz, Ar*H*); 6.03 (s, 1H, O*H*); 4.02 (t, 2H, *J*= 6.3 Hz, OC*H*₂); 2.88-2.79 (m, 12H, NC*H*₃, CH₂CH₂C*H*₂CO, COCH₂C*H*₂CO); 2.06 (m, 2H, CH₂C*H*₂CH₂).

*M = sulfoNHS ester*

### Example 25

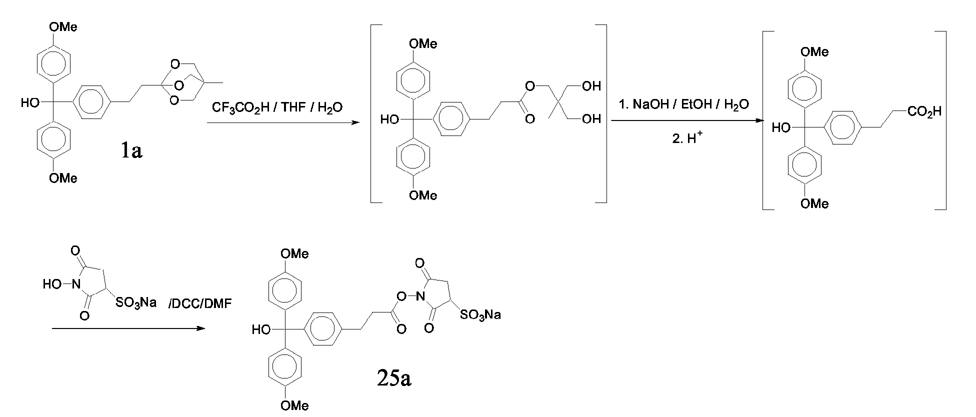

**3-{4-[Hydroxy-bis(4-methoxyphenyl)methyl]phenyl}propionic acid, *N*-oxysulfosuccinimide ester, sodium salt.** The starting 1-(2-{4-[hydroxy-bis(4-methoxyphenyl)methyl]phenyl}ethyl)-4-methyl-2,6,7-trioxabicyclo-[2.2.2]octane (0.96 g, 2.0 mmol) was hydrolysed as above (Example 1). The crude acid obtained was dissolved in dry DMF (20 mL), then *N*-oxysulfosuccinimide sodium salt (435 mg, 2.0 mmol) and DCC (678 mg, 3.3 mmol) were added and the mixture was left under stirring overnight, cooled to +4°C, and the precipitate formed was filtered off. The solution was diluted with dry Et₂O (300 mL) and the desired compound was collected by filtration, washed with ether and dried *in vacuo.* Yield 793 mg (67%), brown solid, *R*_{f} 0.54 (*n*-propanol-EtOAc-water, 8:1:1).

*M = amido*

### Example 26

Example 19 relates to compound 19e in which M is amido.

*M* = *aliphatic or aromatic iodide*

### Example 27

Example 17 relates to compound 17c in which M is an aliphatic iodide.

### Example 28

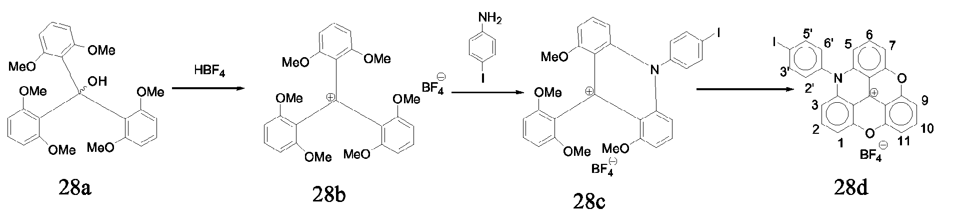

**Tris(2,6-dimethoxyphenyl)carbenium tetrafluoroborate** was prepared from 2,2',2",6,6',6"-hexamethoxytriphenylmethanol as described [Chem. Eur. J., 7(8), 1773-1783 (2001)], yield 96%.

**10-(4-lodophenyl)-9-(2,6-dimethoxyphenyl)acridinium tetrafluoroborate** was prepared similar to known procedure for corresponding methyl derivative [Tetrahedron Lett., **44**(1), 17-21 (2003)]. Tris(2,6-dimethoxyphenyl)carbenium tetrafluoroborate (250 mg, 0.49 mmol) and 4-iodoaniline (135 mg, 0.62 mmol) were suspended in 2,6-lutidine (2 mL). The mixture was refluxed for 2h under Ar, then cooled and diluted with ether-heptane (1:1, 30 mL). The precipitate was collected and washed with ether. The crude material (250 mg) was dissolved in MeCN (2 mL) and precipitated by succesive addition of ether (30 mL) and petroleum ether (15 mL). The precipitated oil was triturated to crystals in cooled petroleum ether. The crystalline solid was filtered off, washed with peproleum ether and dried *in vacuo.* Yield 209 mg (64%).

**4-(4-Iodophenyl)-4-aza-8,12-dioxa-4,8,12,12c-tetrahydrodibenzo[*cd*,*mn*]pyrenium tetrafluoroborate.** 10-(4-Iodophenyl)-9-(2,6-dimethoxyphenyl)acridinium tetrafluoroborate (118 mg, 0.18 mmol) and pyridinium hydrochloride (3.0 g) was heated at 170-180°C for 2h and poored into ice/water (25 mL). 50% HBF₄ (2 mL) was added and the precipitate formed was filtered off, washed with water (10 mL), and dried in desiccator over P₄O₁₀ overnight. Yield 93 mg (91%). NMR (DMSO-*d*₆): 8.32-8.21 (m, 5H, H-2,6,10,3',5'); 7.73 (d, 4H, *J*= 8.5 Hz, H-1,7,9,11); 7.49 (d, 2H, *J*= 8.2 Hz, H-2',6'); 6.95 (d, 2H, *J*= 8.5 Hz, H-3,5).

*M = pentafluorophenyl ester*

### Example 29

Example 16 relates to compound 16a in which M is a pentafluorophenyl ester group.

*M = maleimido group*

### Example 30

Example 18 relates to compound 18d in which M is a maleimido group.

### Example 30A

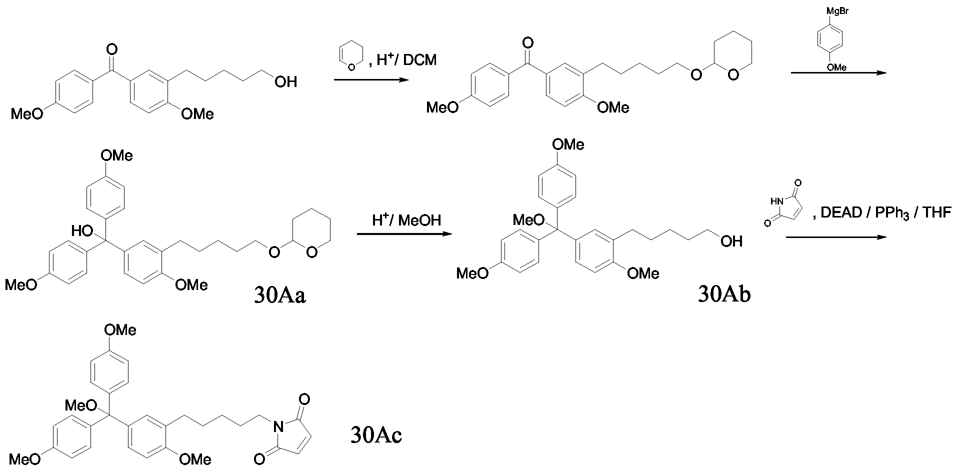

**3-[5-(2-Tetrahydropyranyl)oxypentyl]-4,4'-dimethoxybenzophenone.** To a solution of 3-(5-hydroxypentyl)-4,4'-dimethoxybenzophenone (542 mg, 1.65 mmol) in DCM (10 mL) DHP (0.17 mL, 1.82 mmol) and TsOH·H₂O (16 mg, 0.08 mmol) were added and the mixture was stirred for 1.5 h at ambient temperature (TLC control in 10% EtOAc in toluene). Solid NaHCO₃ (0.5 g) was added and after 5 min the mixture was filtetred, evaporated, and the residue was chromatographed on silica gel (3% EtOAc and 1% Et₃N in toluene) to give the desired compound as a colorless oil (441 mg, 65%). NMR (DMSO-*d*₆): 7.78 (d, 2H, *J*= 8.5 Hz, H-2',6'); 7.63 (m, 2H, ⁴*J*_{2,6} = 1.8 Hz, H-2,6); 6.95 (d, 2H, J= 8.5 Hz, ArH, H-3',5'); 6.87 (d, 1H, *J* = 8.2 Hz, H-5); 4.56 (m, 1H, OCHO); 3.92-3.82 (m, 7H, C*H*₃, OC*H*H); 3.73 (m, 1H), 3.48 (m, 3H), 3.38 (m, 3H) (OC*H*H); 2.64 (t, 2H, *J*= 7.6 Hz, ArC*H*₂); 1.88-1.38 (m, 12H, *O*CH₂C*H*₂C*H*₂C*H*₂).

**3-[5-(2-Tetrahydropyranyl)oxypentyl]-4,4',4"-trimethoxytritanol.** To a solution of 3-[5-(2-tetrahydropyranyl)oxypentyl]-4,4'-dimethoxybenzophenone (363 mg, 0.88 mmol) in THF (5 mL) 1.6 M 4-methoxyphenylmagnesium bromide in THF (0.75 mL, 1.2 mmol) in one portion and the mixture was kept overnight at ambient temperature. The mixture was quenched by addition of water (1 mL) and evaporated. The residue was dissolved in EtOAc (30 mL), washed with 5% NaHCO₃ (2×10 mL), dried over Na₂SO₄ and evaporated. The desired compound was isolated by column chromatography on silica gel (5% EtOAc + 1% Et₃N in toluene). The desired compound was isolated as a colourless oil (304 mg, 66%).

**5-{2-Methoxy-5-[methoxy-bis(4-methoxyphenyl)methyl]phenyl}pentanol.** To a stirred solution of 3-[5-(2-tetrahydropyranyl)oxypentyl]-4,4',4"-trimethoxytritanol (304 mg; 0.58 mmol) in MeOH (20 mL) TsOH·H₂O (10 mg) was added, and the mixture was kept at ambient temperature overnight (monitoring by TLC in EtOAc-toluene 1:9). Solid K₂CO₃ (300 mg) was added ant the mixture was evaporated and chromatographed on silica gel in 20% EtOAc + 1% Et₃N in toluene. Yield 254 mg (97%).

***N*-(5-{2-Methoxy-5-[methoxy-bis(4-methoxyphenyl)methyl]phenyl}pentyl)maleimide.** To a stirred solution of 5-{2-methoxy-5-[methoxy-bis(4-methoxyphenyl)methyl]-phenyl}pentanol (206 mg; 0.46 mmol) in THF (5 mL) maleimide (49 mg, 0.50 mmol), PPh₃ (131 mg, 0.5 mmol) were added followed by DEAD (0.08 mL, 0.53 mmol). The mixture was kept at ambient temperature for 24 h (monitoring by TLC in Me₂CO-toluene 1:9), evaporated and chromatographed on silica gel in 2% EtOAc + 1% Et₃N in toluene. Yield 37 mg (6%).

*M = aryldialkylsilyl chloride*

### Example 31

Example 19 relates to compound 19d in which M is a aryldialkylsilyl chloride group.

*M = trityl group*

### Example 32

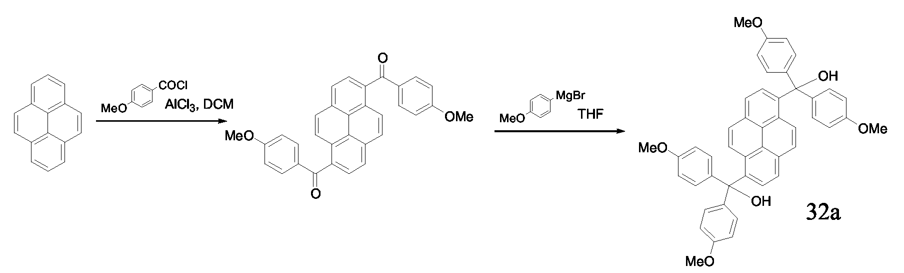

**1,6-Di(4-methoxybenzoyl)pyrene.** To an ice-cooled solution of pyrene (5.06 g, 25 mmol) and 4-methoxybenzoyl chloride (9.20 g, 54 mmol) in dichloromethane (150 mL) powdered AlCl₃ (8.00 g, 60 mmol) was added in small portions within 30 min, so that internal reaction mixture temperature was not higher than 5°C. The mixture was allowed to warm to room temperature and was left to react for 2 days, then poured on ice (300 g) and conc. HCl (50 mL). DCM layer was separated, and aqueous phase was extracted with EtOAc (2×150 mL). Combined organic layers were washed with 5% NaHCO₃ (200 mL), water (200 mL), dried over CaCl₂ and evaporated. The residue was applied to a silica gel column and eluted with CHCl₃ with 0→10% EtOAc. Fractions containing di(4-methoxybenzoyl)pyrenes (several isomers) were combined, evaporated, and the residue was crystallized twice from EtOAc to give the desired compound as brownish yellow solid. Yield 440 mg (3.7%). NMR (CDCl₃): 8.32 (d, 2H, *J* = 9.2 Hz, *H*-5,10 (pyrene)); 8.23 (d, 2H, *J* = 7.8 Hz, *H*-2,7 (pyrene)); 8.11 (d, 2H, *J*= 9.2 Hz, *H*-4,9 (pyrene)); 8.08 (d, 2H, *J*= 7.8 Hz, *H*-3,8 (pyrene)); 7.88 (d, 2H, *J* = 8.7 Hz, Ar*H* (phenyl)); 6.95 (d, 2H, *J* = 8.7 Hz, Ar*H* (phenyl)); 3.88 (s, 6H, OC*H*₃).

**1,6-Di[hydroxy-bis(4-methoxyphenyl)methyl]pyrene.** To a suspension 1,6-di(4-methoxybenzoyl)pyrene (235 mg, 0.5 mmol) in dry THF (20 mL) the solution of 0.9 M 4-methoxyphenylmagnesium bromide (1.45 mL, 1.0 mmol) was added in one portion under argon, and the mixture was refluxed for 3h, then cooled and evaporated. The residue was partitioned between water (200 mL) and EtOAc (200 mL); the organic phase was dried over Na₂SO₄, evaporated, and the residue was separated by column chromatography. The desired compound was obtained as a yellowish solid (46 mg, 13%). NMR (DMSO-*d*₆): 8.58 (d, 2H, *J*= 9.4 Hz, *H*-5,10 (pyrene)); 7.96 (d, 2H, *J*= 8.3 Hz, *H*-2,7 (pyrene)); 7.81 (d, 2H, *J*= 9.4 Hz, *H*-4,9 (pyrene)); 7.30 (d, 2H, *J*= 8.3 Hz, *H*-3,8 (pyrene)); 7.14 (d, 8H, *J* = 8.8 Hz, Ar*H* (phenyl)); 6.87 (m, 10H, Ar*H* (phenyl), O*H*); 3.74 (s, 12H, OC*H*₃).

*M = ester group*

### Example 33

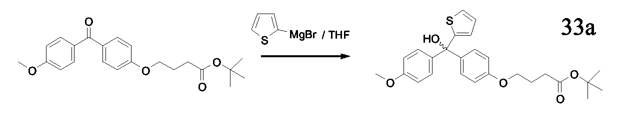

**4-{4-[Hydroxy-(thiophen-2-yl)-(4-methoxyphenyl)-methyl]phenoxy}butanoic acid, *tert*-butyl ester.** To a stirred solution of 4-[3-(*tert*-butyloxycarbonyl)propoxy]-4'-methoxybenzophenone (1.85 g; 5.0 mmol) in dry THF (50 mL) 1.0 M 2-thienylmagnesium bromide (7.5 mL, 7.5 mmol) was added in one portion under nitrogene, and the mixture was kept at ambient temperature overnight (monitoring by TLC in EtOAc-toluene 1:9). After addition of water (10 mL) the reaction mixture was evaporated, dissolved in EtOAc (200 mL) and washed with 5% NaHCO₃ (3×100 mL). The solution was dried over Na₂SO₄, evaporated, and chromatographed on silica gel in toluene with 1.0% of Et₃N to give the desired compound as a violet oil. Yield 1.33 g (58%). NMR (DMSO-*d*₆): 7.40 (m, 1H, H-5 (thiophene)); 7.15 (m, 4H, Ar*H* (phenyl)); 6.93 (m, 1H, H-4 (thiophene)); 6.84 (m, 4H, Ar*H* (phenyl)); 6.62 (m, 1H, H-3 (thiophene)); 6.52 (s, 1H, O*H*); 3.94 (t, 2H, *J*= 6.4 Hz, OC*H*₂); 3.73 (s, 3H, OC*H*₃); 2.35 (t, 2H, *J*= 7.3 Hz, COC*H*₂); 1.91 (m, 2H, CH₂C*H*₂CH₂), 1.39 (s, 9H, CC*H*₃).

*M* = *orthoester group*

### Example 34

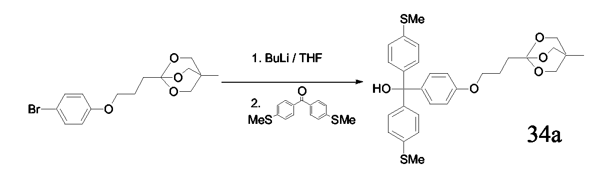

**4-{4-[Hydroxy-bis(4-methylthiophenyl)methyl]phenoxy{butyric acid, *N*-oxysuccinimide ester.** To a stirred and coolled to -70°C mixture of THF (40 mL) and 0.9M BuLi in hexane (11.1 mL, 10 mmol) the solution of 1-[3-(4-Bromophenoxy)propyl]-4-methyl-2,6,7-trioxabicyclo[2.2.2]octane (1.716 g, 5.0 mmol) in THF (50 mL) was added dropwise within 30 min. The mixture was left for 1h at -70°C and the solution of 4,4'-bis(methylthio)benzophenone (1.372 g, 5.0 mmol) in THF (50 mL) was added dropwise within 30 min. The mixture was allowed to warm to room temperature and stirred overnight at ambient temperature. The solution was evaporated to dryness, the residue was dissolved in EtOAc (200 mL), washed with 5% NaHCO₃ (2×200 mL), water (2×200 mL), dried with Na₂SO₄, and evaporated. The residue was dissolved in toluene containing 1% Et₃N (10 mL) and the desired tritanol ortho ester was isolated by a column chromatography. The column was eluted with stepwise gradient 8→12→15% EtOAc in toluene containing 1% Et₃N. The isolated 1-(3-{4-[hydroxy-bis(4-methylthiophenyl)methyl]phenyl}propoxy)-4-methyl-2,6,7-trioxabicyclo[2.2.2]octane (810 mg, 1.5 mmol, 30% yield) was dissolved in the THF-water mixture (19:1, 10mL), and trifluoroacetic acid (0.08 mL, 1 mmol) was added. After stirring for 10 min the mixture was evaporated, the residue was dissolved with EtOAc (200 mL), washed with 5% NaHCO₃ (100 mL) and water (2x100 mL), dried over Na₂SO₄, evaporated, diluted with 10 % NaOH in ethanol-water (8:2, 10mL), stirred overnight, then evaporated, coevaporated with water (2x50 mL), dissolved in water (100 mL), washed with ether (2x100 mL), and then acidified with solid citric acid to pH 4. The reaction mixture was extracted with EtOAc (2x100 mL), the combined organic layers were washed with water (50 mL), dried over Na₂SO₄ and evaporated to dryness. The resulting acid was dissolved in dry DCM (30 mL). Triethylamine (0.7 mL, 5 mmol) and *N,N'*-disuccinimidyl carbonate (0.51 g, 2.0 mmol) were added and the mixture was stirred until the reaction is complete (monitoring by TLC in toluene-EtOAc, 1:1), then evaporated, dissolved in EtOAc (200 mL), washed with 5% NaHCO₃ (100 mL) and water (100 mL), dried with Na₂SO₄, evaporated, and the residue was purified by column chromatography (10 to 40% EtOAc in toluene). Yield 348 mg (42% from ortho ester), white amorphous solid. NMR (DMSO-*d*₆): 7.18 (d, 4H, *J*= 8.5 Hz, Ar*H*); 7.12 (d, 4H, *J*= 8.5 Hz, Ar*H*); 7.07 (d, 2H, *J* = 8.9 Hz, Ar*H*); 6.87 (d, 2H, *J* = 8.9 Hz, Ar*H*); 6.27 (s, 1H, O*H*); 4.03 (t, 2H, *J* = 6.2 Hz, OC*H*₂); 2.87-2.79 (m, 6H, COC*H*₂, COC*H*₂C*H*₂CO); 2.45 (s, 6H, C*H*₃); 2.10-2.03 (m, 2H, CH₂C*H*₂CH₂).

*M = phosphoramidite group*

### Example 35

Example 14 relates to compound 14c in which M is a phosphoramidite group.

*M = -NHNH*_{*2*}

### Example 35A

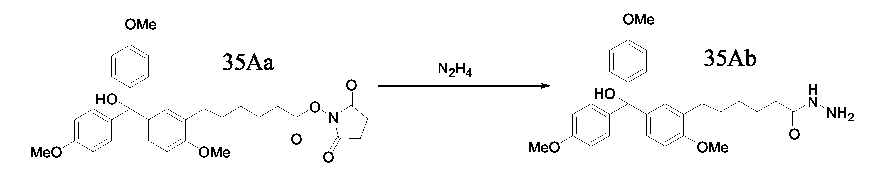

**6-{2-Methoxy-5-[hydroxy-bis(4-methoxyphenyl)methyl]phenyl}hexanoic acid hydrazide.** To a stirred solution of 6-{2-methoxy-5-[hydroxy-bis(4-methoxyphenyl)methyl]phenyl}hexanoic acid, *N*-oxysuccinimide ester (562 mg; 1.00 mmol) in THF (10 mL) hydrazine hydrate (120 mg, 2 mmol) was added in one portion and the mixture was stirred at ambient temperature for 1 h, then diluted with water (100 mL). The gummy precipitate was washed with water by decantation (4×50 mL) and dried in evacuated desiccator over P₄O₁₀ to give pure hydrazide as white solid. Yield 466 mg (97%). NMR (DMSO-*d*₆): 8.28 (br. s, 1H, CON*H*); 7.69 (d, 1H, *J* = 8.8 Hz, Ar*H*); 7.08 (d, 4H, *J* = 8.8 Hz, Ar*H*); 6.97 (d, 1H, ⁴*J* = 2.1 Hz, Ar*H*); 6.92-6.81 (m, 5H, Ar*H*); 6.00 (s, 1H, O*H*); 3.99 (br. s, 2H, N*H*₂); 3.88 (s, 3H), 3.85 (s, 6H) (OC*H*₃); 2.61 (t, 2H, *J* = 7.3 Hz), 2.46 (t, 2H, *J* = 7.6 Hz) (ArC*H*₂CH₂CH₂CH₂C*H*₂); 1.54 (m, 2H), 1.44 (m, 2H), 1.32 (m, 2H) (ArC*H*₂C*H*₂C*H*₂).

### Example 35B

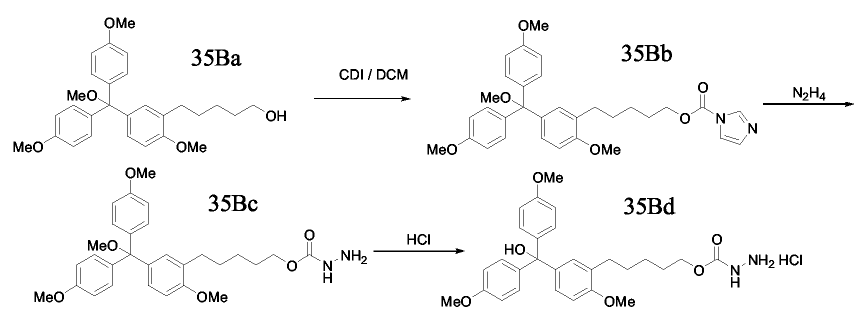

**5-{2-Methoxy-5-[methoxy-bis(4-methoxyphenyl)methyl]phenyl}pentyl carbazate.** To a stirred solution of 5-{2-methoxy-5-[methoxy-bis(4-methoxyphenyl)methyl]phenyl}pentanol (450 mg; 1.0 mmol) in DCM (20 mL) CDI (194 mg, 1.2 mmol) was added, and the mixture was stirred for 2 h at ambient temperature (monitoring by TLC in EtOAc-toluene 1:4). When formation of imidazolide is complete, hydrazine hydrate (100 µL) was added and the solution was stirred for 1 h, then diluted with water (150 mL), and extracted with toluene (3×50 mL). The combined organic layer was dried over Na₂SO₄, evaporated, dissolved in THF (10 mL), and 6 N HCl (200 µL) was added. The mixture was evaporated and coevaporated with toluene (3×50 mL) to give carbazate hydrochloride. Yield 466 mg (88%).

*M = -ONH*_{*2*}

### Example 35C

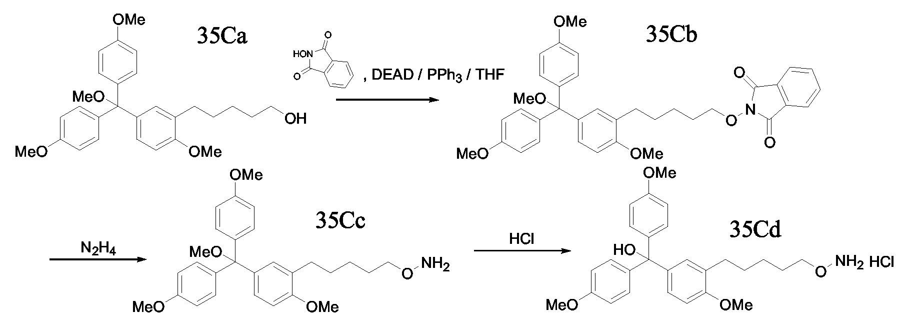

***N*-(5-{2-Methoxy-5-[methoxy-bis(4-methoxyphenyl)methyl]phenyl}pentyloxy)-phthalimide.** To a stirred solution of 5-{2-methoxy-5-[methoxy-bis(4-methoxyphenyl)methyl]-phenyl}pentanol (206 mg; 0.46 mmol) in THF (5 mL) *N*-hydroxyphthalimide (82 mg, 0.50 mmol), PPh₃ (131 mg, 0.5 mmol) were added followed by DEAD (0.08 mL, 0.53 mmol). The mixture was kept at ambient temperature for 24 h (monitoring by TLC in Me₂CO-toluene 1:9), evaporated and chromatographed on silica gel in 1→4% EtOAc + 1% Et₃N in toluene. Yield 182 mg (66%).

***O*-(5-{2-Methoxy-5-[methoxy-bis(4-methoxyphenyl)methyl]phenyl}pentyl)-hydroxylamine.** To a stirred solution of *N*-(5-{2-methoxy-5-[methoxy-bis(4-methoxyphenyl)methyl]phenyl}pentyloxy)phthalimide (120 mg; 0.20 mmol) in THF (2 mL) hydrazine hydrate (50 µL) was added and the solution was stirred for 48 h, then diluted with water (50 mL), and extracted with toluene (2×30 mL). The combined organic layer was dried over Na₂SO₄, evaporated, dissolved in THF (2 mL), and 6 N HCl (40 µL) was added. The mixture was evaporated and coevaporated with toluene (3×20 mL) to give pure hydroxylamine hydrochloride (87 mg, 90%).

*M = adamantyl (a hydrophobic group)*

### Example 36

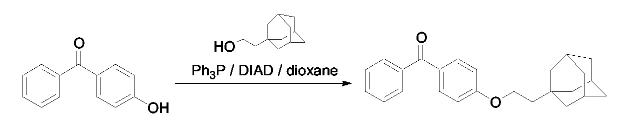

**4-[2-(Adamantan-1-yl)ethoxy]benzophenone.** In a round-bottom flask equipped with magnetic stirrer, room temperature water bath and dropping funnel 4-hydroxybenzophenone (1.98 g, 10 mmol), 1-adamantaneethanol (1.98 g, 11 mmol) and triphenylphosphine (3.15 g, 12 mmol) were dissolved in dry dioxane (50 mL). DIAD (2.46 ml, 12.5 mmol) was added dropwise within 20 min with stirring. The reaction mixture was heated to 40-45°C until starting benzophenone is consumed according to TLC (CHCl₃-MeOH 9:1), then cooled to room temperature; the precipitate formed was filtered off, washed successively with cold dioxane and MeOH and dried *in vacuo.* The solid was purified by column chromatography on silica gel (eluation with benzene). The appropriate fractions were combined and evaporated. The residue was triturated in petroleum ether to give the desired product (1.91 g, 53%), colorless crystals, mp 96°C. *R*_{f} 0.17 (toluene), 0.57 (toluene-EtOAc, 9:1), 0.64 (toluene-EtOAc, 4:1). NMR (DMSO-*d*₆): 7.74 (d, 2H, *J* = 8.7 Hz, Ar*H*); 7.70-7.62 (m, 3H, Ar*H*); 7.55 (m, 2H, Ar*H*); 7.07 (d, 2H, *J*= 8.7 Hz, Ar*H*); 4.14 (t, 2H, *J*= 7.2 Hz, OC*H*₂); 1.93 (br. s, 3H, H-3,5,7 (adamantane)); 1.70-1.60 (m, 6H, H-4,6,10 (adamantane)); 1.58-1.50 (m, 8H, H-2,8,9 (adamantane), OCH₂C*H*₂).

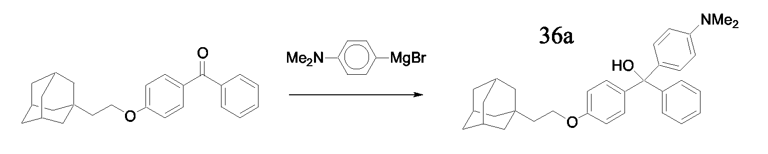

**4-[2-(Adamantan-1-yl)ethoxy]phenyl-(4-dimethylaminophenyl)-phenyl-methanol.** To a stirred solution of 4-[2-(adamantan-1-yl)ethoxy]benzophenone (721 mg; 2.0 mmol) in dry THF (20 mL) 0.5 M 4-dimethylaminophenylmagnesium bromide (6.0 mL, 3.0 mmol) was added in one portion under Ar, and the mixture was kept at ambient temperature overnight (monitoring by TLC in EtOAc-toluene 1:9). The reaction was diluted with water (100 mL) and 5% NaHCO₃ (50 mL), and extracted with EtOAc (2×50 mL). The organic phase was dried over Na₂SO₄, evaporated, and chromatographed on silica gel in toluene with 1.0% of Et₃N to give the desired compound as a pink oil. Yield 684 mg (71%). NMR (DMSO-*d*₆): 7.26 (m, 2H, Ar*H*); 7.20 (m, 3H, Ar*H*); 7.06 (d, 2H, *J*= 8.8 Hz, Ar*H*); 6.96 (d, 2H, *J*= 9.0 Hz, Ar*H*); 6.80 (d, 2H, *J*= 9.0 Hz, Ar*H*); 6.62 (d, 2H, *J*= 8.8 Hz, Ar*H*); 6.01 (s, 1H, O*H*); 3.98 (t, 2H, *J*= 7.1 Hz, OC*H*₂); 2.86 ( s, 6H, NC*H*₃); 1.92 (br. s, 3H, H-3,5,7 (adamantane)); 1.70-1.56 (m, 6H, H-4,6,10 (adamantane)); 1.55 (m, 6H, H-2,8,9 (adamantane)); 1.50 (m, 2H, OCH₂C*H*₂).

*M = phenylalkyl (a hydrophobic group)*

### Example 37

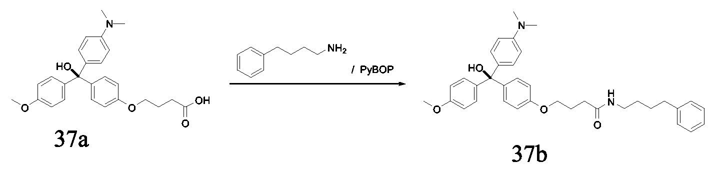

**4-{4-[Hydroxy-(4-dimethylaminophenyl)-(4-methoxyphenyl)-methyl]phenoxy}butanoic acid, 4-phenylbutylamide.** To a stirred solution of 4-{4-[hydroxy-(4-dimethylaminophenyl)-(4-methoxyphenyl)-methyl]phenoxy}butanoic acid (245 mg, 0.56 mmol) in dry DMF (10 mL) *N,N*-diisopropylethylamine (145 µL, 0.84 mmol) and PyBOP (350 mg, 0.67 mmol) were subsequently added. The mixture was stirred 10 min at ambient temperature, and 4-phenylbutylamine (167 µL, 1.12 mmol) was added. The mixture was stirred then 1 h, diluted with EtOAc (200 mL), washed with water (4×100 mL), dried over Na₂SO₄, and chromatographed on silica gel in toluene with 10→15% of EtOAc and 0.5% Et₃N. The product was obtained as pale pink oil (275 mg), yield 86%. NMR (DMSO-*d*₆): 7.80 (m, 1H, N*H*); 7.30-7.10 (m, 5H, Ar*H*); 7.06 (m, 4H, Ar*H*); 6.95 (d, 2H, *J*= 8.8 Hz, Ar*H*); 6.80 (m, 4H, Ar*H*); 6.61 (d, 2H, *J* = 8.8 Hz, Ar*H*); 5.91 (s, 1H, O*H*); 3.91 (t, 2H, *J* = 6.3 Hz, OC*H*₂); 3.72 (s, 3H, OC*H*₃); 3.06 (m, 2H, NC*H*₂); 2.85 (s, 6H, NC*H*₃); 2.55 (m, 2H, PhC*H*₂); 2.20 (t, 2H, *J* = 7.3 Hz, COC*H*₂); 1.91 (m, 2H, COCH₂C*H*₂), 1.54 (m, 2H, PhCH₂C*H*₂), 1.39 (m, 2H, NCH₂C*H*₂).

**4-{4-[Hydroxy-(4-dimethylaminophenyl)-(4-methoxyphenyl)-methyl]phenoxy} butanoic acid, 4-(acetylamino)butylamide.** NMR (DMSO-*d*₆): 7.81 (m, 1H, N*H*); 7.77 (m, 1H, N*H*); 7.06 (m, 4H, Ar*H*); 6.95 (d, 2H, *J*= 8.7 Hz, Ar*H*); 6.81 (m, 4H, Ar*H*); 6.61 (d, 2H, *J*= 8.7 Hz, Ar*H*); 5.92 (s, 1H, O*H*); 3.92 (t, 2H, *J*= 6.4 Hz, OC*H*₂); 3.72 (s, 3H, OC*H*₃); 3.00 (m, 4H, NC*H*₂); 2.85 (s, 6H, NC*H*₃); 2.21 (t, 2H, *J* = 7.3 Hz, COC*H*₂); 1.91 (m, 2H, COCH₂C*H*₂), 1.77 (s, 3H, COC*H*₃); 1.36 (m, 4H, NCH₂C*H*₂C*H*₂).

### Ar¹ and Ar²

*Ar*^{*1*}/*Ar*^{*2*} *=1-Pyrenyl*

### Example 38

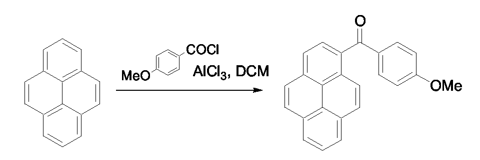

1-(4-Methoxybenzoyl)pyrene. To an ice-cooled solution of recrystallized pyrene (5.06 g, 25 mmol) and 4-methoxybenzoyl chloride (4.60 g, 27 mmol) in dichloromethane (50 mL) powdered AlCl₃ (4.00 g, 30 mmol) was added in small portions within 30 min, so that internal reaction mixture temperature was not higher than 5°C. The mixture was allowed to warm to room temperature, then poured on ice (200 g) and conc. HCl (25 mL). DCM layer was separated, and aqueous phase was extracted with DCM (2×40 mL). Combined organic layers were washed with 5% NaHCO₃ (100 mL), water (100 mL), dried over CaCl₂ and evaporated. The residue was purified by column chromatography on silica gel in CHCl₃ with 0→2% EtOAc to give the desired compound as light yellow crystals, mp 153°C, (NMR (DMSO-*d*₆): 8.41-8.05 (m, 9H, *H* (pyrene)); 7.77 (d, 2H, *J*= 9.0 Hz, Ar*H* (phenyl)); 7.06 (d, 2H, *J* = 9.0 Hz, Ar*H* (phenyl)); 3.85 (s, 3H, C*H*₃).

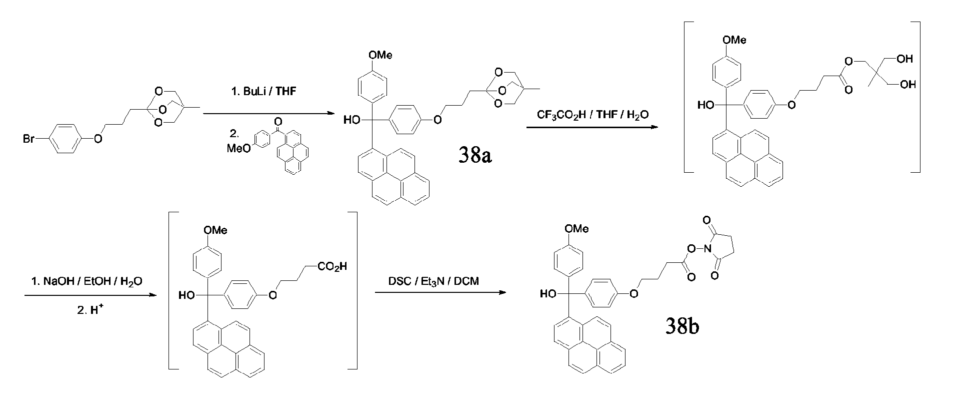

**1-(3-{4-[Hydroxy-4-methoxyphenyl-(pyren-1-yl)methyl]phenoxy}propyl)-4-methyl-2,6,7-trioxabicyclo[2.2.2]octane** was prepared using the above procedure for bis(methylthio) compound, yield 17%. NMR (DMSO-*d*₆): 8.60 (d, 1H, *J* = 9.6 Hz, *H-*10 (pyrene)); 8.25 (d, 1H, *J*= 7.6 Hz, *H*-6,8 (pyrene)); 8.17 (d, 1H, *J* = 7.6 Hz, *H*-6,8 (pyrene)); 8.14 (d, 1H, *J* = 9.0 Hz, *H*-2,3 (pyrene)); 8.10 (d, 1H, *J* = 9.0 Hz, *H*-2,3 (pyrene)); 8.08 (d, 1H, *J* = 8.0 Hz, *H*-4 or *H*-5 (pyrene)); 8.03 (t, 1H, *J* = 7.6 Hz, *H*-7 (pyrene)); 7.90 (d, 1H, *J* = 9.6 Hz, *H*-9 (pyrene)); 7.35 (d, 1H, *J* = 8.0 Hz, *H*-4 or *H-5* (pyrene)); 7.17-7.10 (m, 4H, Ar*H* (phenyl)); 6.90-6.82 (m, 4H, Ar*H* (phenyl)); 3.96-3.90 (m, 2H, ArOC*H*₂); 3.81 (s, 6H, OC*H*₂); 3.74 (s, 3H, OC*H*₃); 1.83-1.68 (m, 4H, OCH₂C*H*₂C*H*₂); 0.74 (s, 3H, C*H*₃). This was then hydrolysed and converted to **4-{4-[Hydroxy-4-methoxyphenyl-(pyren-1-yl)methyl]phenoxy} butyric acid, *N*-oxysuccinimide ester,** 46% yield from ortho ester. NMR (DMSO-*d*₆): 8.62 (d, 1H, *J* = 9.4 Hz, *H*-10 (pyrene)); 8.17 (d, 1H, *J* = 7.6 Hz, *H*-6,8 (pyrene)); 8.15 (d, 1H, *J* = 7.6 Hz, *H*-6,8 (pyrene)); 8.14 (d, 1H, *J* = 9.0 Hz, *H*-2,3 (pyrene)); 8.10 (d, 1H, *J* = 9.0 Hz, *H*-2,3 (pyrene)); 8.08 (d, 1H, *J* = 8.0 Hz, *H*-4 or *H*-5 (pyrene)); 8.03 (t, 1H, *J* = 7.6 Hz, *H*-7 (pyrene)); 7.91 (d, 1H, *J* = 9.4 Hz, *H*-9 (pyrene)); 7.36 (d, 1H, *J* = 8.2 Hz, *H*-4 or *H*-5 (pyrene)); 7.19-7.13 (m, 4H, Ar*H* (phenyl)); 6.92-6.85 (m, 4H, Ar*H* (phenyl)); 4.04 (t, 2H, *J* = 6.1 Hz, OC*H*₂); 3.75 (s, 3H, C*H*₃); 2.88-2.78 (m, 6H, COC*H*₂, COC*H*₂C*H*₂CO); 2.12-2.04 (m, 2H, CH₂C*H*₂CH₂).

### Example 39

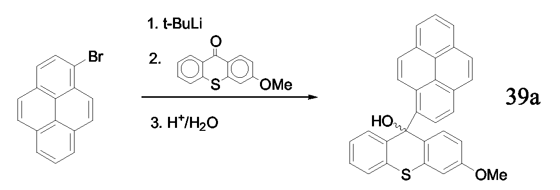

**9-Hydroxy-3-methoxy-9-(pyren-1-yl)thioxantene.** 1-Bromopyrene (1.12 g, 4.00 mmol) was dissolved in THF (40 mL), cooled to -80°C, and the solution of *t*-BuLi in pentane (1.4 M, 6 mL, 2.2 mmol) was added dropwise followed by 3-methoxyxanthen-9-one (968 mg, 4.00 mmol) in THF (40 mL). The mixture was allowed to warm to room temperature and the stirring was continued for 24 h. The mixture was quenched by addition of water (20 mL) and evaporated to 50 mL, and extracted with EtOAc (100 mL). The organic solution was washed with water (2×50 mL) and brine (50 mL), dried over Na₂SO₄, evaporated, and the residue was chromatographed on silica gel (1% Et₃N in toluene). Yield 944 mg (53%), pink foam. NMR (DMSO-*d*₆): 8.92 (d, 1H, *J* = 8.2 Hz, Ar*H*); 8.46 (d, 1H, *J* = 8.0 Hz, Ar*H*); 8.24 (m, 2H, Ar*H*); 8.16 (m, 1H, Ar*H*); 8.07 (m, 1H, Ar*H*); 7.99 (m, 1H, Ar*H*); 7.75 (d, 1H, *J* = 9.4 Hz, Ar*H*); 7.49 (m, 2H, Ar*H*); 7.20 (m, 1H, Ar*H*); 7.05 (d, 2H, ⁴*J* = 2.7 Hz, Ar*H*); 6.91 (m, 1H, Ar*H*); 6.79 (m, 1H, Ar*H*); 6.68 (d, 1H, *J* = 8.8 Hz, Ar*H*); 6.57 (s, 1H, O*H*); 6.51 (dd, 1H, ⁴*J* = 2.7 Hz, *J* = 8.8 Hz, Ar*H*); 3.72 (s, 6H, OC*H*₃).

### Example 40

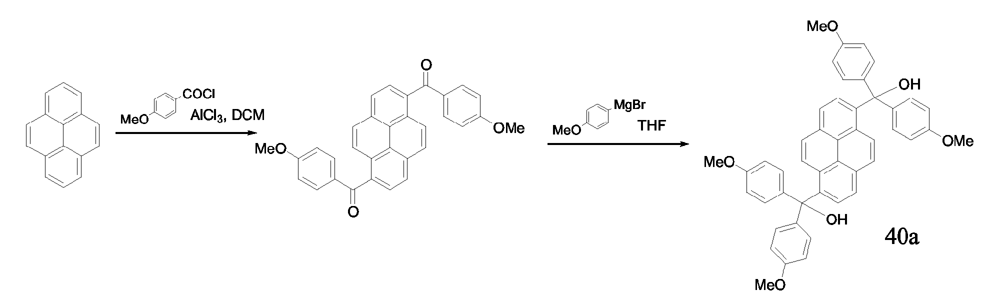

**1,6-Di(4-methoxybenzoyl)pyrene.** To an ice-cooled solution of pyrene (5.06 g, 25 mmol) and 4-methoxybenzoyl chloride (9.20 g, 54 mmol) in dichloromethane (150 mL) powdered AlCl₃ (8.00 g, 60 mmol) was added in small portions within 30 min, so that internal reaction mixture temperature was not higher than 5°C. The mixture was allowed to warm to room temperature and was left to react for 2 days, then poured on ice (300 g) and conc. HCl (50 mL). DCM layer was separated, and aqueous phase was extracted with EtOAc (2×150 mL). Combined organic layers were washed with 5% NaHCO₃ (200 mL), water (200 mL), dried over CaCl₂ and evaporated. The residue was applied to a silica gel column and eluted with CHCl₃ with 0→10% EtOAc. Fractions containing di(4-methoxybenzoyl)pyrenes (several isomers) were combined, evaporated, and the residue was crystallized twice from EtOAc to give the desired compound as brownish yellow solid. Yield 440 mg (3.7%). NMR (CDCl₃): 8.32 (d, 2H, *J* = 9.2 Hz, *H*-5,10 (pyrene)); 8.23 (d, 2H, *J* = 7.8 Hz, *H*-2,7 (pyrene)); 8.11 (d, 2H, *J* = 9.2 Hz, *H*-4,9 (pyrene)); 8.08 (d, 2H, *J* = 7.8 Hz, *H*-3,8 (pyrene)); 7.88 (d, 2H, *J* = 8.7 Hz, Ar*H* (phenyl)); 6.95 (d, 2H, *J* = 8.7 Hz, Ar*H* (phenyl)); 3.88 (s, 6H, OC*H*₃).

**1,6-Di[hydroxy-bis(4-methoxyphenyl)methyl]pyrene.** To a suspension 1,6-di(4-methoxybenzoyl)pyrene (235 mg, 0.5 mmol) in dry THF (20 mL) the solution of 0.9 M 4-methoxyphenylmagnesium bromide (1.45 mL, 1.0 mmol) was added in one portion under argon, and the mixture was refluxed for 3h, then cooled and evaporated. The residue was partitioned between water (200 mL) and EtOAc (200 mL); the organic phase was dried over Na₂SO₄, evaporated, and the residue was separated by column chromatography. The desired compound was obtained as a yellowish solid (46 mg, 13%). NMR (DMSO-*d*₆): 8.58 (d, 2H, *J* = 9.4 Hz, *H*-5,10 (pyrene)); 7.96 (d, 2H, *J* = 8.3 Hz, *H* 2,7 (pyrene)); 7.81 (d, 2H, *J* = 9.4 Hz, *H*-4,9 (pyrene)); 7.30 (d, 2H, *J* = 8.3 Hz, *H*-3,8 (pyrene)); 7.14 (d, 8H, *J* = 8.8 Hz, ArH (phenyl)); 6.87 (m, 10H, ArH (phenyl), O*H*); 3.74 (s, 12H, OCH₃).

### Example 41

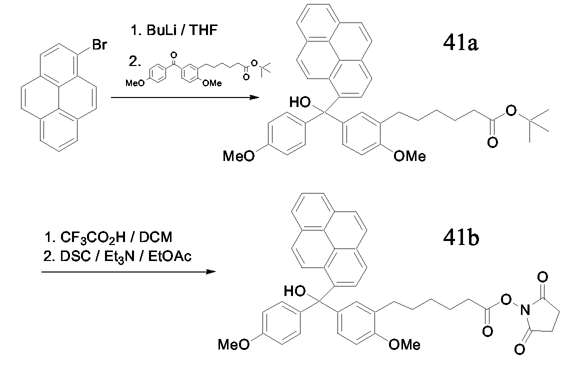

### 6-{2-Methoxy-5-[hydroxy-(4-methoxyphenyl)-(1-pyrenyl)-methyl]phenyl}hexanoic acid, tert-butyl ester.

To a stirred and coolled to -75°C mixture of 1.4M *tert*-BuLi in pentane (2.40 mL, 3.32 mmol) and

THF (20 mL) the solution of 1-bromopyrene (467 mg, 1.66 mmol) in THF (20 mL) was added. The temperature was raised to -40°C, then lowered again to -75°C, and the solution of 3-[5-(*tert*-butyloxycarbonyl)pentyl]-4,4'-dimethoxybenzophenone (685 mg, 1.66 mmol) in THF (40 mL) was added dropwise. The mixture was allowed to warm to room temperature and stirred overnight at ambient temperature. The solution was poured into water (150 mL), extracted with ETOAc (100 mL). Organic layer was washed with 5% NaHCO₃ (2×150 mL), water (100 mL), dried over Na₂SO₄, and evaporated. The residue was purified by column chromatography (toluene). Yield 750 mg (73%), yellowish foam. NMR (DMSO-*d*₆): 8.61 (d, 1H, *J*= 9.6 Hz, *H*-10 (pyrene)); 8.24 (d, 1H, *J*= 7.1 Hz), 8.17 (m, 1H) (*H*-6,8 (pyrene)); 8.11 (m, 2H, *H*-4,5 (pyrene)); 8.08 (m, 1H, *H*-2 or *H*-3 (pyrene)); 8.02 (t, 1H, *J* = 7.6 Hz, *H*-7 (pyrene)); 7.90 (d, 1H, *J* = 9.6 Hz, *H*-9 (pyrene)); 7.36 (d, 1H, *J* = 8.2 Hz, *H*-3 or *H*-2 (pyrene)); 7.16 (d, 2H, *J* = 8.8 Hz, *H*-2",6"); 7.08 (d, 1H, ⁴*J* = 2.3 Hz, *H*-2'); 6.93 (dd, 1H, J= 8.6 Hz, ⁴*J*= 2.3 Hz, *H*-6'); 6.87 (d, 2H, *J*= 8.8 Hz, *H-*3",5"); 6.86 (d, 1H, *J*= 8.6 Hz, *H-5');* 6.82 (s, 1H, O*H*); 3.76 (s, 3H), 3.74 (s, 3H) (OC*H*₃); 2.45 (m, 2H, ArC*H*₂); 2.01 (t, 2H, *J*= 7.4 Hz, COC*H*₂); 1.46-1.36 (m, 4H, ArCH₂C*H*₂CH₂C*H*₂); 1.35 (s, 9H, CC*H*₃); 1.12 (m, 2H, ArCH₂CH₂C*H*₂).

### 6-{2-Methoxy-5-[hydroxy-(4-methoxyphenyl)-(1-pyrenyl)-methyl]phenyl}hexanoic acid, N-oxysuccinimide ester.

To a stirred solution of tert-butyl ester of 6-{2-methoxy-5-[hydroxy-(4-methoxyphenyl)-(1-pyrenyl)-methyl]phenyl}hexanoic acid (730 mg; 1.19 mmol) in dry DCM (5 mL) trifluoroacetic acid (5 mL) was added in one portion and the mixture was stirred at ambient temperature for 3 h, then evaporated, and co-evaporated with DCM (3×20 mL) to give free acid. Product were dissolved in THF (40 mL), and triethylamine (495 µL, 3.57 mmol) and *N,N'*-disuccinimidyl carbonate (460 mg, 1.79 mmol) were added and the mixture was stirred overnight, then evaporated, dissolved in EtOAc (100 mL), washed with 5% NaHCO₃ (50 mL) and water (50 mL), dried over Na₂SO₄, evaporated, and the residue was purified by column chromatography (0→20% EtOAc in toluene). Yield 520 mg (67%), yellowish amorphous solid. 6-{2-Methoxy-5-[hydroxy-(4-methoxyphenyl)-(1-pyrenyl)-methyl]phenyl}hexanoic acid, *N*-oxysuccinimide ester. NMR (DMSO-*d*₆): 8.61 (d, 1H, *J*= 9.6 Hz, *H*-10 (pyrene)); 8.24 (d, 1H, *J*= 7.1 Hz, Ar*H* (pyrene)); 8.20-8.06 (m, 4H, ArH (pyrene)); 8.02 (t, 1H, *J*= 7.6 Hz, *H*-7 (pyrene)); 7.90 (d, 1H, *J*= 9.6 Hz, *H*-9 (pyrene)); 7.36 (d, 1H, *J*= 8.2 Hz, ArH (pyrene)); 7.16 (m, 2H, *H*-2",6"); 7.10 (d, 1H, ⁴*J*= 2.3 Hz, *H*-2'); 6.92 (dd, 1H, *J*= 8.7 Hz, ⁴*J*= 2.3 Hz, *H*-6'); 6.87 (d, 2H, *J*= 8.9 Hz, *H*-3",5"); 6.85 (d, 1H, *J*= 8.7 Hz, *H*-5'); 6.81 (s, 1H, O*H*); 3.76 (s, 3H), 3.74 (s, 3H) (OC*H*₃); 2.80 (s, 4H, COC*H*₂C*H*₂CO); 2.48 (m, 2H, ArC*H*₂, COC*H*₂); 1.52 (m, 2H), 1.45 (m, 2H), 1.23 (m, 2H) ArCH₂C*H*₂C*H*₂C*H*₂).

*Ar*^{*1*}/*Ar*^{*2*} = *2-Pyrenyl*

### Example 42

2-Bromopyrene was prepared according to published procedure (Harwey R.G. et al., Synth. Commun., 18(18), 2207-2209 (1988).

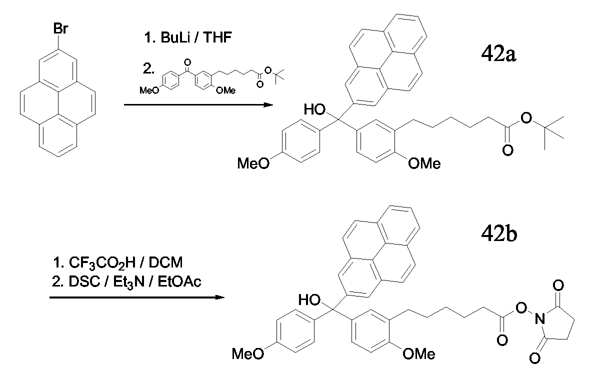

### 6-{2-Methoxy-5-[hydroxy-(4-methoxyphenyl)-(2-pyrenyl)-methyl]phenyl}hexanoic acid, tert-butyl ester.

To a stirred and cooled to -75°C mixture of 1.4M *tert*-BuLi in pentane (1.70 mL, 2.35 mmol) and THF (15 mL) the solution of 2-bromopyrene (326 mg, 1.16 mmol) in THF (15 mL) was added. The temperature was raised to -40°C, then lowered again to -75°C, and the solution of 3-[5-(*tert*-butyloxycarbonyl)pentyl]-4,4'-dimethoxybenzophenone (480 mg, 1.16 mmol) in THF (30 mL) was added dropwise. The mixture was allowed to warm to room temperature and stirred overnight at ambient temperature. The solution was poured into water (150 mL), extracted with ETOAc (100 mL). Organic layer was washed with 5% NaHCO₃ (2×100 mL), water (100 mL), dried over Na₂SO₄, and evaporated. The residue was purified by column chromatography (toluene). Yield 450 mg (63%), yellow foam.

### 6-{2-Methoxy-5-[hydroxy-(4-methoxyphenyl)-(2-pyrenyl)-methyl]phenyl}hexanoic acid, N-oxysuccinimide ester.

To a stirred solution of *tert*-butyl ester of 6-{2-methoxy-5-[hydroxy-(4-methoxyphenyl)-(2-pyrenyl)-methyl]phenyl}hexanoic acid (400 mg; 0.651 mmol) in dry DCM (5 mL) trifluoroacetic acid (5 mL) was added in one portion and the mixture was stirred at ambient temperature for 3 h, then evaporated, and co-evaporated with DCM (3×15 mL) to give free acid. Product were dissolved in THF (30 mL), and triethylamine (280 µL, 2.00 mmol) and *N,N'*-disuccinimidyl carbonate (250 mg, 0.973 mmol) were added and the mixture was stirred overnight, then evaporated, dissolved in EtOAc (100 mL), washed with 5% NaHCO₃ (40 mL) and water (40 mL), dried over Na₂SO₄, evaporated, and the residue was purified by column chromatography (0→20% EtOAc in toluene). Yield 162 mg (38%), yellowish amorphous solid.

*Ar1*/*Ar2 = 4-Pyrenyl*

### Example 43

### 4-Bromopyrene was prepared as described from 1,2,3,6,7,8-hexahydropyrene (JOC, 44 (1979), No. 13, 2158-2160)

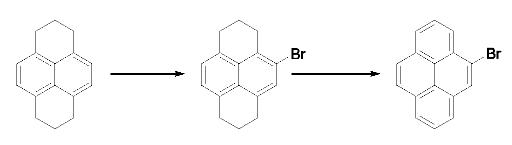

### 4-Bromo-1,2,3,6,7,8-hexahydropyrene.

To a suspension of 1,2,3,6,7,8-hexahydropyrene (6.06g, 29.1 mmol) in AcOH (75 mL) a solution of bromine (1.57 mL, 30 mmol) in AcOH (75 mL) was added dropwise within 1 h. The mixture was kept at room temperature for 30 min, then heated to 100°C (the precipitate dissolves), then cooled and diluted with water (75 mL). The solid was filtered off and dried. Yield ca 90%.

**4-Bromopyrene.** To a solution 4-bromo-1,2,3,6,7,8-hexahydropyrene (2.01 g, 7.0 mmol) in toluene (135 mL) DDQ (5.30 g, 23 mmol) was added in one portion, the flask was flushed with argon and the mixture was refluxed for 20 min, then cooled and filtered. The solution was washed with 10% NaOH (30mL), water (50 mL), and dried over CaCl₂, then evaporated and the residue was flash chromatographed on silica gel in toluene. Yield 1.14 g (58%).

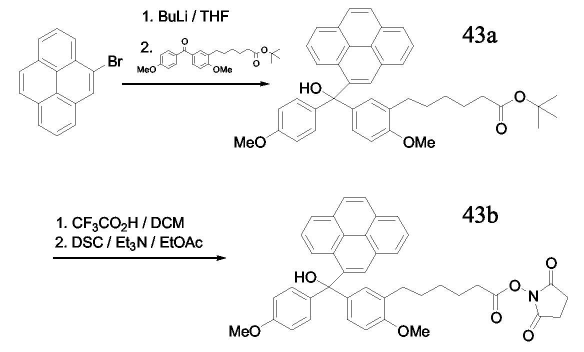

### 6-{2-Methoxy-5-[hydroxy-(4-methoxyphenyl)-(4-pyrenyl)-methyl]phenyl{hexanoic acid, tert-butyl ester.

To a stirred and cooled to -75°C mixture of 1.4M *tert*-BuLi in pentane (2.40 mL, 3.32 mmol) and THF (20 mL) the solution of 1-bromopyrene (467 mg, 1.66 mmol) in THF (20 mL) was added. The temperature was raised to -40°C, then lowered again to -75°C, and the solution of 3-[5-(*tert*-butyloxycarbonyl)pentyl]-4,4'-dimethoxybenzophenone (685 mg, 1.66 mmol) in THF (40 mL) was added dropwise. The mixture was allowed to warm to room temperature and stirred overnight at ambient temperature. The solution was poured into water (150 mL), extracted with ETOAc (100 mL). Organic layer was washed with 5% NaHCO₃ (2×150 mL), water (100 mL), dried over Na₂SO₄, and evaporated. The residue was purified by column chromatography (toluene). Yield 610 mg (60%), yellow foam. NMR (DMSO-*d*₆): 8.66 (d, 1H, *J*= 8.0 Hz, *H*-3 (pyrene)); 8.25 (dd, 1H, *J*= 7.1 Hz, ⁴*J* = 1.6 Hz, *H*-6 or *H*-8 (pyrene)); 8.16 (d, 1H, *J* = 7.1 Hz, *H*-1 (pyrene)); 8.14 (m, 2H, *J* = 9.1 Hz, *H*-9,10 (pyrene)); 7.99-7.93 (m, 2H, *H*-7, *H*-8 or *H*-6 (pyrene)); 7.80 (t, 1H, J= 7.9 Hz, *H*-2 (pyrene)); 7.42 (s, 1H, *H*-5 (pyrene)); 7.26 (d, 2H, *J*= 8.2 Hz, *H*-2",6"); 7.18 (m, 1H, *H*-2'); 7.03 (d, 1H, *J*= 8.2 Hz, *H*-6'); 6.88 (d, 2H, *J*= 8.2 Hz, *H*-3",5"); 6.86 (d, 1H, *J*= 8.7 Hz, *H*-5'); 6.76 (s, 1H, O*H*); 3.76 (s, 3H), 3.75 (s, 3H) (OC*H*₃); 2.46 (m, 2H, ArC*H*₂); 2.03 (t, 2H, *J*= 7.4 Hz, COC*H*₂); 1.47-1.35 (m, 4H, ArCH₂C*H*₂CH₂C*H*₂); 1.34 (s, 9H, CC*H*₃); 1.13 (m, 2H, ArCH₂CH₂C*H*₂).

### 6-{2-Methoxy-5-[hydroxy-(4-methoxyphenyl)-(4-pyrenyl)-methyl]phenyl}hexanoic acid, N-oxysuccinimide ester.

To a stirred solution of tert-butyl ester of 6-{2-methoxy-5-[hydroxy-(4-methoxyphenyl)-(4-pyrenyl)-methyl]phenyl}hexanoic acid (600 mg; 0.976 mmol) in dry DCM (5 mL) trifluoroacetic acid (5 mL) was added in one portion and the mixture was stirred at ambient temperature for 3 h, then evaporated, and co-evaporated with DCM (3×20 mL) to give free acid. Product were dissolved in THF (40 mL), and triethylamine (410 µL, 2.93 mmol) and *N*,*N*'-disuccinimidyl carbonate (375 mg, 1.46 mmol) were added and the mixture was stirred overnight, then evaporated, dissolved in EtOAc (100 mL), washed with 5% NaHCO₃ (50 mL) and water (50 mL), dried over Na₂SO₄, evaporated, and the residue was purified by column chromatography (0→20% EtOAc in toluene). Yield 230 mg (36%), yellowish amorphous solid. 6-{2-Methoxy-5-[hydroxy-(4-methoxyphenyl)-(4-pyrenyl)-methyl]phenyl}hexanoic acid, *N*-oxysuccinimide ester. NMR (DMSO-*d*₆): 8.67 (d, 1H, *J=* 8.0 Hz, *H*-3 (pyrene)); 8.24 (dd, 1H, *J* = 6.6 Hz, ⁴*J =* 1.8 Hz, *H*-6 or *H*-8 (pyrene)); 8.17 (d, 1H, *J* = 7.6 Hz, *H-*1 (pyrene)); 8.14 (m, 2H, *H-*9,10 (pyrene)); 7.96 (m, 2H, *H*-7, *H*-8 or *H*-6 (pyrene)); 7.81 (t, 1H, J = 7.9 Hz, H-2 (pyrene)); 7.44 (s, 1H, *H*-5 (pyrene)); 7.27 (d, 2H, *J=* 8.2 Hz, *H*-2",6"); 7.20 (m, 1H, *H*-2'); 7.05 (m, 1H, *H*-6'); 6.89 (d, 2H, *J*= 8.9 Hz, *H*-3",5"); 6.87 (d, 1H, J= 8.6 Hz, *H-5*')*;* 6.77 (s, 1H, O*H*); 3.77 (s, 3H), 3.75 (s, 3H) (OC*H*₃); 2.79 (s, 4H, COC*H*₂C*H*₂CO); 2.49 (m, 2H, ArC*H*₂, COC*H*₂); 1.53 (m, 2H), 1.45 (m, 2H), 1.23 (m, 2H) ArCH₂C*H*₂C*H*₂C*H*₂).

*Ar1*/*Ar2 = cyclopropyl*

### Example 44

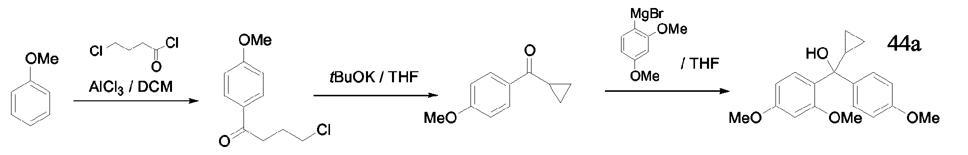

**4-Chloro-4'-methoxybutyrophenone.** To a stirred, ice cooled solution of anisole (5.41 g, 50 mmol) and 4-chlorobutyryl chloride (7.8 g, 55 mmol) in dry DCM (200 mL) anhydrous AlCl₃ (7.60 g, 57 mmol) was added within 1 h maintaining the temperature below 5°C. The mixture was allowed to warm to 15°C (2 h), and then was poured on ice (300 g). Conc. HCl (10 mL) was added, the organic layer was separated, washed with 5% NaHCO₃ (200 mL),dried over CaCl₂, and evaporated. The residue was purified by column chromatography in toluene to give the desired ketone as a colourless oil which solidifies on storage (9.55g, 90%).

**Cyclopropyl-(4-methoxyphenyl)-ketone.** To a solution of 4-chloro-4'-methoxybutyrophenone (2.13 g, 10 mmol) in dry THF (50 mL) potassium *tert*-butoxide (1.23 g, 11 mmol) was added and the mixture was refluxed for 1h, then cooled and evporated. The residue was dissolved in EtOAc (100 mL), washed with 5% citric acid (50 mL) and water (50 mL), dried over CaCl₂, and evaporated. The residue was purified by column chromatography in toluene to give the desired ketone as a colourless crystalline solid (1.34g, 76%).

**(2,4-Dimethoxyphenyl)-cyclopropyl-(4-methoxyphenyl)-methanol.** To a solution of cyclopropyl-(4-methoxyphenyl)-ketone (176 mg, 1.0 mmol) in dry THF (10 mL) 0.5 M 2,4-dimethoxyphenylmagnesium bromide in THF (2.5 mL, 1.25 mmol) was added dropwise within 15 min via a suringe under Ar. The mixture was kept at ambient temperature for 3h. TLC (3% EtOAc in toluene showed the starting compound is consumed. The mixture was quenched by addition of water (1 mL) and evaporated. The residue was dissolved in EtOAc (100 mL), washed with 5% NaHCO₃ (2×50 mL), dried over NaHCO₃ and evaporated. The desired compound was isolated by column chromatography on silica gel (0→1% EtOAc in toluene). Yield 302 mg (96 colourless crystalline solid.

*Ar*^{*1*}/*Ar*^{*2*} *= Phenyl*

### Example 45

Example 24 relates to compounds 24a, 24b and 24c in which Ar¹ and Ar² are phenyl groups.

*Ar*^{*1*}/*Ar*^{*2*} *= 9-acridinium*

### Example 46

Example 28 relates to compound 28c in which Ar¹ and Ar² is a 9-acridinium group.

*Ar*^{*1*}/*Ar*^{*2*} *= 4-Aza-8*,*12-dioxa-4,8,12,12c-tetrahydrodibenzo[*cd,*mn]pyrenium*

### Example 47

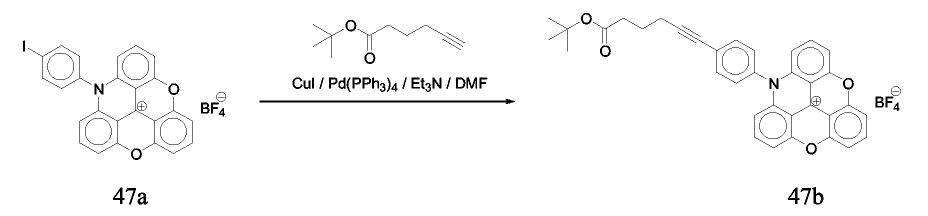

**4-[4-(5-tert-Butyloxycarbonylpentyn-1-yl)phenyl]-4-aza-8,12-dioxa-4,8,12,12c-tetrahydrodibenzo[*cd*,*mn*]pyrenium** tetrafluoroborate. To a solution of 4-(4-iodophenyl)-4-aza-8,12-dioxa-4,8,12,12c-tetrahydrodibenzo[*cd*,*mn*]pyrenium tetrafluoroborate (34 mg, 0.06 mmol; see Example RFG9 in Section 2) in DMF (2.0 mL) *tert*-butyl 5-hexynoate (42 mg, 0.23 mmol), triethylamine (20 µL, 0.13 mmol), Pd(PPh₃)₄ (14 mg, 0.012 mmol) and CuI (10 mg, 0.04 mmol) were added, and the reaction mixture was stirred for 24 h at room temperature under Ar. The reaction was monitored by TLC in 25% MeOH in DCM. Then the mixture was diluted with ether (15 mL), the precipitate was filtered off and purified by preparative TLC eluted with 20% MeOH in CHCl₃. Yield 19 mg (52%). NMR (DMSO-*d*₆): 8.28 (t, 1H, J= 8.5 Hz, *H*-10); 8.25 (t, 2H, J= 8.5 Hz, *H*-2,6); 7.91 (d, 2H, *J* = 8.5 Hz, Ar*H* (C₆H₄)); 7.74 (d, 4H, J = 8.5 Hz, *H*-1,7,9,11); 7.67 (d, 2H, J = 8.5 Hz, ArH (C₆H₄)); 6.93 (d, 2H, *J =* 8.5 Hz, *H*-3,5); 2.56 (t, 2H, *J* = 7.1 Hz), 2.42 (t, 2H, *J* = 7.4 Hz), (CH₂CH₂C*H*₂); 1.83 (m, 2H, CH₂C*H*₂CH₂); 1.43 (s, 9H, C*H*₃).

*Ar*^{*1*}*lAr*^{*2*} *= Thioxanthen-9-yl*

### Example 48

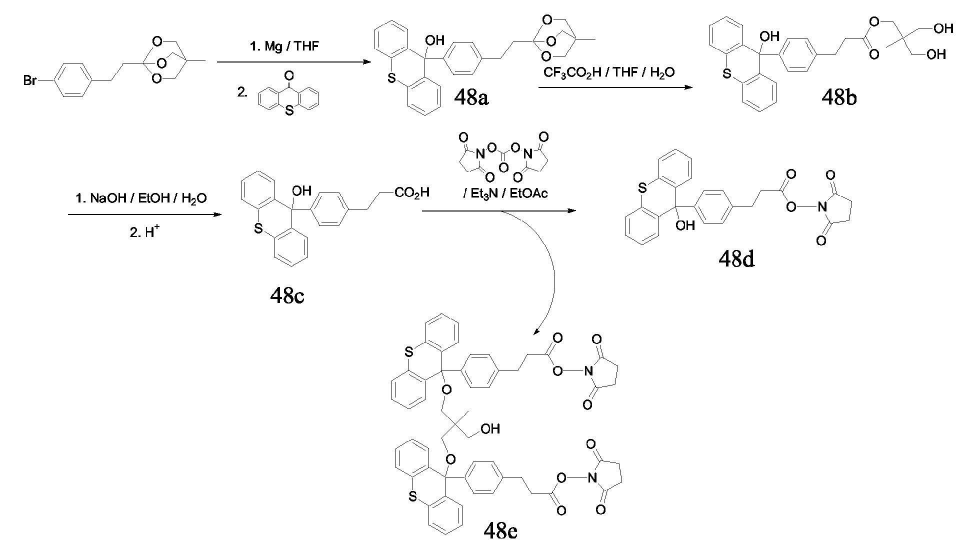

**3-[4-(9-Hydroxythioxanthen-9-yl)phenyl] propionic acid, *N*-oxysuccinimide ester** was prepared from alkyl orthoester and thioxanthone. NMR (DMSO-*d*₆): 8.02 (d, 2H, *J* = 7.8 Hz, ArH (thioxanthene)); 7.46-7.38 (m, 4H, ArH (thioxanthene)); 7.35-7.28 (m, 2H, ArH (thioxanthene)); 7.07 (d, 2H, *J* = 8.2 Hz, ArH (phenyl)); 6.78 (m, 2H, ArH (phenyl)); 5.75 (s, 1H, O*H*); 2.93-2.86 (m, 2H, ArC*H*₂); 2.85-2.74 (m, 6H, ArCH₂C*H*₂, COC*H*₂C*H*₂CO).

A byproduct, **2,2-bis(9-{4-[2-(N-succinimidyloxycarbonyl)ethyl]phenyl{thioxanthen-9-yloxymethyl)propanol,** was isolated and its structure was confirmed by NMR (DMSO-*d*₆): 7.39 (d, 4H, *J* = 7.8 Hz, Ar*H*); 7.30-7.23 (m, 8H, Ar*H*); 7.18-7.05 (m, 12H, Ar*H*); 4.35 (t, 1H, *J* = 4.9 Hz, O*H*); 3.33 (m, 2H, C*H*₂OH); 2.97-2.85 (m, 2H, ArC*H*₂C*H*₂, HOCH₂CC*H*₂); 2.80 (s, 8H, COC*H*₂C*H*₂CO), 0.93 (s, 3H, C*H*₃).

*Ar*^{*1*}/*Ar*^{*2*} *= Xanthen-9-yl*

### Example 49

**3-[4-(9-Hydroxy-6-methoxyxanthen-9-yl)phenyl] propionic acid, *N*-oxysuccinimide ester (49a)** was prepared from alkyl orthoester and xanthone as described above. NMR (DMSO-*d*₆): 8.02 (d, 2H, *J* = 7.8 Hz, Ar*H* (thioxanthene)); 7.46-7.38 (m, 4H, Ar*H* (thioxanthene)); 7.35-7.28 (m, 2H, Ar*H* (thioxanthene)); 7.21 (d, 2H, *J* = 8.2 Hz, Ar*H* (phenyl)); 6.92 (m, 2H, Ar*H* (phenyl)); 5.75 (s, 1H, *OH);* 3.41 (s, 3H, OC*H*₃), 2.93-2.86 (m, 2H, ArC*H*₂); 2.85-2.74 (m, 6H, ArCH₂C*H*₂, COC*H*₂C*H*₂CO).

*Ar*^{*1*}/*Ar*^{*2*} *= Thiophen-2-yl*

### Example 50

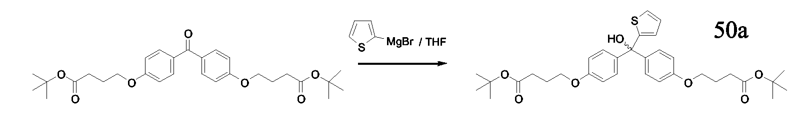

**4,4'-Bis[3-(*tert*-butyloxycarbonyl)propoxy]benzophenone.** Sodium (1.15 g, 50 mmol) was dissolved in MeOH and the resulting solution of MeONa was added to a solution of 4,4'-dihydroxybenzophenone (4.24 g, 20 mmol) in methanol (50 mL). The mixture was evaporated to dryness, the residue was dissolved in HMPA (30 mL) and *tert*-butyl 4-chlorobutyrate (8.04 g, 45 mmol) was added in one portion. The mixture was heated at 100°C for 5 h, then cooled, diluted with water (300 mL) and extracted with EtOAc (2x200 mL). The combined organic layers were washed with water (5x100 mL), dried over Na₂SO₄, and evaporated. The residue was chromatographed on silica gel in 2→5% EtOAc in toluene to give the desired compound as white crystalline solid, mp 92°C. Yield 5.49 g (55%). NMR (DMSO-d₆): 7.69 (d, 4H, J= 8.8 Hz, Ar*H*); 7.05 (d, 2H, J= 8.8 Hz, Ar*H*); 4.08 (t, 4H, J= 6.4 Hz, OC*H*₂); 2.38 (t, 4H, J= 7.3 Hz, COC*H*₂); 1.97 (m, 4H, CH₂C*H*₂CH₂); 1.41 (s, 18H, C*H*₃).

**Thiophen-2-yl-bis{4-[3-(*tert*-butyloxycarbonyl)propoxy]phenyl{methanol.** To a stirred solution of 4,4'-bis[3-(*tert*-butyloxycarboxy)propoxy]benzophenone (2.49 g; 5.0 mmol) in dry THF (50 mL) 1.0 M 2-thienylmagnesium bromide (7.5 mL, 7.5 mmol) was added in one portion under nitrogene, and the mixture was kept at ambient temperature overnight (monitoring by TLC in EtOAc-toluene 1:9). After addition of water (10 mL) the reaction mixture was evaporated, dissolved in EtOAc (200 mL) and washed with 5% NaHCO₃ (3×100 mL). The solution was dried over Na₂SO₄, evaporated, and chromatographed on silica gel in toluene with 1.0% of Et₃N to give the desired compound as a violet oil. Yield 2.86 g (98%). NMR (DMSO-*d*₆): 7.40 (m, 1H, H-5 (thiophene)); 7.15 (d, 4H, J= 8.9 Hz, ArH (phenyl)); 6.93 (m, 1H, H-4 (thiophene)); 6.83 (d, 4H, J = 8.9 Hz, ArH (phenyl)); 6.61 (m, 1H, H-3 (thiophene)); 6.52 (s, 1H, O*H*); 3.94 (t, 4H, *J* = 6.3 Hz, OC*H*₂); 2.35 (t, 4H, *J* = 7.3 Hz, COC*H*₂); 1.91 (m, 4H, CH₂C*H*₂CH₂), 1.39 (s, 18H, CC*H*₃).

### Example 51

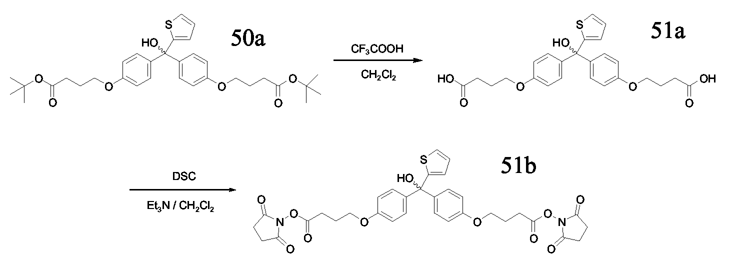

**2-Thienyl-4',4"-bis[3-(*N*-succinimidylcarbonyl)propoxy]triphenylmethanol.** To a stirred solution of 2-thienyl-4',4"-bis[3-(*tert*-butyloxycarbonyl)propoxy]triphenylmethanol (2.50 g; 4.29 mmol) in dry DCM (5 mL) trifluoroacetic acid (5 mL) was added in one portion and the mixture was stirred at ambient temperature for 3 h, then evaporated, and co-evaporated with DCM (4x50 mL) to give free acid. Product were dissolved in DCM (50 mL), and triethylamine (1.2 mL, 8.6 mmol) and *N,N*-disuccinimidyl carbonate (1.65 g, 6.4 mmol) were added and the mixture was stirred overnight, then evaporated, dissolved in EtOAc (150 mL), washed with 5% NaHCO₃ (100 mL) and water (100 mL), dried over Na₂SO₄, evaporated, and the residue was purified by column chromatography (10→50% EtOAc in toluene). Yield 2.05 g (72%), pink amorphous solid. NMR (DMSO-*d*₆): 7.41 (m, 1H, H-5 (thiophene)); 7.16 (d, 4H, J = 8.8 Hz, Ar*H*); 6.93 (dd, 1H, *J*_{4,5} = 4.8 Hz, *J*_{3,4} = 3.7 Hz, H-4 (thiophene)); 6.87 (d, 4H, J= 8.8 Hz, Ar*H*); 6.63 (m, 1H, H-3 (thiophene)); 6.54 (s, 1H, O*H*); 4.03 (t, 4H, J= 6.2 Hz, OC*H*₂); 2.87-2.78 (m, 12H, COC*H*₂, COC*H*₂C*H*₂CO); 2.07 (m, 4H, CH₂C*H*₂CH₂).

*Ar1*/*Ar2 = 2-Naphthyl*

### Example 52

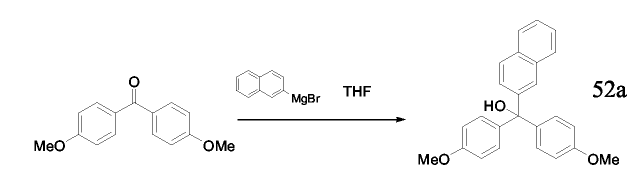

**Bis-4-methoxyphenyl-2-naphthylmethanol.** To a solution of 4,4'-dimethoxylbenzophenone (2.420 g, 10.0 mmol) in THF (35 mL) 0.5 M solution of 2-naphthylmagnesium bromide in THF (22.0 mL, 11 mmol) was added in one portion. The mixture was kept at ambient temperature overnight, then quenched with water (1 mL), evaporated, the residue was partitioned between with saturated NaHCO₃ solution (150 mL) and EtOAc (150 mL). The organic layer was washed with water (2×100 mL) and dried over Na₂SO₄. The residue was chromatographed on silica gel in gradient of EtOAc in toluene (0 to 10%) with addition of 1% of Et₃N. Yield 2.95 g (80%), a white solid. NMR (DMSO-*d*₆): 7.86 (m, 1H, H-5 or H-8 (naphthalene)); 7.81 (d, 1H, *J* = 8.7 Hz, H-4 (naphthalene)); 7.79 (m, 1H, H-8 or H-5 (naphthalene)); 7.64 (br.s, 1H, H-1 (naphthalene)); 7.47 (m, 2H, H-6,7 (naphthalene)); 7.41 (dd, 1H, *J* = 8.7 Hz, ⁴*J* = 1.9 Hz, H-3 (naphthalene)); 7.14 (d, 4H, *J* = 8.8 Hz, Ar*H* (C₆H₄)); 6.86 (d, 4H, *J* = 8.8 Hz, Ar*H* (C₆H₄)); 6.34 (s, 1H, O*H*); 3.73 (s, 6H, C*H*₃).

### Substituent Groups A

*A* = *thiomethyl*

### Example 53

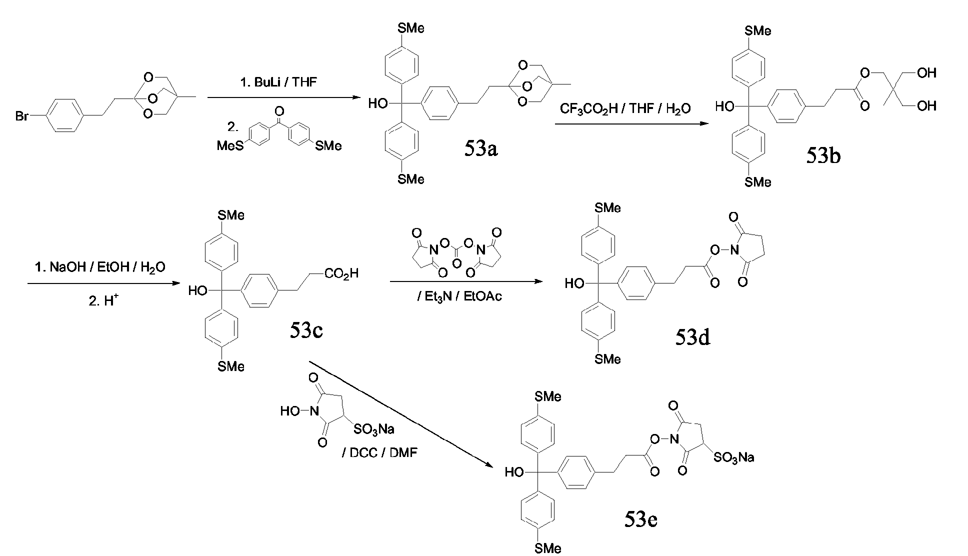

**1-(2-{4-[Hydroxy-bis(4-methylthiophenyl)methyl]phenyl}ethyl)-4-methyl-2,6,7-trioxabicyclo[2.2.2]octane.** To a stirred and coolled to -70°C mixture of THF (20 mL) and 0.9M BuLi in hexane (7.0 mL, 6.3 mmol) the solution of 1-[2-(4-bromophenoxy)ethyl]-4-methyl-2,6,7-trioxabicyclo[2.2.2]octane (1.566 g, 5.0 mmol) in THF (50 mL) was added dropwise within 30 min. The mixture was then stirred for 30 min, and the solution of 4,4'-dimethylthiobenzophenone (1.372 g, 5.0 mmol) in THF (50 mL) was added dropwise within 30 min. The mixture was allowed to warm to room temperature and stirred overnight at ambient temperature. The solution was evaporated to dryness, the residue was dissolved in EtOAc (200 mL) with few drops of triethylamine, washed with 5% NaHCO₃ (2×200 mL), water (2×200 mL), dried over Na₂SO₄, and evaporated. The residue was dissolved in toluene containing 1% Et₃N (10 mL) and chromatographed on a silica gel column. The column was eluted with stepwise gradient 2→2.5→3.5→4.0→4.5→5.0% Me₂CO in toluene containing 0.5% Et₃N. The desired product was triturated in petroleum ether filtered off and dried *in vacuo.* Yield 1.249 g (49.1%), amorphous solid. *R*_{f} 0.53 (toluene-EtOAc, 4:1). NMR (DMSO-*d*₆): 7.20-7.05 (m, 12H, Ar*H*); 6.30 (s, 1H, O*H*); 3.84 (s, 6H, CH₂O); 2.63 (m, 2H, ArC*H*₂); 2.44 (s, 6H, SC*H*₃); 1.81 (m, 2H, ArCH₂C*H*₂); 0.76 (s, 3H, CC*H*₃).

**3-{4-[Hydroxy-bis(4-methylthiophenyl)methyl]phenyl}propionic acid, *N*-oxysuccinimide ester.** 1-(2-{4-[Hydroxy-bis(4-methylthiophenyl)methyl]phenyl}ethyl)-4-methyl-2,6,7-trioxabicyclo-[2.2.2]octane (1.08 g, 2.0 mmol) was dissolved in the THF-water mixture (19:1, 10mL), and trifluoroacetic acid (0.08 mL, 1 mmol) was added. After stirring for 10 min the mixture was evaporated, the residue was dissolved with EtOAc (200 mL), washed with 5% NaHCO₃ (100 mL) and water (2x100 mL), dried over Na₂SO₄, evaporated, and chromatographed on silica gel column in 10 → 40% Me₂CO in toluene to give 3-{4-[hydroxy-bis(4-methylthiophenyl)methyl]phenyl}propionic acid 2-hydroxymethyl-2-methyl-3-hydroxypropyl ester as a colorless oil, yield 895 mg (85%). NMR (DMSO-*d*_{*6*}): 7.20-7.07 (m, 12H, Ar*H*); 6.32 (s, 1H, trityl O*H*); 4.43 (m, 2H, CH₂O*H*); 3.87 (s, 2H, C*H*₂OCO); 3.24 (m, 4H, C*H*₂OH); 2.84 (m, 2H, ArC*H*₂); 2.62 (m, 2H, C*H*₂CO); 2.44 (s, 6H, SC*H*₃); 0.74 (s, 3H, CC*H*₃). The ester was dissolved in 10% NaOH in ethanol-water (8:2, 10mL), stirred overnight, then evaporated, coevaporated with water (2x50 mL), dissolved in water (100 mL), washed with ether (2x100 mL), and then acidified with solid citric acid to pH 4. The reaction mixture was extracted with EtOAc (2x100 mL), the combined organic layers were washed with water (50 mL), dried over Na₂SO₄ and evaporated to dryness. The resulting 3-{4-[hydroxy-bis(4-methylthiophenyl)methyl]phenyl}propionic acid was obtained as a colorless oil, 637 mg (88%). NMR (DMSO-*d*₆): 12.09 (s, 1H, CO₂*H*); 7.20-7.08 (m, 12H, Ar*H*); 6.32 (s, 1H, trityl O*H*); 2.81 (m, 2H, ArC*H*₂); 2.52 (m, 2H, C*H*₂CO); 2.44 (s, 6H, SC*H*₃). This was dissolved in anhydrous DCM (20 mL) and triethylamine (0.35 mL, 2.5 mmol) and *N*,*N*'-disuccinimidyl carbonate (512 g, 2.0 mmol) were added and the mixture was stirred until the reaction is complete (monitoring by TLC in toluene-EtOAc, 1:1), then evaporated, dissolved in EtOAc (200 mL), washed with 5% NaHCO₃ (100 mL) and water (100 mL), dried over Na₂SO₄, evaporated, and the residue was purified by column chromatography (5 to 10% EtOAc in toluene). Yield 542 mg (69%), white crystalline solid, mp 137°C. *R*_{f} 0.36 (toluene-EtOAc, 4:1). NMR (DMSO-*d*₆): 7.24-7.10 (m, 12H, Ar*H*); 6.34 (s, 1H, O*H*); 3.02-2.91 (m, 4H, ArC*H*₂C*H*₂); 2.81 (s, 4H, COC*H*₂C*H*₂CO); 2.45 (s, 6H, SC*H*₃).

**3-{4-[Hydroxy-bis(4-methylthiophenyl)methyl]phenyl}propionic acid, *N*-oxysulfosuccinimide ester, sodium salt.** The 3-{4-[hydroxy-bis(4-methylthiophenyl)-methyl]phenyl}propionic acid (425 mg, 1.0 mmol), prepared as above, was dissolved in dry DMF (2.5 mL), then *N*-oxysulfosuccinimide sodium salt (250 mg, 1.17 mmol) and DCC (375 mg, 1.8 mmol) were added and the mixture was left under stirring overnight, then cooled to +4°C and stirred for 2 h, and the precipitate formed was filtered off. The solution was diluted with EtOAc (10 mL), cooled to +4°C overnight, filtered, and diluted with dry Et₂O (200 mL). The mixture was kept at ambient temperature for 2-3 h and the desired compound was collected by filtration, washed with ether and dried *in vacuo.* Yield 412 mg (66%), white solid, *R*_{f} 0.58 (*n*-propanol-EtOAc-water, 8:1:1). NMR (DMSO-*d*₆): 7.24-7.08 (m, 12H, Ar*H*); 6.35 (s, 1H, O*H*); 3.94 (br., 1H, COC*H*S); 3.15 (br., 1H, COCH*H*CHS); 3.02-2.83 (m, 4H, ArC*H*₂C*H*₂, COC*H*HCHS); 2.45 (s, 6H, SC*H*₃).

*A = dialkylamino*

### Example 54

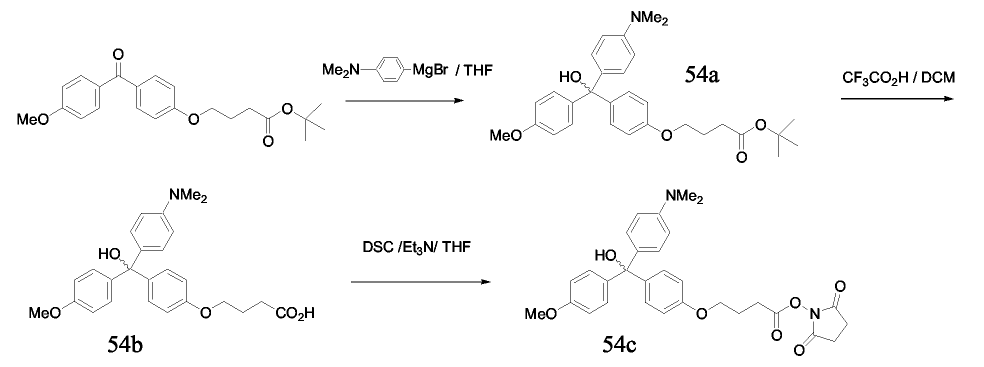

**4-{4-[Hydroxy-(4-dimethylaminophenyl)-(4-methoxyphenyl)-methyl]phenoxy}butano-ic** acid, ***tert*-butyl ester.** To a stirred solution of 4-[3-(*tert*-butyloxycarbonyl)propoxy]-4'-methoxybenzophenone (1.48 g; 4.0 mmol) in dry THF (30 mL) 0.5 M 4-dimethylaminophenylmagnesium bromide (10.0 mL, 5.0 mmol) was added in one portion under Ar, and the mixture was kept at ambient temperature overnight (monitoring by TLC in EtOAc-toluene 1:9). The reaction was diluted with water (200 mL) and 5% NaHCO₃ (100 mL), and extracted with EtOAc (2×100 mL). The organic phase was dried over Na₂SO₄, evaporated, and chromatographed on silica gel in toluene with 1.0% of Et₃N to give the desired compound as a colourless oil. Yield 1.514 g (77%). NMR (DMSO-*d*₆): 7.07 (m, 4H, Ar*H*); 6.95 (d, 2H, *J*= 8.8 Hz, Ar*H*); 6.81 (m, 4H, Ar*H*); 6.62 (d, 2H, *J*= 8.8 Hz, Ar*H*); 5.91 (s, 1H, O*H);* 3.94 (t, 2H, *J*= 6.3 Hz, OC*H*₂); 3.72 (s, 3H, OC*H*₃); 2.85 (s, 6H, NC*H*₃); 2.35 (t, 2H, *J* = 7.3 Hz, COC*H*₂); 1.91 (m, 2H, CH₂C*H*₂CH₂), 1.40 (s, 9H, CC*H*₃).

**4-{4-[Hydroxy-(4-dimethylaminophenyl)-(4-methoxyphenyl)-methyl]phenoxy}butano-ic acid, *N*-oxysuccinimide ester.** To a stirred solution of *tert*-butyl 4-{4-[hydroxy-(4-dimethylaminophenyl)-(4-methoxyphenyl)-methyl]phenoxy}butanoate (983 mg; 2.0 mmol) in dry DCM (3 mL) trifluoroacetic acid (3 mL) was added in one portion and the mixture was stirred at ambient temperature for 1 h, then evaporated, and co-evaporated with DCM (4×50 mL) to give the desired acid. This was dissolved in DCM (40 mL), water (2 mL) and Et₃N (5 mL), washed with water (2×50 mL), dried over Na₂SO₄, and evaporated to dryness. The residue was dissolved in DCM (15 mL), triethylamine (0.7 mL, 5.0 mmol) and *N*,*N*'-disuccinimidyl carbonate (0.77 g, 3 mmol) were added and the mixture was stirred until the reaction is complete (monitoring by TLC in toluene-EtOAc, 2:1), then evaporated, dissolved in EtOAc (100 mL), washed with 5% NaHCO₃ (100 mL) and water (100 mL), dried with Na₂SO₄, evaporated, and the residue was purified by column chromatography (10→15% EtOAc in toluene). Yield 660 mg (62%), violet amorphous solid. NMR (DMSO-*d*₆): 7.07 (m, 4H, Ar*H*); 6.95 (d, 2H, J= 8.7 Hz, Ar*H*); 6.82 (m, 4H, Ar*H*); 6.62 (d, 2H, J= 8.7 Hz, Ar*H*); 5.92 (s, 1H, O*H*); 4.02 (t, 2H, *J* = 6.3 Hz, OC*H*₂); 3.72 (s, 3H, OC*H*₃); 2.88-2.79 (m, 12H, NC*H*₃, CH₂CH₂CH₂CO, COC*H*₂C*H*₂CO); 2.06 (m, 2H, CH₂C*H*₂CH₂).

*A = methoxyl*

### Example 55

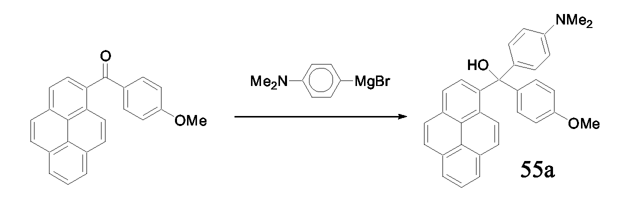

**(4-Dimethylaminophenyl)-(4-methoxyphenyl)-(pyren-1-yl)-methanol.** To a stirred solution of 1-(4-methoxybenzoyl)pyrene (673 mg; 2.0 mmol) in dry THF (20 mL) 0.5 M 4-dimethylaminophenylmagnesium bromide (6.0 mL, 3.0 mmol) was added in one portion under Ar, and the mixture was kept at ambient temperature overnight (monitoring by TLC in EtOAc-toluene 1:9). The reaction was diluted with water (100 mL) and 5% NaHCO₃ (50 mL), and extracted with EtOAc (2×50 mL). The organic phase was dried over Na₂SO₄, evaporated, and chromatographed on silica gel in 2% EtOAc in toluene with 1.0% of Et₃N to give the desired compound as a dark green oil. Yield 595 mg (65%). NMR (DMSO-*d*₆): 8.65 (d, 1H, J= 9.5 Hz, *H*-10 (pyrene)); 8.24 (d, 1H, J= 7.8 Hz, *H*-2 (pyrene)); 8.19-8.06 (m, 4H, *H*-4,5,6,8 (pyrene)); 8.02 (m, 1H, *H*-7 (pyrene)); 7.89 (d, 1H, *J*= 9.5 Hz, *H*-9 (pyrene)); 7.39 (d, 1H, J= 7.8 Hz, *H*-3 (pyrene)); 7.15 (d, 2H, J= 8.7 Hz, ArH (phenyl)); 7.04 (d, 2H, *J* = 8.7 Hz, ArH (phenyl)); 6.86 (d, 2H, *J* = 8.9 Hz, ArH (phenyl)); 6.72 (s, 1H, *OH*); 6.66 (d, 2H, *J* = 8.9 Hz, ArH (phenyl)); 3.73 (s, 3H, OC*H*₃); 2.87 (s, 6H, NC*H*₃).

*A = sulfoxide*

### Example 56

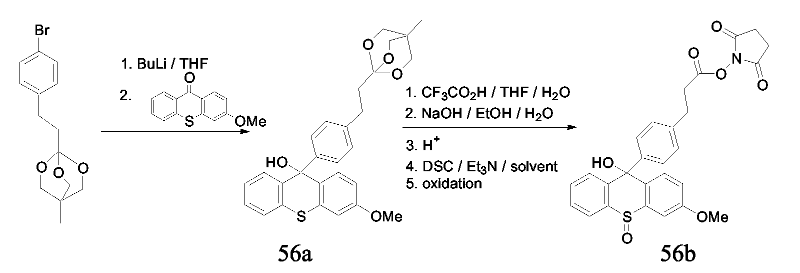

### 1-{2-[4-(9-Hydroxy-3-methoxythioxanthen-9-yl)phenyl]ethyl}-4-methyl-2,6,7-trioxabicyclo [2.2.2] octane.

To a stirred and cooled to -80°C solution of 1-[2-(4-bromophenoxy)ethyl]-4-methyl-2,6,7-trioxabicyclo[2.2.2]octane (845 mg, 2.70 mmol) in THF (30 mL) 1.4M *tert-*BuLi in pentane (3.86 mL, 5.40 mmol) was added dropwise. The temperature was raised to -40°C, then lowered again to -80°C, and the solution of 3-methoxythioxanthone (653 g, 2.70 mmol) in THF (40 mL) was added dropwise. The mixture was allowed to warm to room temperature and stirred overnight at ambient temperature. The solution was evaporated to dryness, the residue was dissolved in EtOAc (150 mL) with few drops of triethylamine, washed with 5% NaHCO₃ (2×100 mL), water (2×100 mL), dried over Na₂SO₄, and evaporated. The residue was dissolved in toluene containing 1% Et₃N (10 mL) and chromatographed on a silica gel. The column was eluted with 10% acetone in toluene containing 0.5% Et₃N. Yield 1.14 g (94%), white amorphous solid. NMR (DMSO-*d*₆): 7.98 (d, 1H, *J* = 7.8 Hz, *H*-8 (thioxanthene)); 7.86 (d, 1H, *J* = 8.7 Hz, *H*-1 (thioxanthene)); 7.42-7.36 (m, 1H, *H*-5,7 (thioxanthene)); 7.29 (m, 1H, *H*-6 (thioxanthene)); 7.00-6.94 (m, 2H, ⁴*J* = 2.6 Hz, *H*-2,4 (thioxanthene)); 6.93 (d, 2H, *J =* 8.0 Hz, Ar*H* (C₆H₄)); 6.77 (d, 2H, *J =* 8.0 Hz, ArH (C₆H₄)); 6.63 (s, 1H, O*H);* 3.81 (s, 6H, OC*H*₂); 3.78 (s, 3H, OC*H*₃); 2.52 (m, 2H, ArC*H*₂); 1.71 (m, 2H, ArCH₂C*H*₂); 0.74 (s, 3H, CC*H*₃).

### 3-[4-(9-Hydroxy-10-oxo-3-methoxythioxanthen-9-yl)phenyl] propionic acid, N-oxysuccinimide ester.

1-{2-[4-(9-Hydroxy-3-methoxythioxanthen-9-yl)phenyl]ethyl}-4-methyl-2,6,7-trioxabicyclo[2.2.2]octane (1.14 g, 2.54 mmol) was dissolved in the THF-water mixture (4:1, 60 mL), and trifluoroacetic acid (290 µL, 3.82 mmol) was added. After stirring for 1 h the mixture was half evaporated, the residue was dissolved with EtOAc (100 mL), washed with 5% NaHCO₃ (100 mL) and water (2×100 mL), dried over Na₂SO₄ and evaporated. The residue was dissolved in EtOH (120 mL), and NaOH (1.22 g, 30.5 mmol) was added. The solution was stirred overnight at ambient temperature, filtered and evaporated. The residue was dissolved in water (100 mL), washed with EtOAc (50 mL), acidified with solid citric acid to pH 3, and extracted with EtOAc (3×50 mL). Combined organic layers were dried over Na₂SO₄ and evaporated to volume 30 mL. Triethylamine (1.76 mL, 13.7 mmol) and *N,N'*-disuccinimidyl carbonate (1.95 g, 7.60 mmol) were added and the mixture was stirred until the reaction is complete (monitoring by TLC), then dissolved in EtOAc (50 mL), washed with 5% NaHCO₃ (2×50 mL) and water (50 mL), dried over Na₂SO₄, evaporated, and the residue was dissolved in ether/H₂O₂ (10%) and stirred for an hour. Hydrogen peroxide was quenched, the reaction mixture was evaporated and purified by column chromatography (15% EtOAc in toluene). Yield 528 mg (45%), white amorphous solid. NMR (DMSO-*d*₆): 7.99 (d, 1H, *J* = 7.8 Hz, ArH (thioxanthene)); 7.87 (d, 1H, *J* = 8.7 Hz, *H*-1 (thioxanthene)); 7.48 (m, 2H, ArH (thioxanthene)); 7.29 (m, 1H, ArH (thioxanthene)); 7.07 (d, 2H, *J* = 8.2 Hz, Ar*H* (C₆H₄)); 7.00 (d, 1H, *J*= 2.5 Hz, *H*-4 (thioxanthene)); 6.96 (dd, 1H, *J*= 8.7 Hz, ⁴*J*= 2.5 Hz, *H*-2 (thioxanthene)); 6.81 (d, 2H, *J* = 8.2 Hz, Ar*H* (C₆H₄)); 6.67 (s, 1H, O*H*); 3.79 (s, 3H, OC*H*₃); 2.90 (m, 2H, ArC*H*₂); 2.81 (m, 6H, COC*H*₂).

*A* = *fluoro*

### Example 57

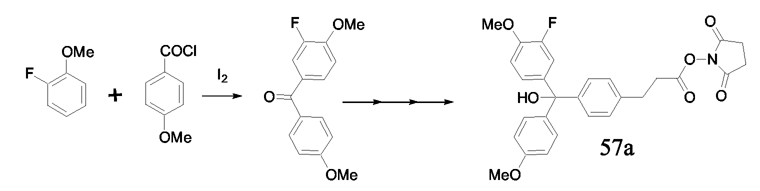

**3-Fluoro-4,4'-dimethoxybenzophenone.** A mixture of 2-fluoroanisole (6.30 g, 50 mmol), 4-methobybenzoyl chloride (9.4 g, 55 mmol) and iodine (2.0 g) was refluxed for 10 h, then cooled, and diluted with CHCl₃ (300 mL). The solution was washed with 5% NaHCO₃ (2x150 mL), 5% NaHSO₃ (150 mL), and water (150 mL), dried with CaCl₂, and evaporated. The residue was purified by column chromatography in CHCl₃ and triturated in petroleum ether togive pure benzophenone as white solid (6.25g, 48%). *R*_{f} 0.06 (toluene), *R*_{f} 0.30 (toluene-EtOAc, 9:1), mp 135°C (petroleum ether). NMR (DMSO-*d*₆): 7.73 (d, 2H, *J* = 8.8 Hz, Ar*H*); 7.57-7.52 (m, 2H, Ar*H*); 7.31 (m, 1H, Ar*H*); 7.08 (d, 2H, J= 8.8 Hz, Ar*H*); 3.94 (s, 3H), 3.86 (s, 3H) (C*H*₃).

The title trityl was then synthesized as described above (for example, see L1 in Section 1) with a total yield of 12%.

*A = methyl*

### Example 58

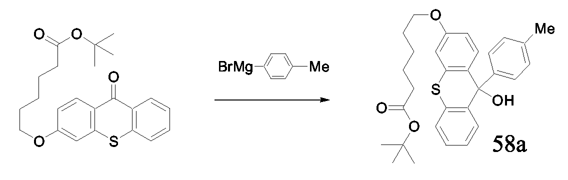

**9-Hydroxy-9-(4-methylphenyl)-6-alkoxy-thioxantene.** Corresponding ketone (1.06 g) was dissolved in dry THF (100 mL) and 1 M solution in THF of 4-methylphenyhnagnesium bromide (6 mL, 6 mmol) was added under N₂ within 5 min. The workup was as above. The desired compound isolated by chromatography in toluene (+0.5 % Et₃N). Fractions containing product were evaporated, and the residue was filtered, washed with hexane, and dried *in vacuo.* Yield 1.38 g (91 %), mp 168-171°C. NMR (DMSO-*d*₆): 8.05-8.00 (m, 2H, *H*-1,8 (thioxanthene)); 7.46-7.38 (m, 4H, *H*-2,4,5,7 (thioxanthene)); 7.35-7.28 (m, 2H, *H*-3,6 (thioxanthene)); 6.93 (d, 2H, *J* = 8.2 Hz, ArH (phenyl)); 6.74 (d, 2H, *J* = 8.2 Hz, ArH (phenyl)); 6.70 (s, 1H, O*H*); 2.16 (s, 3H, C*H*₃).

*A =alkoxy*

### Example 59

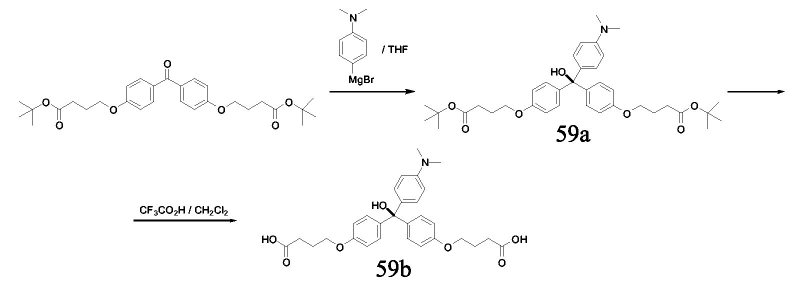

**4,4'-Bis[3-(*tert-*butyloxycarbonyl)propoxy]benzophenone.** Sodium (1.15 g, 50 mmol) was dissolved in MeOH and the resulting solution of MeONa was added to a solution of 4,4'-dihydroxybenzophenone (4.24 g, 20 mmol) in methanol (50 mL). The mixture was evaporated to dryness, the residue was dissolved in HMPA (30 mL) and *tert*-butyl 4-chlorobutyrate (8.04 g, 45 mmol) was added in one portion. The mixture was heated at 100°C for 5 h, then cooled, diluted with water (300 mL) and extracted with EtOAc (2x200 mL). The combined organic layers were washed with water (5x100 mL), dried over Na₂SO₄, and evaporated. The residue was chromatographed on silica gel in 2→5% EtOAc in toluene to give the desired compound as white crystalline solid, mp 92°C. Yield 5.49 g (55%). NMR (DMSO-*d*₆): 7.69 (d, 4H, *J*= 8.8 Hz, Ar*H*); 7.05 (d, 2H, *J*= 8.8 Hz, Ar*H*); 4.08 (t, 4H, J= 6.4 Hz, OC*H*₂); 2.38 (t, 4H, J= 7.3 Hz, COC*H*₂); 1.97 (m, 4H, CH₂C*H*₂CH₂); 1.41 (s, 18H, C*H*₃).

**4-Dimethylaminophenyl-bis{4-[3-(*tert*-butyloxycarbonyl)propoxy]phenyl}methanol.** To a stirred solution of 4,4'-bis[3-(*tert*-butyloxycarbonyl)propoxy]benzophenone (2.49 g; 5.0 mmol) in dry THF (50 mL) 0.5 M 4-(*N,N*-dimethylamino)phenylmagnesium bromide (15 mL, 7.5 mmol) was added in one portion under nitrogen, and the mixture was kept at ambient temperature overnight (monitoring by TLC in EtOAc-toluene 1:4). After addition of water (10 mL) the reaction mixture was evaporated, dissolved in EtOAc (200 mL) and washed with 5% NaHCO₃ (3×100 mL). The solution was dried over Na₂SO₄, evaporated, and chromatographed on silica gel in toluene with 5% of EtOAc and 1% of Et₃N to give the desired compound as pale violet oil. Yield 2.22 g (71%). NMR (DMSO-*d*₆): 7.06 (d, 4H, J= 8.7 Hz, Ar*H*); 6.94 (d, 2H, J= 8.8 Hz, Ar*H*); 6.80 (d, 4H, *J*= 8.7 Hz, Ar*H*); 6.61 (d, 2H, J= 8.8 Hz, Ar*H*); 5.91 (br.s, 1H, O*H*); 3.93 (t, 2H, *J*= 6.2 Hz, OC*H*₂); 2.85 (s, 6H, NC*H*₃); 2.34 (t, 4H, *J*= 7.2 Hz, COC*H*₂); 1.91 (m, 4H, CH₂C*H*₂CH₂), 1.40 (s, 18H, CC*H*₃).

**4-(*N*,*N*-Dimethylaminophenyl)-4',4"-bis[3-carboxypropoxy]triphenylmethanol.** To a stirred solution of 4-(*N*,*N*-dimethylaminophenyl)-4',4"-bis[3-(*tert*-butyloxycarbonyl)propoxy]-triphenylmethanol (1.55 g, 2.5 mmol) in dry DCM (5 mL) trifluoroacetic acid (5 mL) was added in one portion and the mixture was stirred at ambient temperature overnight (monitoring by TLC in EtOAc-toluene 1:4). The solution was evaporated to dryness, and co-evaporated with DCM (4×30 mL) to give desired acid as dark violet oil. Yield 1.23 g (97 %). NMR (DMSO-*d*₆): 7.49 (d, 2H, *J*= 9.6 Hz, ArH); 7.33 (d, 4H, *J*= 8.9 Hz, ArH); 7.27 (d, 2H, *J*= 9.6 Hz, ArH); 7.21 (d, 4H, *J*= 8.9 Hz, Ar*H*); 4.18 (t, 4H, *J*= 6.4 Hz, OC*H*₂); 3.46 (s, 6H, NC*H*₃); 2.43 (t, 4H, *J*= 7.2 Hz, COC*H*₂); 2.01 (m, 4H, CH₂C*H*₂CH₂).

*A* = ^{*18*}*O*^{*13*}*CH*^{*13*}_{*2*}*CH*_{*2*}

### Example 60

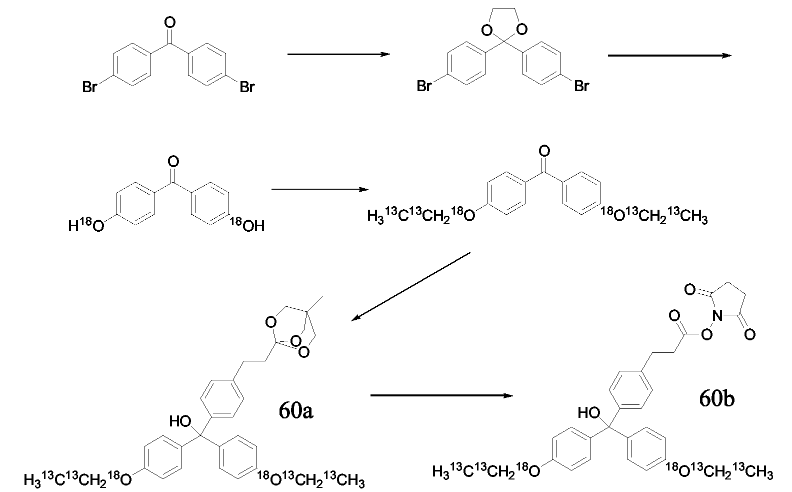

**2,2-Di(4-bromophenyl)-1,3-dioxolane.** 4,4'-Dibromobenzophenone (3.40 g, 10.0 mmol), ethylene glycol (0.84 mL, 15 mmol) and TsOH (50 mg) in toluene (200 mL) were refluxed with Dean-Stark apparatus for 6 h. The mixture was cooled, the solid K₂CO₃ (200 mg) was added and the solution was filtered and evaporated. The residue was purified by short column chromatography in toluene to give dioxolane as a colourless oil (3.52 g, 92%). NMR (DMSO-*d*_{*6*}): 7.55 (d, 4H, J= 8.8 Hz, Ar*H*); 7.26 (d, 4H, J= 8.8 Hz, Ar*H*); 3.92 (m, 4H, OC*H*₂).

**4,4'-Di[**^{**18**}**O]hydroxy-benzophenone.** To a stirred and cooled to -75°C solution of 2,2-di(4-bromophenyl)-1,3-dioxolane (1.92 g, 5.0 mmol) in in dry THF (100 mL) 1.4 M *tert*-BuLi in pentane (12.5 mL, 200 mmol) was added dropwise under argon. The temperature was raised to -40°C for 1 h and [¹⁸O]oxygen (115 mL, 5.0 mmol) was introduced slowly by needle through a rubber septum and allowed to consume. After 3 h the cooling was removed and the solution was stirred overnight. The mixture was quenched by water (3 mL), evaporated, diluted with 5% HCl and refluxed for 1 h, then evaporated and the residue was purified by column chromatography (toluene-methanol 7:1) to give dihydroxybenzophenone as a white solid. Yield 120 mg (11%). NMR (DMSO-*d*₆): 10.33 (br.s, 2H, O*H*), 7.70 (d, 4H, *J*= 8.8 Hz, Ar*H*); 7.02 (d, 4H, *J*= 8.8 Hz, Ar*H*).

**4,4'-di[**^{**18**}**O][**^{**13**}**C]ethoxy-benzophenone.** To a solution of 4,4'-di[¹⁸O]hydroxy-benzophenone (109 mg, 0.5 mmol) in in dry acetone (5 mL) dry K₂CO₃ (0.7 g, 5.0 mol) and [¹³C]ethyl bromide (166 mg, 1.5 mmol) were added, and the mixture was stirred for 48 h at ambient temperature, then filtered, and the solid was washed with acetone. The combined filtrate was evaporated, and the residue was dissolved in CHCl₃ (10 mL), filtered, and evaporated. The residue was chromatographed on silica gel in 0→4% EtOAc in toluene to give desired product (120 mg, 86%). NMR (DMSO-*d*₆): 7.78 (d, 4H, *J* = 8.7 Hz, Ar*H*); 6.95 (d, 4H, *J* = 8.7 Hz, Ar*H*); 4.21 (q, 4H, *J* = 6.9 Hz, OC*H*₂); 1.59 (t, 6H, *J* = 6.9 Hz, C*H*₃).

**1-[2-(4-{Hydroxy-bis[4-([**^{**18**}**O][**^{**13**}**C]ethoxy)phenyl]methyl}phenyl)ethyl]-4-methyl-2,6,7-trioxabicyclo[2.2.2]octane.** To a stirred and cooled to -75°C mixture of 1.4 M *tert*-BuLi in pentane (0.5 mL, 0.7 mmol) and THF (2 mL) the solution of 1-[2-(4-bromophenyl)ethyl]-4-methyl-2,6,7-trioxabicyclo[2.2.2]octane (109 mg, 0.35 mmol) in THF (2 mL) was added. The temperature was raised to -40°C, then lowered again to -75°C, and the solution of 4,4'-di[¹⁸O][¹³C]ethoxybenzophenone (98 mg, 0.35 mmol) in THF (3 mL) *via* a suringe. The mixture was allowed to warm to room temperature and stirred overnight at ambient temperature. The solution was poured into water (10 mL), extracted with EtOAc (10 mL). Organic layer was washed with 5% NaHCO₃ (2×10 mL), water (10 mL), dried over Na₂SO₄, and evaporated. The residue was purified by column chromatography (3% EtOAc in toluene). Yield 142 mg (79%).

**3-(4-{Hydroxy-bis[4-([**^{**18**}**O][**^{**13**}**C]ethoxy)phenyl]methyl}phenyl)propionic acid, *N*-oxysuccinimide ester.** 1-[2-(4-{Hydroxy-bis[4-([¹⁸O][¹³C]ethoxy)phenyl]methyl}phenyl)ethyl]-4-methyl-2,6,7-trioxabicyclo[2.2.2]octane. (128 mg, 0.25 mmol) was dissolved in the THF-water mixture (9:1, 0.5 mL), and trifluoroacetic acid (0.05 mL) was added. After stirring for 10 min the mixture was evaporated and diluted with 10 % NaOH in ethanol-water (8:2, 3mL). The reaction mixture was refluxed for 1 h, cooled, and evaporated, the residue was diluted with water (5 mL), washed with Et₂O (2×5 mL), filtered, acidified with 5% citric acid, and extracted with ethyl acetate (2×5 mL). The solution was dried with Na₂SO₄, evaporated, and the residue was dissolved in dry DCM (2 mL). Triethylamine (0.14 mL, 1 mmol) and *N*,*N*'-disuccinimidyl carbonate (128 mg, 0.5 mmol) were added and the mixture was stirred until the reaction is complete (monitoring by TLC in toluene-acetone, 9:1), then evaporated, dissolved in EtOAc (10 mL), washed with 5% NaHCO₃ (10 mL) and water (10 mL), dried with Na₂SO₄, evaporated, and the residue was purified by column chromatography (5 to 20% of acetone in toluene). Yield 85 mg (65%), white amorphous solid.

***X and X*** 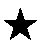 ***Groups***

*X* = *hydroxyl*

### Example 61

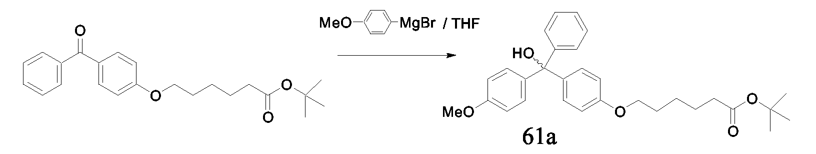

**6-{4-[Hydroxy-(4-methoxyphenyl)-phenyl-methyl]phenoxy{hexanoic acid, *tert*-butyl** ester. To a stirred solution of 4-[5-(tert-butyloxycarbonyl)pentyloxy]benzophenone (1.84 g; 5.0 mmol) in dry THF (30 mL) 1 M 4-methoxyphenylmagnesium bromide (7.0 mL, 7.0 mmol) was added in one portion under Ar, and the mixture was kept at ambient temperature overnight (monitoring by TLC in EtOAc-toluene 1:4). The reaction was diluted with water (300 mL) and 5% citric acid (20 mL), and extracted with EtOAc (2×100 mL). The organic phase was dried over Na₂SO₄, evaporated, and chromatographed on silica gel in 0→5% EtOAc in toluene with 0.5% of Et₃N to give the desired compound as a colourless oil. Yield 1.597 g (67%).

*X* = *ethyl ether*

### Example 62

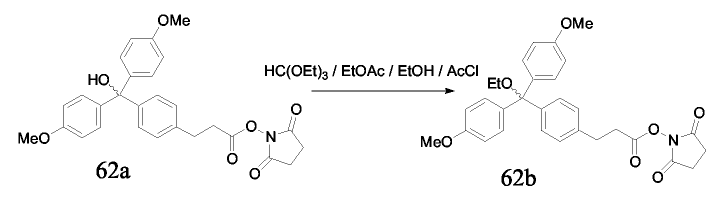

**3-{4-[Ethoxy-bis(4-methoxyphenyl)methyl]phenyl}propionic acid, *N*-oxysuccinimide ester. 3**-{4-[Hydroxy-bis(4-methoxyphenyl)methyl]phenyl}propionic acid, *N*-oxysuccinimide ester (978 mg, 2.0 mmol) was dissolved in ethyl acetate (50 mL) and absolute ethanol (5 mL). Triethyl orthoformiate (600 mg, 4 mmol) and acetyl chloride (25 µL) were added to the mixture and this was left with stirring overnight at ambient temperature. The mixture was evaporated and the residue was chromatographed on silica gel column in 30% EtOAc in toluene. Yield 724 mg (70%).

*X* = *alkyl ether*

### Example 63

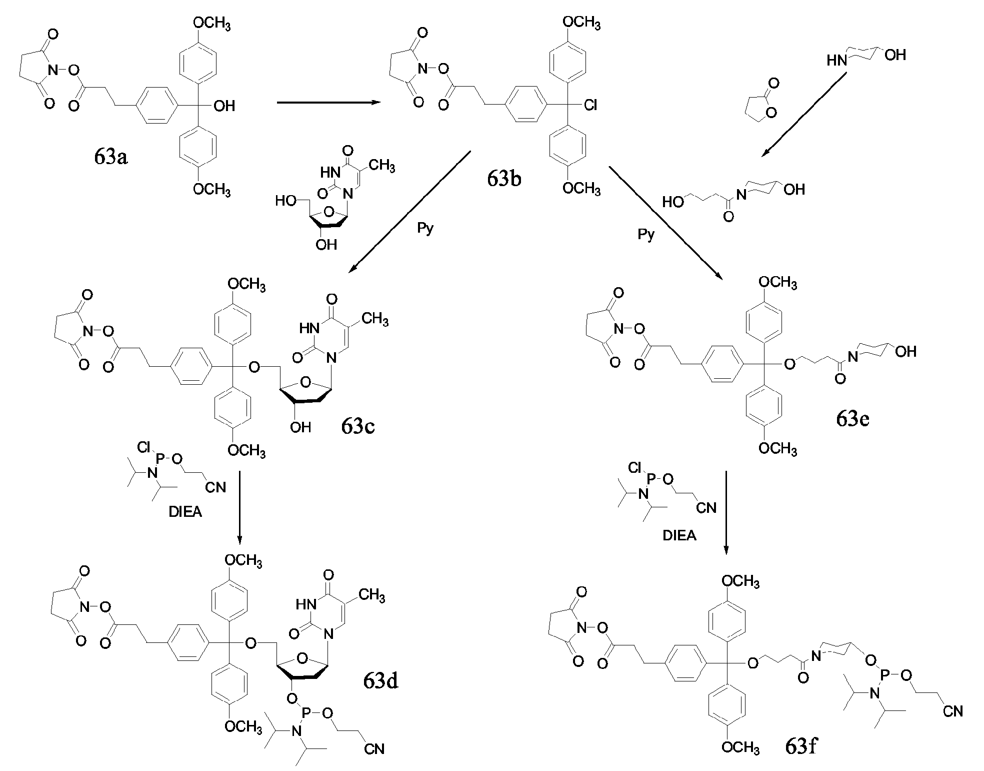

**5'-*O*-{4,4'-Dimethoxy-4"-[2-(N-succinimidyloxycarbonyl)ethyl]trityl}thymidine.** 3-{4-[Hydroxy-bis(4-methoxyphenyl)methyl]phenyl}propionic acid, *N*-oxysuccinimide ester (1.81 g, 3.7 mmol was dissolved in freshly distilled AcCl (20 mL) and the mixture was refluxed (4 h), then diluted with hexane (50 mL) and cooled to room temperature. The trityl chloride was filtered off, washed with hexane, and dried *in vacuo.* Yield 1.42 g (76%). Thymidine (727 mg, 3.0 mmol) was coevaporated with dry pyridine (2×20 mL), dissolved in pyridine (30 mL), half-evaporated, and cooled on ice bath. 3-{4-[Chloro-bis(4-methoxyphenyl)methyl]phenyl} propionic acid, *N*-oxysuccinimide ester (1.42 g, 2.8 mmol) was added in one portion with stirring, and the stirring was continued for 4 h. The mixture was warmed to room temperature, diluted with CHCl₃ (150 mL), washed with water (2×100 mL), dried over Na₂SO₄, evaporated, and the residue was purified by column chromatography (elution with 20→35% EtOAc in toluene, then 15→30% acetone in EtOAc-toluene (1:2), and, finally, with acetone-toluene (1:1)). Yield 1.35 g (68%), white amorphous solid. NMR (DMSO-*d*₆): 11.3 (s, 1H, N*H*); 7.50 (s, 1H, *H*-6); 7.32-7.20 (m, 8H, Ar*H*); 6.88 (m, 4H, Ar*H*); 6.20 (apparent t, 1H, *J*= 6.7 Hz, H-1'); 5.30 (d, 1H, J= 4.4 Hz, O*H*); 4.31 (m, 1H, *H-*3'); 3.88 (m, 1H, *H*-4'); 3.74 (s, 6H, OC*H*₃); 3.25-3.13 (m, 2H, *H*-5'); 3.03-2.89 (m, 4H, ArC*H*₂C*H*₂); 2.81 (s, 4H, COC*H*₂C*H*₂CO); 2.3-2.1 (m, 2H, *H*-2'); 1.45 (s, 3H, CC*H*₃).

**5'-*O*-{4,4'-Dimethoxy-4"-[2-(*N*-succinimidyloxycarbonyl)ethyl]trityl}-3'-*O*-(diisopropylamino-2-cyanethoxyphosphinyl)thymidine.** To a solution of 5'-*O*-{4,4'-dimethoxy-4"-[2-(*N*-succinimidyloxycarbonyl)ethyl]trityl} thymidine (250 mg, 0.35 mmol) in dry DCM (10 mL) and DIEA (85 µL, 0.49 mmol) 2-cyanoethyl diisopropylchlorophosphoramidite (54 µL, 0.49 mmol) was added dropwise. After 2 h the reaction mixture was diluted with CHCl₃ (50 mL), washed with washed with water (2×50 mL), dried over Na₂SO₄, evaporated, and the residue was dissolved in DCM (2 mL) and precipitated in hexane (200 mL). The solid was filteres off and dried *in vacuo.* Yield 310 mg, white amorphous solid. NMR (DMSO-*d*₆): 8.96 (s, 1H, N*H*); 7.47 (m, 1H, *H*-6); 7.39 (m, 2H, Ar*H*); 7.33 (m, 4H, Ar*H*); 7.24 (m, 2H, Ar*H*); 6.89 (m, 4H, Ar*H*); 6.26 (m, 1H, J= 6.7 Hz, H-1'); 4.64 (m, 1H, *H-*3')*;* 4.12 (m, 0.5H, *H*-4'); 4.07 (m, 0.5H, *H*-4'); 3.82-3.50 (m, 10H, OC*H*₃, NCH, POC*H*₂); 3.39-3.24 (m, 2H, *H*-5'); 3.05-2.90 (m, 4H, ArC*H*₂C*H*₂); 2.78 (s, 4H, COC*H*₂C*H*₂CO); 2.66 (m, 1H, C*H*₂CN); 2.54 (m, 1H, C*H*₂CN); 2.48-2.33 (m, 2H, *H*-2'); 1.54-0.88 (m, 12H, C(C*H*₃)₂). ³¹P NMR (MeCN-*d*₃): 149.3144, 148.2837.

**1-(4-Hydroxybutyryl)-4-hydroxypiperidine.** A solution of 4-hydroxypiperidine (4.05 g, 40 mmol), γ-butyrolactone (6.89 g, 80 mmol) and DMAP (20 mg) in MeOH (20 mL) was refluxed for 48 h. The mixture was evaporated, and the residue was purified on silica gel column (30% MeOH in CHCl₃). Yield 5.54 g (74%), colorless oil. NMR (DMSO-*d*₆): 4.69 (d, 1H, *J*= 4.1 Hz, CHO*H*); 4.41 (t, 1H, *J* = 5.3 Hz, CH₂O*H*); 3.90 (m, 1H, NC*H*H); 3.67 (m, 2H, CHOH, NC*H*H); 3.39 (m, 2H, C*H*₂OH); 3.12 (m, 1H, NCH*H*); 2.96 (m, 1H, NCH*H*); 2.31 (t, 2H, *J*= 7.4 Hz, COC*H*₂); 1.72 (m, 1H, NCH₂C*H*H); 1.63 (m, 3H, CH₂C*H*₂CH₂, NCH₂C*H*H); 1.31 (m, 1H, NCH₂CH*H*); 1.21 (m, 1H, NCH₂CH*H*).

**1-(4-{4,4'-Dimethoxy-4"-[2-(*N*-succinimidyloxycarbonyl)ethyl]trityloxy}butyryl)-4-hydroxypiperidine.** 1-(4-Hydroxybutyryl)-4-hydroxypiperidine (169 mg, 0.90 mmol) was tritylated with *N*-oxysuccinimide ester of 3-{4-[chloro-bis(4-methoxyphenyl)methyl]phenyl} propionic acid (300 mg, 0.65 mmol) in pyridine as above. The product was eluted with 30% acetone in toluene. Yield 140 mg (33%), white amorphous solid.

**1-(4-{4,4'-Dimethoxy-4"-[2-(*N*-succinimidyloxycarbonyl)ethyl]trityloxy}butyryl)-4-(diisopropylamino-2-cyanethoxyphosphinyloxy)piperidine.** 1-(4-{4,4'-Dimethoxy-4"-[2-(*N*-succinimidyloxycarbonyl)ethyl]trityloxy}butyryl)-4-hydroxypiperidine (120 mg, 0.18 mmol) was phosphitylated in DCM (5 mL) as above with DIEA (42 µL, 0.25 mmol) and 2-cyanoethyl diisopropylchlorophosphoramidite (27 µL, 0.25 mmol). The compound was dissolved in DCM (1 mL) and precipitated in hexane (100 mL). Yield 100 mg (65%), white amorphous solid.

*X = chloride*

### Example 64

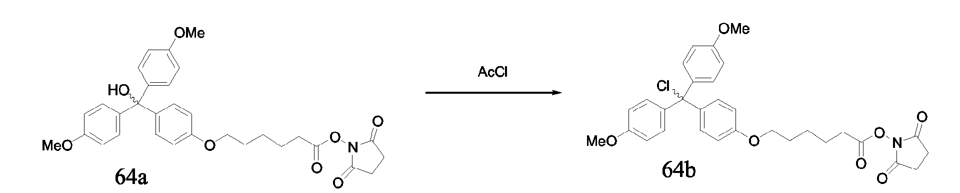

**6-{4-[Chloro-bis(4-methoxyphenyl)methyl]phenoxy}hexanoic** acid, ***N*-oxysuccinimide ester.** 6-{4-[Hydroxy-bis(4-methoxyphenyl)methyl]phenoxy}hexanoic acid, *N*-oxysuccinimide ester (1.095 g, 2.0 mmol was dissolved in freshly distilled AcCl (10 mL) and the mixture was refluxed (2 h), then diluted with petroleum ether (30 mL) and cooled to room temperature. The trityl chloride was filtered off, washed with petroleum ether, and dried *in vacuo.* Yield 962 mg (85%).

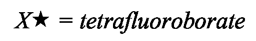

### Example 65

Example 28 relates to compounds 28b, 28c and 28d in which X 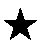 is tetrafluoroborate.

It will be understood that the invention is described above by way of example only and modifications may be made whilst remaining within the scope and spirit of the invention.

## Claims

1. A compound of formula (IIa): 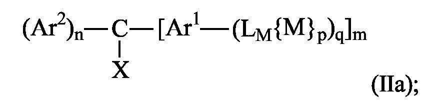 where:
X is a group capable of being cleaved from the α-carbon atom to form an ion of formula (I') 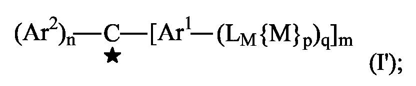
C 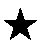 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more; and
q independently = 1 or more.

2. A compound of formula (IIb): 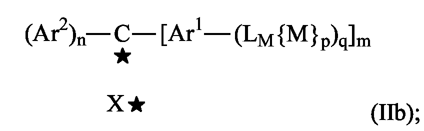 where:
X 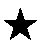 is a counter-ion to C 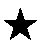 ;
C 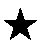 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more; and
q independently = 1 or more.

3. An ion of formula (I'): 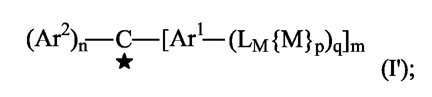 where:
C 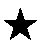 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more; and
q independently = 1 or more.

4. A solid support of formula (IVai), (IVaii) or (IVaiii): 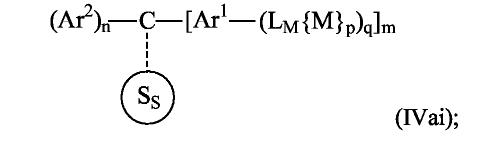 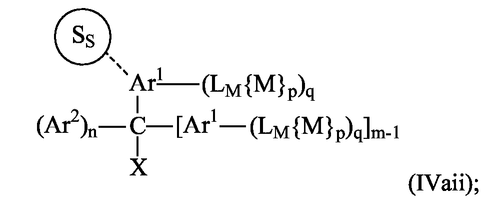 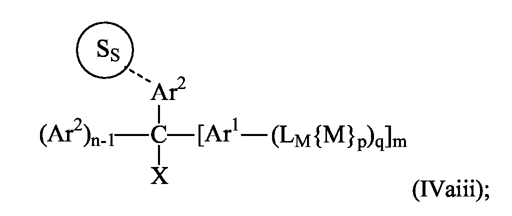 where:
X is a group capable of being cleaved from the α-carbon atom of the compound of formula (II) to form an ion of formula (I') 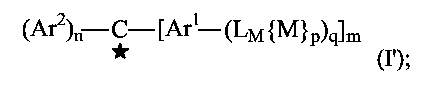
C 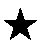 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more;
q independently = 1 or more;
Sₛ is a solid support;
C- - -Sₛ comprises a cleavable bond between C and Sₛ;
Sₛ---Ar¹ comprises a cleavable bond between Ar¹ and Sₛ; and
Sₛ---Ar² comprises a cleavable bond between Ar² and Sₛ.

5. A solid support of formula (IVbii) or (IVbiii): 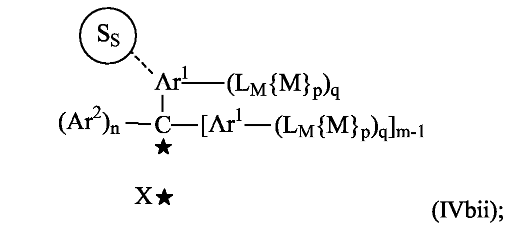 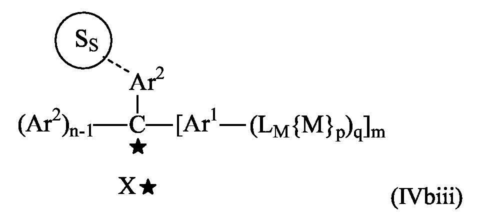 where:
X 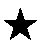 , Ar¹, Ar², L_{M}, M, n, m, p, q, Sₛ, C- - -Sₛ, Sₛ- - -Ar¹ and Sₛ---Ar² are as defined above.
X 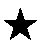 is a counter-ion to C 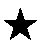 ;
C 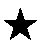 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more;
q independently = 1 or more;
Sₛ is a solid support;
C- - -Sₛ comprises a cleavable bond between C and Sₛ;
Sₛ---Ar¹ comprises a cleavable bond between Ar¹ and Sₛ; and
Sₛ---Ar² comprises a cleavable bond between Ar² and Sₛ.

6. A solid support of formula (IVaiv) or (IVbiv): 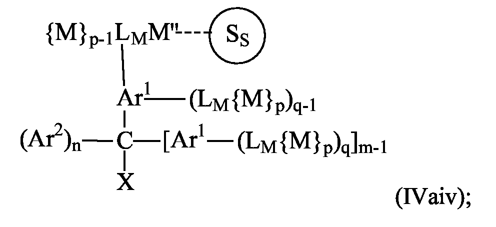 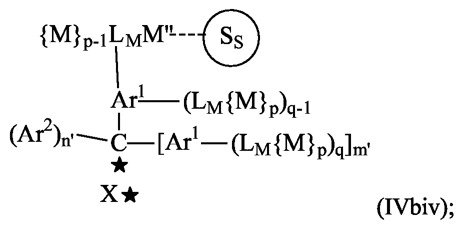 where:
X is a group capable of being cleaved from the α-carbon atom of the compound of formula (II) to form an ion of formula (I') 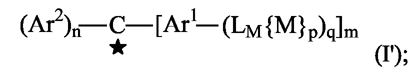
X 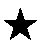 is a counter-ion to C 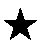 ;
C 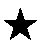 is a carbon atom bearing a single positive charge or a single negative charge;
M is independently a reactive functional group;
Ar¹ is independently an aromatic group or an aromatic group substituted with one or more A;
Ar² is independently an aromatic group or an aromatic group substituted with one or more A;
optionally wherein (a) two or three of the groups Ar¹ and Ar² are linked together by one or more L⁵, where L⁵ is independently a single bond or a linker atom or group; and/or (b) two or three of the groups Ar¹ and Ar² together form an aromatic group or an aromatic group substituted with one or more A;
A is independently a substituent;
L_{M} is independently a single bond or a linker atom or group;
n = 0, 1 or 2 and m = 1, 2, or 3, provided the sum of n+m = 3;
p independently = 1 or more;
q independently = 1 or more;
Sₛ is a solid support;
M"- - -Sₛ comprises a bond between M" and Sₛ; and
M" is the same as M except that Sₛ is bound to a portion of M which does not form part of the residue of M" remaining attached to the ion of formula (I') which residue is produced after reaction of group M".
